# EUROPEAN PATENT SPECIFICATION

(11) **EP 1 398 629 B1**
(45) Date of publication and mention of the grant of the patent: **20.04.2016**
(21) Application number: 03020695.7
(22) Date of filing: 11.09.2003
(51) Int. Cl.: B60H 1/00, G01N 33/00, B60H 3/00

(54) **Gas detection and automatic ventilation system for vehicle**
Gasnachweis- und automatisches Fahrzeugventilationsystem
Système automatique de ventilation d'une véhicule comprenant détection de gaz

(30) Priority: 12.09.2002 JP 2002266606
(43) Date of publication of application: 17.03.2004
(73) Proprietor: NGK SPARK PLUG CO., LTD., Nagoya-shi, Aichi 467-8525 (JP)
(72) Inventor: Kimoto, Yuji, Mizuho-ku, Nagoya, Aichi (JP); Ito, Shingo, Mizuho-ku, Nagoya, Aichi (JP); Iwasaki, Yuko, Mizuho-ku, Nagoya, Aichi (JP)
(74) Representative: Zimmermann & Partner Patentanwälte mbB

(56) References cited:
- EP-A- 1 316 799
- WO-A-01/92864
- PATENT ABSTRACTS OF JAPAN vol. 2002, no. 02, 2 April 2002 (2002-04-02) & JP 2001 281185 A (NGK SPARK PLUG CO LTD), 10 October 2001 (2001-10-10)
- PATENT ABSTRACTS OF JAPAN vol. 2002, no. 06, 4 June 2002 (2002-06-04) & JP 2002 055068 A (NGK SPARK PLUG CO LTD), 20 February 2002 (2002-02-20)
- PATENT ABSTRACTS OF JAPAN vol. 1999, no. 05, 31 May 1999 (1999-05-31) & JP 11 042925 A (DENSO CORP;TOYOTA MOTOR CORP), 16 February 1999 (1999-02-16)

## Description

### Field of the Invention

The present invention relates to gas detection apparatuses and the usage thereof. Further, the present invention relates to gas detection apparatus for use in a vehicle ventilation system. Even further, the present invention relates to a gas detection apparatus that detects changes in the concentration of a specific gas in an environment by use of a gas sensor element, and to an automatic ventilation system for a vehicle. Specifically, it relates to a gas detection apparatus, a method of usage thereof and an automatic ventilation system for a vehicle.

### Background of the Invention

Conventionally, there has been known a gas sensor element which utilizes a metal oxide semiconductor such as WO₃ or SnO₂. Since the sensor resistance of such a gas sensor element changes with concentration of a specific gas, such as an oxidative gas (e.g., NOₓ) or a reducing gas (e.g., CO or HC (hydrocarbon)) present in an environment, changes in the concentration of the specific gas can be detected on the basis of changes in the sensor resistance. Further, there has been known a gas detection apparatus which utilizes an electric circuit in order to obtain a sensor output value corresponding to the sensor resistance of such a gas sensor element, and detects changes in the concentration of a specific gas on the basis of changes in the sensor output value. Moreover, there have been known various types of control systems using such a gas detection apparatus; e.g., an automatic ventilation system for a vehicle in which a flap is opened and closed in accordance with the degree of contamination of air outside the vehicle compartment in order to effect switching between introduction of external air into the compartment and recirculation of air inside the compartment (see, for example, Japanese Patent Application Laid-Open (kokai) No. 11-42925).

However, in general, changes in the sensor resistance of such a gas sensor element do not necessarily coincide with changes in concentration of a specific gas. Specifically, changes in the sensor resistance delay with respect to changes in the concentration of the specific gas. In particular, once the gas sensor element is exposed to a specific gas of high concentration and assumes a high sensor resistance corresponding to the high concentration, even when the concentration of the specific gas decreases thereafter, the sensor resistance of the gas sensor element returns to a resistance value corresponding to low concentration gradually rather than quickly. In other words, a hysteresis phenomenon occurs in the sensor resistance of such a gas sensor element, so that the sensor resistance depends not only on concentration of a specific gas at each point in time, but also on the past history, such as concentration of the specific gas to which the gas sensor element has been exposed previously.

Conceivably, such a phenomenon occurs because molecules of the specific gas are adsorbed by the gas sensor element when the concentration of the specific gas increases, and, when the concentration of the specific gas decreases, the absorbed molecules of the specific gas separate gradually rather than quickly.

Moreover, if the concentration of the specific gas increases again in a period during which the sensor resistance is gradually returning to a resistance value corresponding to low gas concentration, the sensor resistance does not change immediately, but starts to change after the concentration of the specific gas has increased to some degree. Therefore, in the case of a gas detection apparatus which detects changes in the concentration of a specific gas by use of a sensor output value corresponding to the sensor resistance, during a period in which the above-described hysteresis phenomenon occurs, the sensitivity in detecting an increase in concentration of the specific gas decreases substantially, whereby the gas detection apparatus may fail to detect an increase in concentration of the specific gas or may take time to detect an increase in concentration of the specific gas.

WO 01/92864 describes a gas detector which includes a gas sensor element for detecting the concentration of a gas and generating an output signal which is fed to a microcomputer via an A/D converter. The gas sensor also suffers from the hysteresis phenomenon as described above.

JP 2001-281185 discloses a control system for generating a signal indicative of the concentration of a gas.

JP 2002-055068 discloses a gas detector comprising a gas sensor for a vehicle.

EP 1 316 799 published after the priority date of the present application describes a gas detection apparatus and automatic ventilation system which includes a gas sensor element for generating a signal indicative of the concentration of a gas. A difference value is calculated from a difference between the sensor output value and a previously calculated base value. The rule for calculating the base value is different for a situation where a low concentration signal is generated and for a situation where a high concentration signal is generated and that these different values are also compared with different thresholds Tu and Td.

The present invention has been accomplished in view of the forgoing problems, and an object of the present invention is to provide a gas detection apparatus which can detect reliably, or can detect quickly, an increase in concentration of a specific gas, as well as an automatic ventilation system for a vehicle using the same.

### Summary of the Invention

The present invention intends to provide an improved gas detection apparatus. According to aspects of the present invention, a gas detection apparatus according to independent claim 1, an automatic ventilation system according to claim 21 and a method of operating a gas detection apparatus according to independent claim 22 are provided.

Further advantages, features, aspects and details of the invention are evident from the dependent claims, the description and the accompanying drawings.

According to one aspect, the means for solution is a gas detection apparatus using a gas sensor element whose sensor resistance changes in accordance with concentration of a specific gas, comprising: acquisition means for acquiring a sensor output value at predetermined cycle intervals, the sensor output value corresponding to the sensor resistance; and concentration detection means for detecting a change in the concentration of the specific gas and for generating a low concentration signal when the concentration of the specific gas is low and a high concentration signal when the concentration of the specific gas is high, wherein the concentration detection means comprises concentration increase detection means for generating the high concentration signal in place of the low concentration signal when, during a period in which the low concentration signal is generated, the sensor 'output value shifts toward a high concentration direction with respect to the reference value by an amount equal to or greater than a high concentration threshold, and concentration decrease detection means for generating the low concentration signal in place of the high concentration signal when, during a period in which the high concentration signal is generated, the sensor output value shifts toward a low concentration direction with respect to the reference value by an amount equal to or greater than a low concentration threshold, wherein the concentration increase detection means uses a first high concentration threshold as the high concentration threshold during a sensitized period of the period in which the low concentration signal is generated, the sensitized period following a switching from the high concentration signal to the low concentration signal, and a second high concentration threshold as the high concentration threshold during the remaining portion of the period in which the low concentration signal is generated, and wherein the first high concentration threshold is a value on a sensitivity-increasing-direction side with respect to the second high concentration threshold.

In the gas detection apparatus of the present invention, two high concentration thresholds; i.e., first and second high concentration thresholds, are selectively used for the sensitized period and the remaining portion of the period in which the low concentration signal is generated. Further, the first high concentration threshold is a value on a sensitivity-increasing-direction side with respect to the second high concentration threshold. Therefore, during the sensitized period in which the first high concentration threshold is used, a high concentration signal can be generated in response to a small change in the sensor output value toward the high density direction, whereby a drop in detection sensitivity for an increase in the concentration of the specific gas stemming from a hysteresis phenomenon can be compensated.

Notably, the reference value may be a fixed value which does not change with time. Alternatively, the reference value may be calculated every time a predetermined number of cycles have elapsed. Specifically, the reference value may be calculated upon passage of a period which corresponds to a single cycle interval or an integral multiple of the cycle interval, such as two cycle intervals.

In the present specification, the term "hysteresis phenomenon" refers to a phenomenon in which once the gas sensor element is exposed to a specific gas of high concentration and assumes a sensor resistance corresponding to the high concentration, even when the concentration of the specific gas decreases thereafter, a change in the sensor resistance of the gas sensor element is delayed with respect to a change in the gas concentration, so that the sensor resistance of the gas sensor element returns gradually rather than quickly to a resistance value corresponding to low concentration. Further, the gas detection apparatus obtains and uses a sensor output value corresponding to the sensor resistance. Therefore, the term "hysteresis phenomenon" used in relation to the sensor output value refers to a phenomenon in which once the gas sensor element is exposed to a specific gas of high concentration and the sensor output value changes to a value corresponding to the high concentration, even when the concentration of the specific gas decreases thereafter, a change in the sensor output value is delayed with respect to a change in the gas concentration, so that the sensor output value returns gradually rather than quickly to a value corresponding to low concentration.

Further, the term "hysteresis period" refers to a period between a point in time at which the gas detection apparatus effects switching from the high concentration signal to the low concentration signal in response to a decrease in the concentration of the specific gas and a point in time at which a hysteresis phenomenon substantially ends, and the sensor resistance and the sensor output value assume respective values corresponding to low concentration of the specific gas.

Gas sensor elements are classified into two types; i.e., a gas sensor element having characteristics such that its sensor resistance increases as the concentration of a specific gas (e.g., oxidative gas such as NOx) increases, and a gas sensor element having characteristics such that its sensor resistance decreases as the concentration of a specific gas (e.g., reducing gas such as CO, HC) increases.

Further, gas detection apparatuses are classified into two types; i.e., a gas detection apparatus in which an electronic circuit for obtaining a corresponding sensor output value from a sensor resistance is configured in such a manner that the obtained sensor output value increases as the sensor resistance increases, and a gas detection apparatus in which the electronic circuit is configured in such a manner that the obtained sensor output value decreases as the sensor resistance increases.

In view of the foregoing, in the present specification, for the sake of convenience, a direction in which the sensor output value changes when the concentration of the specific gas increases is called a high concentration direction.

Accordingly, for a gas detection apparatus in which the characteristics of the gas sensor element and the configuration of the electronic circuit are determined in such a manner that the sensor output value increases as the sensor resistance increases, the high concentration direction used in relation to the sensor output value refers to the direction of increase of the sensor output value. In contrast, for a gas detection apparatus designed in such a manner that the sensor output value decreases as the sensor resistance increases, the high concentration direction used in relation to the sensor output value refers to the direction of decrease of the sensor output value.

By extension, other values, such as a reference value, a difference value, and a maximum difference, which change in response to a change in the sensor output value, are handled in the same manner. That is, the high concentration direction used in relation to the reference value or any other value refers to the direction in which the calculated reference value or any other value changes when the concentration of the specific gas increases.

In contrast, the term "low concentration direction" refers to a direction opposite the high concentration direction.

Moreover, the expression "change in the sensitivity increasing direction" used in relation to high concentration threshold refers to change in a direction which facilitates generation of a high concentration signal in response to a change in the sensor output value that occurs when the concentration of the specific gas increases. For example, in the case where the high concentration threshold is lowered so as to cause the high concentration signal to be generated more easily in response to the same change in the sensor output value, lowering the high concentration threshold corresponds to changing the high concentration threshold in the sensitivity increasing direction. Therefore, when the high concentration threshold is changed in the sensitivity increasing direction, the high concentration signal can be generated in response to a smaller change in the sensor output value, as compared with the case where the high concentration threshold is used without being changed.

Furthermore, the high concentration threshold may be set freely in accordance with a reference value to be used or a calculation formula for calculating the reference value, and in some cases, instead or a positive value, zero or a negative value may be employed as the high concentration threshold. Similarly, the low concentration threshold may be set freely in accordance with a reference value to be used or a calculation formula for calculating the reference value, and in some cases, instead or a positive value, zero or a negative value may be employed as the low concentration threshold.

Preferably, the gas detection sensor comprises first-high-concentration-threshold update means for causing, in the sensitized period, the first high concentration threshold to gradually approach the second high concentration threshold with elapse of time.

In general, the difference between a theoretical sensor resistance or sensor output value corresponding to an actual concentration of the specific gas and an actually measured sensor resistance or sensor output value (i.e., a measured sensor resistance or sensor output value affected by a hysteresis phenomenon), which difference is produced as a result of the hysteresis phenomenon, gradually decreases with lapse of time after the concentration of the specific gas has decreased.

In the present invention, since the first-high-concentration-threshold update means causes the first high concentration threshold to gradually approach the second high concentration threshold, gas detection can be performed by use of the first high concentration threshold that assumes a proper magnitude at each point in time during a sensitized period, while reflecting the above-described phenomenon.

Notably, the manner of changing the first high concentration threshold may be determined freely. An example manner of changing is linearly changing the first high concentration threshold.

Preferably, the gas detection sensor comprises first-high-concentration-threshold determining means for determining the first high concentration threshold on the basis of hysteresis strength information.

In the gas detection sensor of the present invention, since the first high concentration threshold is determined on the basis of hysteresis strength information, the first high concentration threshold can be determined to have a proper magnitude, and the thus-determined first high concentration threshold can be used.

The term "hysteresis strength information" refers to information that relates to the degree of a hysteresis phenomenon predicted to occur during a sensitized period. Specifically, examples of such information include a change (inclination) in the sensor output value at the time of switching from the high concentration signal to the low concentration signal or at a point in time before or after that time, the length of a period before the switching in which the high concentration signal continues, the peak magnitude of the sensor output value in a high-concentration-signal generation period before the switching, and the peak magnitude of the difference between a reference value and the sensor output value in a high-concentration-signal generation period before the switching. Other examples of such information include the sum (integral) of differences between the sensor output value at the time of switching to the high concentration signal and sensor output values at respective points in time in a high-concentration-signal generation period, and the sum (integral) of differences between the reference value and respective sensor output values in a high-concentration-signal generation period. Moreover, there can be used an average value which is obtained by dividing any of these sums by a period between the switching to the high concentration signal and the switching to the low concentration signal.

Preferably, the gas detection sensor comprises threshold-determining moving-difference-value calculation means for calculating a first-high-concentration-threshold-determining moving difference value from a present sensor output value and an m^{th} past sensor output value which is a sensor output value obtained m cycles before the present sensor output value (m is an integer not less than 1), wherein the hysteresis strength information includes the first-high-concentration-threshold-determining moving difference value which is obtained at the time of switching from the high concentration signal to the low concentration signal, a point in time a predetermined time earlier than the time of switching, or a point in time a predetermined time later than the time of switching, and wherein the first-high-concentration-threshold update means shifts the first high concentration threshold in the sensitivity increasing direction in accordance with the absolute value of the first-high-concentration-threshold-determining moving difference value, which is used as the hysteresis strength information.

The present inventors found that, in general, in the case where a hysteresis phenomenon occurs, if the sensor output value changes greatly in the low concentration direction immediately after the concentration of the specific gas decreases, a subsequent change in the sensor output value in the low concentration direction is also large. Further, the present inventors found that in such a case, the difference between a theoretical sensor resistance or sensor output value corresponding to an actual concentration of the specific gas and an actual sensor resistance or sensor output value increases as a result of the hysteresis phenomenon, and the sensitivity in detecting the specific gas decreases greatly.

In view of the above, in the gas detection apparatus of the present invention, the hysteresis strength information includes the first-high-concentration-threshold-determining moving difference value which is obtained at the time of switching or a point in time before or after the time of switching. By virtue of its characteristics, the moving difference value represents a change between two sensor output values which are separated from each other by a predetermined number (m) of cycles; i.e., an inclination. Therefore, it is surmised that the greater the separation between the moving difference value and zero; i.e., the greater the absolute value of the moving difference value, the greater the subsequent change in the sensor output value in the low concentration direction, and thus, the greater the degree of the hysteresis phenomenon, resulting in a great decrease in the sensitivity in detecting the specific gas. In view of the foregoing, the first high concentration threshold is shifted toward the sensitivity increasing direction to assume a proper magnitude, whereby the drop in detection sensitivity can be properly prevented.

Notably, although the predetermined cycle number "m" in relation to the moving difference value for determining the first high concentration threshold may be determined freely in consideration of sampling intervals, responsiveness, etc., m is preferably set to 2 or greater. This is because, when m is set to 1, the gas detection apparatus is greatly affected by noise. In contrast, when m is set to an extremely large value, it becomes difficult to properly reflect on the moving difference value a change in the sensor output value in the low concentration direction after drop of the concentration of the specific gas.

In the present specification, the term "moving difference value" refers to a difference which is obtained from two numerical values which are selected from a time series of numerical values and are separated from each other by a predetermined interval, while the two numerical values to be selected are shifted successively with elapse of time. For example, in the case where the time series of numerical values are represented by S(1), S(2), ... S(n-m), S(n-m+1), ... S(n), S(n+1), ..., the term "moving difference value" refers to, for example, a difference DD(n) between the numerical value S(n) and the numerical value S(n-m), which is obtained m cycles before the numerical value S(n); i.e., DD(n) = S (n) - S(n-m), DD(n+1) = S(n+1) - S(n-m+1), etc. Notably, depending on the characteristics of the gas sensor element and the configuration of the electronic circuit for obtaining sensor output values, DD(n) = S(n-m)- S(n), DD(n+1) = S(n-m+1) - S(n+1), etc. may be used as moving difference values.

Preferably, in the gas detection sensor, the hysteresis strength information includes a maximum difference which is a difference between a peak sensor output value and a start-point sensor output value, the peak sensor output value being a value that shifts in the high concentration direction to the greatest degree among sensor output values obtained in a high-concentration-signal generation period immediately before the time of switching from the high concentration signal to the low concentration signal, and the start-point sensor output value being a sensor output value obtained at the beginning of the high-concentration-signal generation period, and wherein the first-high-concentration-threshold update means shifts the first high concentration threshold in the sensitivity increasing direction as the absolute value of the maximum difference increases.

In the gas detection apparatus of the present invention, the first high concentration threshold is determined on the basis of the hysteresis strength information that includes the maximum difference. The maximum difference, which is the difference between the start-point sensor output value which relates to the state in which the concentration of the specific gas is low and the peak sensor output value which relates to the maximum value of the concentration of the specific gas, is considered to assume a magnitude corresponding to the maximum concentration of the specific gas to which the gas sensor element is exposed during the high-concentration-signal generation period immediately before the time of switching from the high concentration signal to the low concentration signal. In addition, it is surmised that the greater the absolute value of the maximum difference, the greater the degree of a hysteresis phenomenon that occurs after the switching. Therefore, by means of shifting the first high concentration threshold toward the sensitivity increasing direction to assume a proper magnitude, the drop in detection sensitivity can be properly prevented.

Notably, depending on the characteristics of the gas sensor element and the configuration of the electronic circuit for obtaining sensor output values, the maximum difference may be obtained through subtraction of the start-point sensor output value from the peak sensor output value or subtraction of the peak sensor output value from the start-point sensor output value.

Alternatively, preferably, in the gas detection sensor, the hysteresis strength information includes a maximum difference value which is a difference selected from differences between the reference value and respective sensor output values obtained in a high-concentration-signal generation period immediately before the time of switching from the high concentration signal to the low concentration signal, the selected difference having the highest absolute value among the differences, and wherein the first-high-concentration-threshold update means shifts the first high concentration threshold in the sensitivity increasing direction as the absolute value of the maximum difference increases.

In the gas detection apparatus of the present invention, the first high concentration threshold is determined on the basis of the hysteresis strength information that includes the maximum difference value, which is a difference selected from differences between the reference value and sensor output values and having the highest absolute value. The differences between respective sensor output values and the reference value are considered to reflect, to some degree, gas concentrations at respective points in time. Therefore, the maximum difference value is considered to assume a magnitude corresponding to the maximum concentration of the specific gas to which the gas sensor element is exposed in a high-concentration-signal generation period immediately before the time of switching from the high concentration signal to the low concentration signal. In addition, it is surmised that the greater the absolute value of the maximum difference value, the greater the degree of a hysteresis phenomenon that occurs after the switching. Therefore, by means of shifting the first high concentration threshold toward the sensitivity increasing direction to assume a proper magnitude, the drop in detection sensitivity can be properly prevented.

Notably, depending on the characteristics of the gas sensor element and the configuration of the electronic circuit for obtaining sensor output values, the difference between the sensor output value and the reference value may be obtained through subtraction of the reference value from the sensor output value or subtraction of the sensor output value from the reference value.

Preferably, in the gas detection sensor, the hysteresis strength information includes duration time of a high-concentration-signal generation period immediately before the time of switching from the high concentration signal to the low concentration signal, wherein the first-high-concentration-threshold update means shifts the first high concentration threshold in the sensitivity increasing direction as the length of the duration time increases.

The degree of a hysteresis phenomenon; i.e., the degree of drop in detection sensitivity, is considered to depend on the duration time of a period in which the gas sensor element is exposed to the specific gas of high concentration. This is because, even in the case where the concentration of the specific gas is constant, if the gas sensor element is exposed to the gas for a long period of time, a large number of gas molecules are considered to be adsorbed, and the degree of hysteresis increases, whereby the degree of drop in detection sensitivity increases.

In view of the above, in the gas detection apparatus of the present invention, the first high concentration threshold is determined on the basis of the hysteresis strength information that includes the duration time of the high-concentration-signal generation period immediately before the time of switching from the high concentration signal to the low concentration signal. In addition, it is surmised that the longer the duration time, the greater the degree of a hysteresis phenomenon. Therefore, by means of shifting the first high concentration threshold toward the sensitivity increasing direction to assume a proper magnitude, the drop in detection sensitivity can be properly prevented.

Preferably, the gas detection sensor comprises end means for ending the sensitized period when a first predetermined period of time has passed after the time of switching from the high concentration signal to the low concentration signal.

Once having entered a high-concentration-signal generation period; i.e., once the gas sensor element is exposed to the specific gas of high concentration, a hysteresis phenomenon unavoidably occurs over a period of some length after the time of switching to the low concentration signal, irrespective of concentration of the specific gas to which the gas sensor element has been exposed or the duration of the exposure.

In view of the above, in the gas detection apparatus of the present invention, the sensitized period, in which the first high concentration threshold is used, is ended when the first predetermined period of time has passed after the time of switching. In other words, the first predetermined period of time subsequent to the time of switching is used as the sensitized period. This operation enables securing at least the sensitized period, to thereby prevent a drop in detection sensitivity at least in that period. Further, in general, the greatest drop in detection sensitivity stemming from a hysteresis phenomenon occurs immediately after the switching, and in many cases, setting the sensitized period to include a period immediately after the switching provides a sufficient effect. Moreover, this operation facilitates the relevant processing, and simplifies the control.

Alternatively, preferably, the gas detection sensor comprises end-timing advance determining means for previously determining the end of the sensitized period on the basis of hysteresis period information.

In the gas detection apparatus of the present invention, the end-timing advance determining means previously determines the end of the sensitized period on the basis of hysteresis period information. Therefore, the end of the sensitized period can be properly determined in advance.

The term "hysteresis period information" refers to information regarding the length of a hysteresis period; specifically, information regarding the length of a period subsequent to the time of switching from the high concentration signal to the low concentration signal, over which period a hysteresis phenomenon is predicted to occur. More specifically, examples of such information include a change (inclination) in the sensor output value at the time of switching from the high concentration signal to the low concentration signal or at a point in time before or after that time, the length of a period before the switching during which the high concentration signal continues, the peak magnitude of the sensor output value in a high-concentration-signal generation period before the switching, and the peak magnitude of the difference between a reference value and the sensor output value in a high-concentration-signal generation period before the switching. Other examples of such information include the sum (integral) of differences between the sensor output value at the time of switching to the high concentration signal and sensor output values at respective points in time during a high-concentration-signal generation period, and the sum (integral) of differences between the reference value and respective sensor output values in a high-concentration-signal generation period. Moreover, there can be used an average value which is obtained by dividing any of these sums by a period between the switching to the high concentration signal and the switching to the low concentration signal.

Notably, since information that is obtained before determination of the end of the sensitized period can be used as the hysteresis period information, there can be used any one of information obtained at the time of switching from the high concentration signal to the low concentration signal, information obtained before the time of switching, and information obtained during a period between the time of switching and the time at which the end of the sensitized period is determined.

Preferably, the gas detection sensor comprises end-timing-determining moving-difference-value calculation means for calculating an end-timing-determining moving difference value from a present sensor output value and an j^{th} past sensor output value which is a sensor output value obtained j cycles before the present sensor output value (j is an integer not less than 1), wherein the hysteresis period information includes the end-timing-determining moving difference value which is obtained at the time of switching from the high concentration signal to the low concentration signal, a point in time a predetermined time earlier than the time of switching, or a point in time a predetermined time later than the time of switching, and wherein the end-timing advance determining means delays the end of the sensitized period to a greater degree with increasing absolute value of the end-timing-determining moving difference value, which is used as the hysteresis period information.

The present inventors found that in general, if the sensor resistance or the sensor output value greatly changes in the low concentration direction immediately after the concentration of the specific gas decreases, after that, the sensor resistance or the sensor output value continuously changes in the low concentration direction for a long time. Further, the present inventors found that in such a case, a change in the sensor resistance or the sensor output value stemming from a hysteresis phenomenon continues for a long time, whereby a hysteresis period is prolonged.

In view of the above, in the gas detection apparatus of the present invention, the end of the sensitized period is previously determined on the basis of the hysteresis period information that includes the end-timing-determining moving difference value which is obtained at the time of switching from the high concentration signal to the low concentration signal, or a point in time before or after the time of switching. Due to its characteristics, the moving difference value represents a change between two sensor output values which are separate from each other by a predetermined number (j) of cycles; i.e., an inclination. Therefore, it is surmised that the greater the absolute value of the end-timing-determining moving difference value, for example, at the time of switching; i.e., the greater the absolute value of the moving difference value, the greater the subsequent change in the sensor output value in the low concentration direction, and thus, the longer the period in which the hysteresis phenomenon continue, so that the sensitivity in detecting the specific gas decreases for a long period of time. In view of the foregoing, the end of the sensitized period is delayed more as the absolute value of the moving difference value increases, whereby a sensitized period having a proper length is set in order to properly prevent the drop in detection sensitivity.

Preferably, in the gas detection sensor, the hysteresis period information includes a maximum difference which is a difference between a peak sensor output value and a start-point sensor output value, the peak sensor output value being a value that shifts in the high concentration direction to the greatest degree among sensor output values obtained in a high-concentration-signal generation period immediately before the time of switching from the high concentration signal to the low concentration signal, and the start-point sensor output value being a sensor output value obtained at the beginning of the high-concentration-signal generation period, wherein the end-timing advance determining means delays the end of the sensitized period to a greater degree with increasing absolute value of the maximum difference.

The duration time of a hysteresis phenomenon; i.e., the duration time of drop in detection sensitivity is considered to depend on the maximum concentration of the specific gas to which the gas sensor element is exposed in a high-concentration-signal generation period immediately before the time of switching from the high concentration signal to the low concentration signal. Further, when the maximum concentration is high, the length of a hysteresis period is considered to increase, because the specific gas is adsorbed in a greater amount.

In view of the above, in the gas detection apparatus of the present invention, the end of the sensitized period is previously determined on the basis of the hysteresis period information that includes the maximum difference, which is the difference between the start-point sensor output value which relates to the state in which the concentration of the specific gas is low and the peak sensor output value which relates to the maximum value of the concentration of the specific gas. In addition, it is surmised that the greater the separation of the maximum difference from zero, the longer the length of a hysteresis period. Therefore, by means of delaying the end of the sensitized period, the drop in detection sensitivity can be properly prevented over a proper period.

Notably, depending on the characteristics of the gas sensor element and the configuration of the electronic circuit for obtaining sensor output values, the maximum difference may be obtained through subtraction of the start-point sensor output value from the peak sensor output value or subtraction of the peak sensor output value from the start-point sensor output value.

Alternatively, preferably, in the gas detection sensor, the hysteresis period information includes a maximum difference value which is a difference selected from differences between the reference value and respective sensor output values obtained in a high-concentration-signal generation period immediately before the time of switching from the high concentration signal to the low concentration signal, the selected difference having the greatest absolute value among the differences, wherein the end-timing advance determining means delays the end of the sensitized period to a greater degree with increasing absolute value of the maximum difference value.

In the gas detection apparatus of the present invention, the end of the sensitized period is previously determined on the basis of the hysteresis period information that includes the maximum difference value, which is a difference which is selected from differences between the reference value and sensor output values and is most separated from zero. In addition, it is surmised that the greater the separation of the maximum difference value from zero, the longer the length of a hysteresis period. Therefore, by means of delaying the end of the sensitized period, the drop in detection sensitivity can be properly prevented over a proper period.

Notably, depending on the characteristics of the gas sensor element and the configuration of the electronic circuit for obtaining sensor output values, the difference between the sensor output value and the reference value may be obtained through subtraction of the reference value from the sensor output value or subtraction of the sensor output value from the reference value.

Preferably, in the gas detection sensor, the hysteresis period information includes duration time of the high-concentration-signal generation period immediately before the time of switching from the high concentration signal to a low concentration signal, wherein the end-timing advance determining means delays the end of the sensitized period to a greater degree as the length of the duration time increases.

The duration time of a hysteresis phenomenon is considered to depend on the duration time of the specific gas to which the gas sensor element is exposed. That is, even in the case where the concentration of the specific gas is constant, if the gas sensor element is exposed to the gas for a long time, the length of the hysteresis period is considered to increase accordingly.

In the gas detection apparatus of the present invention, the end of the sensitized period is previously determined on the basis of the hysteresis period information that includes the duration time of the high-concentration-signal generation period immediately before the time of switching from the high concentration signal to the low concentration signal. In addition, it is surmised that the longer the duration time, the longer the length of a hysteresis period. Therefore, by means of delaying the end of the sensitized period more, the drop in detection sensitivity can be properly prevented over a proper period.

Preferably, the gas detection sensor comprises: alternation-determining moving-difference-value calculation means for calculating an alternation-determining moving difference value from a present sensor output value and an i^{th} past sensor output value which is a sensor output value obtained i cycles before the present sensor output value (i is an integer not less than 1); and moving-difference-value determining means for determining whether the alternation-determining moving difference value is located on the side toward 0 with respect to a predetermined value serving as a reference, wherein the concentration increase detection means uses, in place of the first high concentration threshold, the second high concentration threshold as the high concentration threshold after the alternation-determining moving difference value has shifted to the side toward 0.

In the case where the concentration of the specific gas does not increase again after the switching from the high concentration signal to the low concentration signal, it is considered that a hysteresis phenomenon gradually ends with elapse of time, the change in the sensor output value in the low concentration direction decreases, and a sensor output value generally corresponding to a decreased concentration of the specific gas is obtained.

In view of the above, in the gas detection apparatus of the present invention, a determination is made as to whether the alternation-determining moving difference value (e.g., S(n) - S(n-i)) has shifted to the side toward 0 with respect to the predetermined value serving as a reference; and when the alternation-determining moving difference value has shifted to the side toward 0, in place of the first high concentration threshold, the second high concentration threshold is used. That is, the sensitized period is ended, because the alternation-determining moving difference value having shifted to the side toward 0 with respect to the predetermined value serving as a reference indicates that the change in the sensor output value in the low concentration direction has decreased, and the moving difference value has approached zero. In this manner, the high concentration signal can be switched from the first high concentration threshold to the second high concentration threshold at a proper timing, whereby the drop in detection sensitivity can be properly prevented over a proper period.

Alternatively, the gas detection sensor comprises: alternation-determining moving-difference-value calculation means for calculating an alternation-determining moving difference value from a present sensor output value and an i^{th} past sensor output value which is a sensor output value obtained i cycles before the present sensor output value (i is an integer not less than 1); and moving-difference-value determining means for determining whether the alternation-determining moving difference value is located on the side toward 0 with respect to a predetermined value serving as a reference, wherein the concentration increase detection means uses, in place of the first high concentration threshold, the second high concentration threshold as the high concentration threshold after elapse of a predetermined period of time after the alternation-determining moving difference value has shifted to the side toward 0.

As described above, in the case where the concentration of the specific gas does not increase again after the switching from the high concentration signal to the low concentration signal, the change in the sensor output value in the low concentration direction decreases with elapse of time.

In view of the above, in the gas detection apparatus of the present invention, a determination is made as to whether the alternation-determining moving difference value (e.g., S(n) - S(n-i)) has shifted to the side toward 0 with respect to the predetermined value serving as a reference; and when a predetermined period of time has elapsed after the alternation-determining moving difference value has shifted to the side toward 0, in place of the first high concentration threshold, the second high concentration threshold is used. That is, the sensitized period is ended.

This is because the shift of the alternation-determining moving difference value (e.g., S(n) - S(n-i)) to the side toward 0 with respect to the predetermined value serving as a reference indicates that the change in the sensor output value in the low concentration direction has decreased, and the moving difference value has approached zero.

Incidentally, in the case where the sensor output value had changed in the high concentration direction in response to an increase in the concentration of the specific gas with the result that the moving difference value has shifted to the side toward 0 with respect to the predetermined value serving as a reference, if the high concentration signal is switched, at that point in time, to the second high concentration threshold, which is a value toward the high concentration direction with respect to the first high concentration threshold (the sensitized period is ended to thereby switch the high concentration threshold to a value toward the direction opposite the sensitivity increasing direction), the sensor output value encounters difficulty in deviating in the high concentration direction with respect to the reference value by the high concentration threshold or more, so that in some cases, detection of an increase in the concentration of the specific gas becomes difficult or involves delay.

In view of the above, the present gas detection apparatus uses the second high concentration threshold in place of the first high concentration threshold after waiting elapse of a predetermined period. That is, the sensitized period is extended by the predetermined period. Therefore, even in the case where the sensor output value changes in the high concentration direction in response to an increase in the concentration of the specific gas, if such a change occurs in the predetermined period, an increase in the concentration of the specific gas can be detected by use of the first high concentration threshold, whereby the increase in the concentration of the specific gas can be detected reliably and quickly.

Preferably, in the gas detection apparatus, from the time of switching from the high concentration signal to the low concentration signal, the alternation-determining moving-difference-value calculation means starts the calculation of the alternation-determining moving difference value, or resumes the calculation of the alternation-determining moving difference value without use of sensor output values obtained before the time of switching, wherein during a period from the time of switching up to a point in time at which obtainment of sensor output values corresponding to the predetermined number (i) of cycles is completed, the difference between a sensor output value obtained at the time of switching and a sensor output value obtained in each subsequent cycle is used as the alternation-determining moving difference value in respective cycles.

In the gas detection apparatus, which utilizes the alternation-determining moving difference value, if successive calculation of the alternation-determining moving difference value is performed continuously before the time of switching from the high concentration signal to the low concentration signal, depending on the manner of change of the sensor output value or the value of i (number of cycles), the moving difference value calculated at the time of switching may fail to assume a magnitude that indicates occurrence of a hysteresis phenomenon even though a hysteresis phenomenon has occurred. Such a phenomenon is considered to occur, for example, in the case where the concentration of the specific gas increases and then decreases (i.e., increases temporarily) within a short period of time. In such a case, the width of a change period in which the sensor output value temporarily increases (or temporarily decreases) due to the temporary increase in the concentration of the specific gas is about the same or longer than a period required for the predetermined number i of cycles. In such a case, even though the detection sensitivity has been lowered for reasons of a hysteresis phenomenon, the sensitized period cannot be set properly, so that detection sensitivity remains at a dropped level.

In the gas detection apparatus of the present invention, from the time of switching from the high concentration signal to the low concentration signal, calculation of the alternation-determining moving difference value is started or resumed. Incidentally, during the period from the start or resumption up to a point in time at which obtainment of sensor output values corresponding to the predetermined number (i) of cycles is completed, naturally, no moving difference value can be obtained. However, in the present gas detection apparatus, in such a period, the difference (DI(n) = S(n) - S(n+b)) between a sensor output value (S(n)) obtained at the time of switching and a sensor output value (S(n+b), b = 1, 2, ..., i) obtained in each subsequent cycle is used as the alternation-determining moving difference value in respective cycles, whereby obtainment of the alternation-determining moving difference value is started when one cycle has passed from the time of switching from the high concentration signal to the low concentration signal. Then, the moving-difference-value determining means determines whether the alternation-determining moving difference value is located on the side toward 0 with respect to a predetermined value serving as a reference; and when the alternation-determining moving difference value has shifted to the side toward 0, switching is effected so as to use the second high concentration threshold in place of the first high concentration threshold.

Since the above-operation enables the alternation-determining moving difference value to be calculated by use of sensor output values obtained after the time of switching from the high concentration signal to the low concentration signal, the above-described problem does not occur, and a drop in detection sensitivity stemming from a hysteresis phenomenon can be prevented without fail.

Alternatively, in the gas detection apparatus, from the time of switching from the high concentration signal to the low concentration signal, the alternation-determining moving-difference-value calculation means starts the calculation of the alternation-determining moving difference value, or resumes the calculation of the alternation-determining moving difference value without use of sensor output values obtained before the time of switching, wherein during a period from the time of switching up to a point in time at which obtainment of sensor output values corresponding to the predetermined number (i) of cycles is completed, a value obtained through multiplying, by i/b, the difference between a sensor output value obtained at the time of switching and a sensor output value obtained b cycles after the time of switching is used as the alternation-determining moving difference value in respective cycles.

As described already, in the gas detection apparatus, which utilizes the alternation-determining moving difference value, if successive calculation of the alternation-determining moving difference value is performed continuously before the time of switching from the high concentration signal to the low concentration signal, depending on the manner of change of the sensor output value or the value of i (number of cycles), the moving difference value calculated at the time of switching may fail to assume a magnitude that indicates occurrence of a hysteresis phenomenon even though a hysteresis phenomenon has occurred.

In the gas detection apparatus of the present invention, from the time of switching from the high concentration signal to the low concentration signal, calculation of the alternation-determining moving difference value is started or resumed. Incidentally, during the period from the start or resumption up to a point in time at which obtainment of sensor output values corresponding to the predetermined number (i) of cycles is completed, naturally, no moving difference value can be obtained. However, in the present gas detection apparatus, a value (DI(n) = (S(n) - S(n+b))·i/b) obtained through multiplying, by i/b, the difference between a sensor output value (S(n) obtained at the time of switching and a sensor output value (S(n+b), b = 1, 2, ..., i) obtained in each subsequent cycle is used as the alternation-determining moving difference value in respective cycles, whereby obtainment of the alternation-determining moving difference value is started when one cycle has passed from the time of switching from the high concentration signal to the low concentration signal. Then, the moving-difference-value determining means determines whether the alternation-determining moving difference value is located on the side toward 0 with respect to a predetermined value serving as a reference; and when the alternation-determining moving difference value had shifted to the side toward 0, switching is effected so as to use the second high concentration threshold in place of the first high concentration threshold.

Since the above-operation enables the alternation-determining moving difference value to be calculated by use of sensor output values obtained after the time of switching from the high concentration signal to the low concentration signal, the above-described problem does not occur, and a drop in detection sensitivity stemming from a hysteresis phenomenon can be prevented without fail.

Moreover, in the case where the difference value (S(n) - S(n+b)) is used as the moving difference value without being multiplied by i/b, the shorter the period between the switching and the calculation point (the smaller the value of b), the smaller the moving difference value. Therefore, a restriction is imposed on selection of a predetermined value which is compared with the moving difference value. In contrast, when a value obtained through multiplying the difference value by i/b is used as a moving difference value as in the case of the present invention, in each cycle, there can be obtained a value corresponding to a moving difference value obtained in the case where a change that occurred during a period of b cycles continues over i cycles, so that the selection of a reference value which is compared with the moving difference value can be performed more properly.

Preferably, the gas detection sensor comprises: determination means for determining whether the present sensor output value has shifted to the side toward the low concentration direction with respect to a cancel threshold which is an intermediate value between a start-point sensor output value and a peak sensor output value, the start-point sensor output value being a sensor output value obtained at the beginning of a high-concentration-signal generation period immediately before the switching from the high concentration signal to the low concentration signal, and the peak sensor output value being a value that shifts in the high concentration direction to the greatest degree among sensor output values obtained in the high-concentration-signal generation period, wherein the concentration increase detection means uses, in place of the first high concentration threshold, the second high concentration threshold as the high concentration threshold when the present sensor output value has shifted to the side toward the low concentration direction with respect to the cancel threshold.

As described above, in the case where the concentration of the specific gas does not increase again after the switching from the high concentration signal to the low concentration signal, a hysteresis phenomenon gradually ends with elapse of time, the change in the sensor output value in the low concentration direction decreases, and a sensor output value generally corresponding to a decreased concentration of the specific gas is obtained.

If, during that period, there does not occur a drift phenomenon in which the sensor resistance and the sensor output value change with humidity or temperature irrespective of change in gas concentration, the obtained sensor output value is considered to assume the same magnitude as that of the start-point sensor output value obtained at the beginning of a high-concentration-signal generation period immediately before the switching from the high concentration signal to the low concentration signal. Accordingly, there can be employed a method in which when the present sensor output value obtained after the switching from the high concentration signal to the low concentration signal has shifted to the side toward the low concentration direction with respect to the start-point sensor output value, a hysteresis phenomenon is considered to have ended, and the second high concentration threshold is used in place of the first high concentration threshold.

However, when such a method is employed, when a drift phenomenon occurs, depending on the direction of change stemming from the drift, the sensor output value may fail to return to a value substantially equal to the sensor output value at the time when the high concentration signal was generated. In particular, when the peak sensor output value obtained in the high-concentration-signal generation period is large, the length of the hysteresis period increases. In such a case, the sensor output value is likely to be influenced by the drift, thereby increasing the possibility that the sensor output value fails to return to a value substantially equal to the sensor output value at the time when the high concentration signal was generated. As a result, in some cases, the present sensor output value fails to shift to the side toward the low concentration direction with respect to the start-point sensor output value. In other words, there may occur a case in which the first high concentration threshold is used continuously even after the hysteresis phenomenon has ended.

In the gas detection apparatus of the present invention, determination means determines whether the present sensor output value has shifted to the side toward the low concentration direction with respect to the cancel threshold, which is an intermediate value between the start-point sensor output value and the peak sensor output value, and when the present sensor output value has shifted to the side toward the low concentration direction with respect to the cancel threshold, the second high concentration threshold is used in place of the first high concentration threshold. As described above, since the cancel threshold is set to a value between the start-point sensor output value and the peak sensor output value, the sensitized period during which the first high concentration threshold is used is rendered a finite period.

Preferably, in the gas detection sensor, the cancel threshold is a value which is shifted toward the peak sensor output value from the start-point sensor output value by an amount obtained through division by a of the difference between the peak sensor output value and the start-point sensor output value, where a > 1.

In the gas detection apparatus of the present invention, the cancel threshold becomes a value corresponding to the start-point sensor output value and the peak sensor output value. Although the start-point sensor output value and the peak sensor output value change from time to time, by virtue of the above-described operation, a proper cancel threshold can always be obtained.

Notably, the magnitude of the numerical value a may be determined properly in consideration of a gas sensor element to be used in the gas detection apparatus, cycle interval, influence of drift, and other factors.

According to another aspect, the means for solution is a gas detection apparatus using a gas sensor element whose sensor resistance changes in accordance with concentration of a specific gas, comprising: acquisition means for acquiring a sensor output value at predetermined cycle intervals, the sensor output value corresponding to the sensor resistance; and concentration detection means for detecting a change in the concentration of the specific gas and for generating a low concentration signal when the concentration of the specific gas is low and a high concentration signal when the concentration of the specific gas is high, and wherein the concentration detection means comprises end-timing determining means for determining the end of a sensitized period, wherein of a period in which the low concentration signal is generated, in a period between the time of switching from the high concentration signal to the low concentration signal and the end of the sensitized period, the high concentration signal is caused to be generated, in place of the low concentration signal, in response to a smaller change in the sensor output value in the high concentration direction, as compared with the case where the high concentration signal is generated, in place of the low concentration signal, in response to a change in the sensor output value in the high concentration direction, in the remaining portion of the period in which the low concentration signal is generated.

In the gas detection apparatus of the present invention, in the sensitized period, the high concentration signal can be generated in response to a smaller change in the sensor output value in the high concentration direction, as compared with other periods. Therefore, in the sensitized period, an increase in gas concentration can be detected without fail, while drop in detection sensitivity stemming from a hysteresis phenomenon is compensated.

According to another aspect, the means for solution is a gas detection apparatus using a gas sensor element whose sensor resistance changes in accordance with concentration of a specific gas, comprising: acquisition means for acquiring a sensor output value at predetermined cycle intervals, the sensor output value corresponding to the sensor resistance; and concentration detection means for detecting a change in the concentration of the specific gas and for generating a low concentration signal when the concentration of the specific gas is low and a high concentration signal when the concentration of the specific gas is high, wherein the concentration detection means comprises concentration increase detection means for generating the high concentration signal in place of the low concentration signal when, in a period in which the low concentration signal is generated, the sensor output value shifts toward a high concentration direction with respect to the reference value by an amount equal to or greater than a high concentration threshold, concentration decrease detection means for generating the low concentration signal in place of the high concentration signal when, in a period in which the high concentration signal is generated, the sensor output value shifts toward a low concentration direction with respect to the reference value by an amount equal to or greater than a low concentration threshold, and end-timing determining means for determining the end of a sensitized period, and wherein of a period in which the low concentration signal is generated, in a period between the time of switching from the high concentration signal to the low concentration signal and the end of the sensitized period, the high concentration signal is caused to be generated, in place of the low concentration signal, in response to a smaller change in the sensor output value in the high concentration direction, as compared with the case where the high concentration signal is generated, in place of the low concentration signal, in response to a change in the sensor output value in the high concentration direction, in the remaining portion of the period in which the low concentration signal is generated.

In the gas detection apparatus of the present invention, in the sensitized period, the high concentration signal can be generated in response to a smaller change in the sensor output value in the high concentration direction, as compared with other periods. Therefore, in the sensitized period, an increase in gas concentration can be detected without fail, while drop in detection sensitivity stemming from a hysteresis phenomenon is compensated.

According to another aspects, the means for solution is a gas detection apparatus using a gas sensor element whose sensor resistance changes in accordance with concentration of a specific gas, comprising: acquisition means for acquiring a sensor output value at predetermined cycle intervals, the sensor output value corresponding to the sensor resistance; and concentration detection means for detecting a change in the concentration of the specific gas and for generating a low concentration signal when the concentration of the specific gas is low and a high concentration signal when the concentration of the specific gas is high, wherein the concentration detection means comprises concentration increase detection means for generating the high concentration signal in place of the low concentration signal when, during a period in which the low concentration signal is generated, the sensor output value shifts toward a high concentration direction with respect to the reference value by an amount equal to or greater than a high concentration threshold, wherein the period during which the low concentration signal is generated includes a sensitized period in which a first high concentration threshold is used as the high concentration threshold and a period which is subsequent to the sensitized period and in which a second high concentration signal is used as the high concentration threshold, and wherein the first high concentration threshold is a value located toward the sensitivity increasing direction with respect to the second high concentration threshold.

Preferably, in the gas detection apparatus, the sensitized period starts at the switching from the high concentration signal to the low concentration signal, and ends after a pint in time when the direction of change of the sensor output value changes from the low concentration direction to the high concentration direction.

Preferably, the gas detection apparatus is incorporated in an automatic ventilation system for a vehicle.

Since this automatic ventilation system for a vehicle uses a gas detection apparatus capable of compensating drop in detection sensitivity for increase in the concentration of the specific gas, which drip occurs stemming from a hysteresis phenomenon, the flap can be opened and closed properly even when such a hysteresis phenomenon occurs. The invention is also directed to methods by which the described apparatus operates. It includes method steps for carrying out every function of the apparatus. Yet, the invention is also directed to a process of manufacturing the described apparatuses. It includes process steps for providing every feature of the apparatus. The method steps of these methods may be performed by way of hardware components, a computer programmed by appropriate software, by any combination of the two or in any other manner.

### BRIEF DESCRIPTION OF THE DRAWINGS

Some of the above indicated and other more detailed aspects of the invention will be described in the following description and partially illustrated with reference to the figures. As used herein, like numerals throughout the various figures represent the same or equivalent features of the present invention. Therein:
FIG. 1 shows an explanatory diagram relating to the first embodiment, etc., and schematically showing a gas detection apparatus and a vehicle automatic ventilation system;
FIG. 2 shows an explanatory diagram relating to the first embodiment, etc., and showing a control flow of the vehicle automatic ventilation system;
FIG. 3 shows an explanatory diagram relating to the first embodiment, etc., and showing a control flow of the microcomputer incorporated in the gas detection apparatus;
FIG. 4 shows an explanatory diagram relating to the first embodiment, etc., and showing the high-concentration-threshold setting routine among the control routines performed in the microcomputer;
FIG. 5 shows an explanatory diagram relating to the first embodiment, etc., and showing the gas-concentration-change detecting routine among the control routines performed in the microcomputer;
FIG. 6 shows an explanatory diagram relating to the first embodiment and the first modification, and showing the hysteresis-processing-data obtaining routine among the control routines performed in the microcomputer;
FIG. 7 shows an explanatory diagram relating to the first embodiment and the first modification, and showing the sensitized-period processing routine among the control routines performed in the microcomputer;

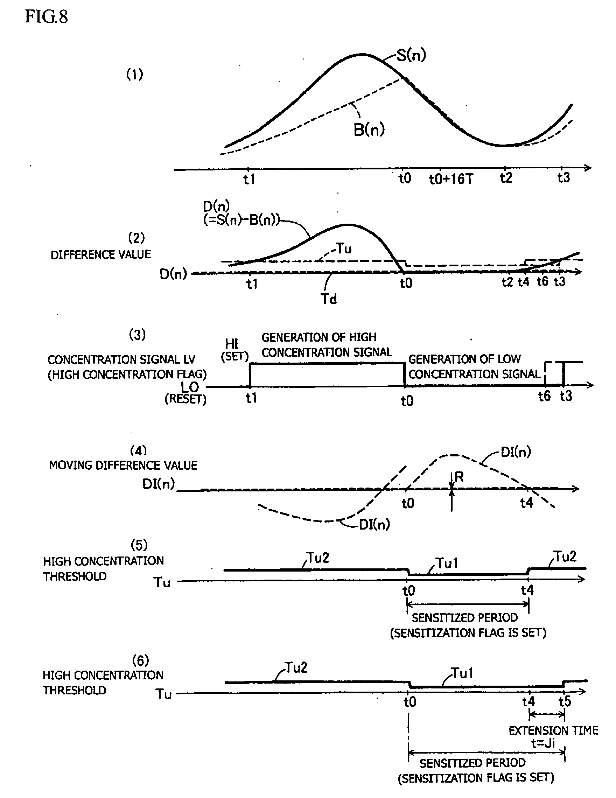

FIG. 8 shows explanatory graphs relating to the first embodiment, wherein (1) shows changes in sensor output value S(n) corresponding to an increases and a decrease in the concentration of NOx, as well as changes in base value B(n) that follows the sensor output value S(n); (2) shows changes in difference value D(n) and changes in concentration threshold; (3) shows changes in the set state of the high concentration flag; (4) shows changes in moving difference value DI(n) obtained from the sensor output value S(n); and (5) shows changes in high concentration threshold Tu. Further, (6), replacing (5), shows changes in high concentration threshold Tu in the first modification;
FIG. 9 shows explanatory graphs relating to the first embodiment and the first modification, wherein (1) shows a sensor output value S(n) and the manner of obtaining a moving difference value DI(n) on the basis of the sensor output value S(n); and (2) shows changes in the thus-obtained moving difference value DI(n);
FIG. 10 shows an explanatory diagram relating to the second modification, and showing the hysteresis-processing-data obtaining routine among the control routines performed in the microcomputer;
FIG. 11 shows explanatory graphs relating to the second modification, wherein (1) shows a sensor output value S(n) and the manner of obtaining a moving difference value DI(n) on the basis of the sensor output value S(n); and (2) shows changes in the thus-obtained moving difference value DI(n);
FIG. 12 shows an explanatory diagram relating to the second embodiment and the third modification, and showing the hysteresis-processing-data obtaining routine among the control routines performed in the microcomputer;
FIG. 13 shows an explanatory diagram relating to the second embodiment and the third modification, and showing the sensitized-period processing routine among the control routines performed in the microcomputer;
FIG. 14 shows explanatory graphs relating to the second embodiment, wherein (1) shows changes in sensor output value S(n), as well as changes in base value B(n) that follows the sensor output value S(n); (2) shows changes in difference value D(n) and changes in concentration threshold; (3) shows changes in the set state of the high concentration flag; (4) shows changes in moving difference value DM(n) obtained from the sensor output value S(n); and (5) shows changes in high concentration threshold Tu;
FIG. 15 shows explanatory graphs relating to the second embodiment, wherein (1) shows a sensor output value S(n) and the manner of obtaining a moving difference value DI(n) on the basis of the sensor output value S(n); and (2) shows changes in the thus-obtained moving difference value DI(n);
FIG. 16 shows an explanatory diagram relating to the third modification, and showing the high-concentration-threshold setting routine among the control routines performed in the microcomputer;
FIG. 17 shows an explanatory diagram relating to the fourth modification, and showing the sensitized-period processing routine among the control routines performed in the microcomputer;
FIG. 18 shows explanatory graphs relating to the fourth modification, wherein (1) shows changes in sensor output value S(n), as well as changes in base value B(n) that follows the sensor output value S(n); (2) shows changes in difference value D(n) and changes in concentration threshold; (3) shows changes in the set state of the high concentration flag; and (4) shows changes in moving difference values DM(n) and DJ(n) obtained from the sensor output value S(n);
FIG. 19 shows an explanatory diagram relating to the fourth modification, and showing changes in high concentration threshold Tu in a sensitized period;
FIG. 20 shows an explanatory diagram relating to the third embodiment and the fifth modification, and showing the hysteresis-processing-data obtaining routine among the control routines performed in the microcomputer;
FIG. 21 shows an explanatory diagram relating to the third embodiment, and showing the sensitized-period processing routine among the control routines performed in the microcomputer;
FIG. 22 shows explanatory graphs relating to the third embodiment, wherein (1) shows changes in sensor output value S(n), as well as changes in base value B(n) that follows the sensor output value S(n); (2) shows changes in difference value D(n), changes in concentration threshold, and the maximum difference value Dmax; (3) shows changes in the set state of the high concentration flag; and (4) shows changes in high concentration threshold Tu;
FIG. 23 shows an explanatory diagram relating to the fifth modification, and showing the sensitized-period processing routine among the control routines performed in the microcomputer;
FIG. 24 shows explanatory graphs relating to the fifth modification, wherein (1) shows changes in sensor output value S(n), as well as changes in base value B(n) that follows the sensor output value S(n); (2) shows changes in difference value D(n), changes in concentration threshold, and the maximum difference value Dmax; (3) shows changes in the set state of the high concentration flag; and (4) shows changes in high concentration threshold Tu;
FIG. 25 shows an explanatory diagram relating to the fourth embodiment, and showing the hysteresis-processing-data obtaining routine among the control routines performed in the microcomputer;
FIG. 26 shows an explanatory diagram relating to the fourth embodiment, and showing the sensitized-period processing routine among the control routines performed in the microcomputer;
FIG. 27 shows explanatory graphs relating to the fourth embodiment, wherein (1) shows changes in sensor output value S(n), as well as changes in base value B(n) that follows the sensor output value S(n); (2) shows changes in difference value D(n) and changes in concentration threshold; (3) shows changes in the set state of the high concentration flag; and (4) shows changes in high concentration threshold Tu;
FIG. 28 shows an explanatory diagram relating to the sixth modification, and showing the sensitized-period processing routine among the control routines performed in the microcomputer;
FIG. 29 shows explanatory graphs relating to the sixth modification, wherein (1) shows changes in sensor output value S(n), as well as changes in base value B(n) that follows the sensor output value S(n); (2) shows changes in difference value D(n) and changes in concentration threshold; (3) shows changes in the set state of the high concentration flag; and (4) shows changes in high concentration threshold Tu;
FIG. 30 shows an explanatory diagram relating to the fifth embodiment, and showing the hysteresis-processing-data obtaining routine among the control routines performed in the microcomputer;
FIG. 31 shows an explanatory diagram relating to the fifth embodiment, and showing the sensitized-period processing routine among the control routines performed in the microcomputer;
FIG. 32 shows explanatory graphs relating to the fifth embodiment, wherein (1) shows changes in sensor output value S(n), as well as changes in base value B(n) that follows the sensor output value S(n); (2) shows changes in difference value D(n) and changes in concentration threshold; (3) shows changes in the set state of the high concentration flag; (4) shows changes in difference value sum SD; and (5) shows changes in high concentration threshold Tu;
FIG. 33 shows an explanatory diagram relating to the sixth embodiment, the seventh embodiment, and the seventh modification, and showing the hysteresis-processing-data obtaining routine among the control routines performed in the microcomputer;
FIG. 34 shows an explanatory diagram relating to the sixth embodiment, and showing the sensitized-period processing routine among the control routines performed in the microcomputer;
FIG. 35 shows explanatory graphs relating to the sixth embodiment, wherein (1) shows changes in sensor output value S(n), as well as changes in base value B(n) that follows the sensor output value S(n); (2) shows changes in difference value D(n) and changes in concentration threshold; (3) shows changes in the set state of the high concentration flag; and (4) shows changes in high concentration threshold Tu;
FIG. 36 shows an explanatory diagram relating to the seventh modification, and showing the sensitized-period processing routine among the control routines performed in the microcomputer;
FIG. 37 shows an explanatory graphs relating to the seventh modification, wherein (1) shows changes in sensor output value S(n), as well as changes in base value B(n) that follows the sensor output value S(n); (2) shows changes in difference value D(n) and changes in concentration threshold; (3) shows changes in the set state of the high concentration flag; and (4) shows changes in high concentration threshold Tu;
FIG. 38 shows an explanatory diagram relating to the seventh embodiment, and showing the sensitized-period processing routine among the control routines performed in the microcomputer; and
FIG. 39 shows explanatory graphs relating to the seventh embodiment, wherein (1) shows changes in sensor output value S(n), changes in base value B(n) that follows the sensor output value S(n), start-point sensor output value Sb, and peak sensor output value Smax, difference MD therebetween, and cancel threshold Sb + MD/a; (2) shows changes in difference value D(n) and changes in concentration threshold; (3) shows changes in the set state of the high concentration flag; and (4) shows changes in high concentration threshold Tu.

### Detailed Description of the Invention

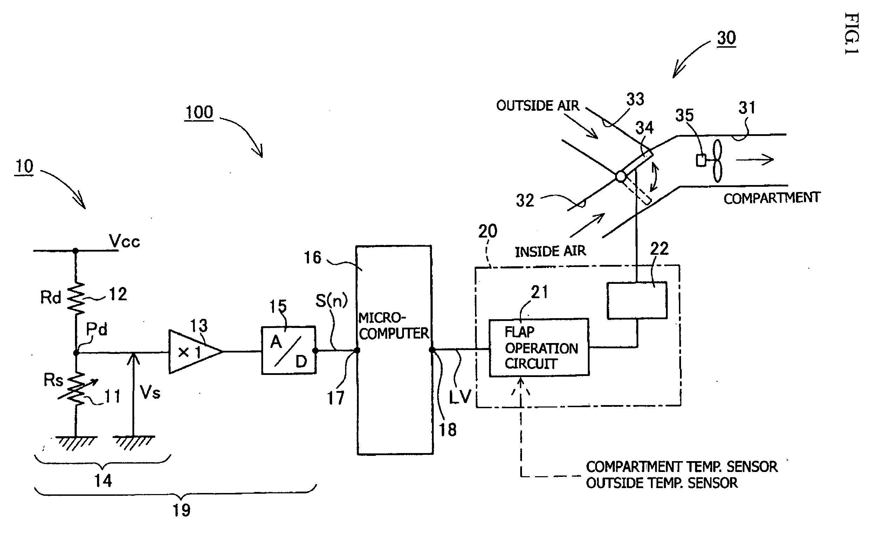

A first embodiment of the present invention will now be described with reference to FIGs. 1 through 8. FIG. 1 includes a circuit diagram and a block diagram of a gas detection apparatus 10 according to the first embodiment, and a schematic representation of the structure of a vehicle automatic ventilation system 100 incorporating the gas detection apparatus 10. The system 100 includes the gas detection apparatus 10, which outputs a concentration signal LV in accordance with the concentration of a specific gas; a ventilation system 30, in which a flap 34 is rotated to thereby connect a duct 31 to either a duct 32 for introducing inside air or a duct 33 for introducing outside air; and an electronic control assembly 20 for controlling the flap 34 of the ventilation system 30 in accordance with the concentration signal LV.

The gas detection apparatus 10 will now be described. The gas detection apparatus 10 includes a gas sensor element 11 formed of an oxide semiconductor. The gas sensor element 11 responds to an oxidative gas component such as NOₓ contained in a measurement gas (air in the present embodiment), and the sensor resistance Rs of the sensor element 11 increases in response to an increase in the concentration of the oxidative gas component. The gas sensor element 11 is provided in an automobile, outside the passenger compartment thereof.

A sensor output value S(n) is obtained by means of the gas sensor element 11 and a sensor output acquisition circuit 19 including a sensor resistance conversion circuit 14, a buffer 13, and an A/D conversion circuit 15. The sensor resistance conversion circuit 14 outputs a sensor output electric potential Vs corresponding to the sensor resistance Rs of the gas sensor element 11. Specifically, the sensor output electric potential Vs at a node Pd, which is obtained by dividing a power supply voltage Vcc by means of the gas sensor element 11 and a detection resistor 12 having a resistance of Rd, is output via the buffer 13. Therefore, in the sensor resistance conversion circuit 14, the sensor resistance Rs and hence the sensor output electric potential Vs increase in accordance with an increase in the concentration of an oxidative gas such as NOₓ.

A signal output from the buffer 13 (the sensor output electric potential Vs) is input to the A/D conversion circuit 15. At predetermined cycle intervals, the A/D conversion circuit 15 converts the signal to a digital signal that represents a present sensor output value S(n). The digital signal is output from the A/D conversion circuit 15 and then input to an input terminal 17 of a microcomputer 16. Reference letter "n" denotes an integer for expressing chronological order.

Therefore, in the first embodiment, the term "high concentration direction" used in relation to the sensor resistance Rs refers to a direction in which the sensor resistance Rs increases. Further, since the sensor output value S(n) increases with concentration of the oxidative gas, the term "high concentration direction" used in relation to the sensor output value S(n) refers to a direction in which the sensor output value S(n) increases. Further, as the sensor output value S(n) increases with concentration of the oxidative gas, a base value B(n) and a difference value D(n), which will be described later, increase. Therefore, the term "high concentration direction" used in relation to the base value B(n) or the difference value D(n) refers to a direction in which the base value B(n) or the difference value D(n) increases. Meanwhile, needless to say, the term "low concentration direction" refers to respective directions opposite the above-described corresponding directions. These same conventions also apply in second to eighth embodiments and first to ninth modifications, which will be described later.

The concentration signal LV (high concentration signal or low concentration signal) for controlling the electronic control assembly 20 is output from an output terminal 18 of the microcomputer 16. The electronic control assembly 20 controls the flap 34 of the ventilation system 30 for controlling recirculation of air inside the automobile and introduction of outside air. Specifically, in the present embodiment, the ventilation system 30 controls the flap 34 such that, in terms of air flow, the inside air introduction duct 32 or the outside air introduction duct 33 is connected to the duct 31, which is connected to the compartment of the automobile.

A flap operation circuit 21 of the electronic control assembly 20 operates an actuator 22 in accordance with the concentration signal LV output from the output terminal 18 of the microcomputer 16 (in the first embodiment, the concentration signal LV showing whether the concentration of an oxidative gas component (e.g., NOₓ) increases or decreases), to thereby rotate the flap 34 such that the inside air introduction duct 32 or the outside air introduction duct 33 is connected to the duct 31.

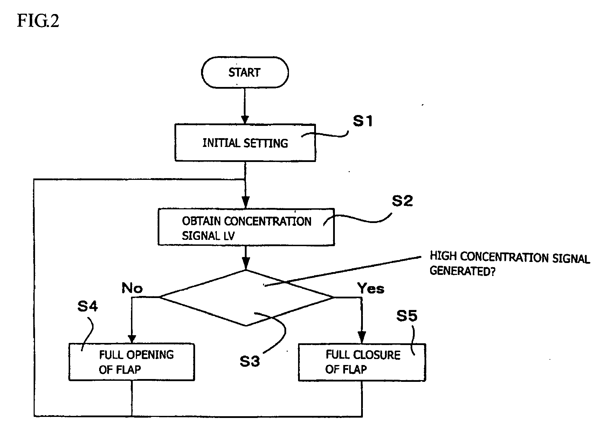

As shown in a flowchart of FIG. 2, after having performed initial setting in step S1, the flap operation circuit 21 obtains the concentration signal LV in step S2, and in step S3, determines whether or not the level of the concentration signal LV is high; i.e., whether or not a high concentration signal is being generated. In the case where the result of determination is No; i.e., in the case where a low concentration signal is being generated, the concentration of a specific gas under measurement is low. Therefore, in step S4, the flap operation circuit 21 instructs full opening of the flap 34. As a result, the flap 34 is rotated so as to connect the outside air introduction duct 33 to the duct 31, thereby introducing outside air into the compartment of the automobile. Meanwhile, in the case where the result of determination is Yes in step S3; i.e., in the case where a high concentration signal is being generated, the concentration of the specific gas is high. Therefore, in step S5, the flap operation circuit 21 instructs full closure of the flap 34. As a result, the flap 34 is rotated so as to connect the inside air introduction duct 32 to the duct 31, thereby stopping introduction of outside air and recirculating the air inside the compartment of the automobile.

A fan 35 for feeding air under pressure is provided in the duct 31. The flap operation circuit 21 is configured so as to rotate the flap 34 in accordance with merely the concentration signal LV; however, the flap operation circuit 21 may be configured such that the flap 34 is rotated by means of a microcomputer, on the basis of not only the concentration signal LV output from the gas detection apparatus 10 but also data obtained from, for example, a compartment temperature sensor, a humidity sensor, or an outside temperature sensor (see a broken line in FIG. 1).

The microcomputer 16 processes the sensor output value S(n) input through the input terminal 17, in accordance with the below-described processing flow, to thereby detect changes in the concentration of the oxidative gas component on the basis of the sensor resistance Rs of the gas sensor element 11 or change in the sensor resistance Rs. Although not illustrated in detail in FIG. 1, the microcomputer 16, which has a known configuration, includes a microprocessor for performing calculations, RAM for temporarily storing programs and data, and ROM for storing programs and data. The microcomputer 16 may incorporate the buffer 13 and the A/D conversion circuit 15.

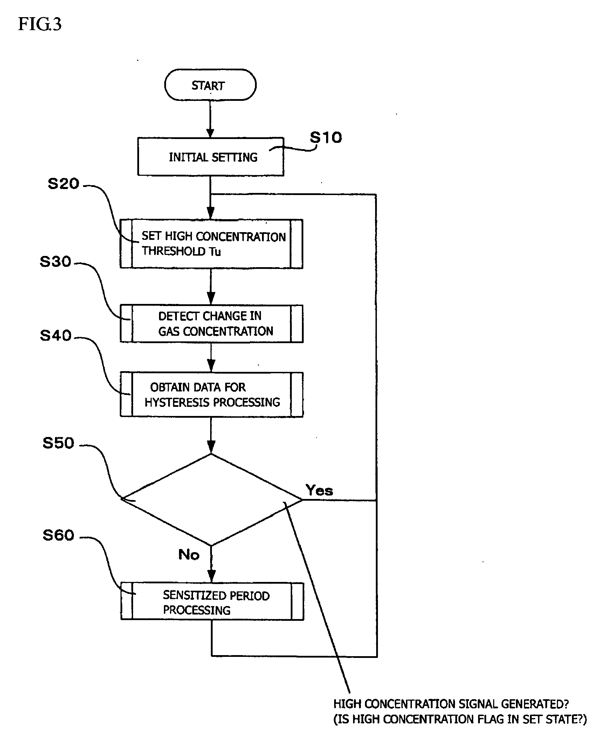

Operation of the microcomputer 16 will next be described with reference to a flowchart of FIG. 3. When the engine of the automobile is started, the present control system starts. After the gas sensor element 11 is activated, in step S10, the microcomputer 16 performs initial setting. The microcomputer 16 stores, as a base value B(0), a sensor output value S(0) at a point in time when the gas sensor element 11 has entered an activated state (B(0) = S(0)). Furthermore, the microcomputer 16 generates a concentration signal LV of low level as a low concentration signal.

In the next step, S20, the microcomputer 16 sets a high concentration threshold Tu. In subsequent step S30, the microcomputer 16 detects a change in gas concentration. As will be described later, in the subroutine of this step S30, the microcomputer 16 detects an increase or decrease in the concentration of the oxidative gas component, and in accordance with the detection result generates a high concentration signal or a low concentration signal. In the next step, S40, the microcomputer 16 obtains data for hysteresis processing. Further, in step S50, the microcomputer 16 determines whether or not the high concentration signal is being generated; i.e., whether or not a high concentration flag is in the set state. In the case where the result of determination is Yes; i.e., in the case where the high concentration signal is being generated, the microcomputer 16 returns to step S20. In the case where the result of determination is No; i.e., in the case where the low concentration signal is being generated, the microcomputer 16 proceeds to step S60 and performs processing for a sensitized period. During a period in which the low concentration signal is being generated; i.e., some period of time after the switching from the high concentration signal to the low concentration signal, there is a great possibility that a hysteresis phenomenon is occurring. During a period in which a hysteresis phenomenon continues, the detection sensitivity for concentration increase is rendered high as compared with ordinary periods, in order to enable generation of the high concentration signal in response to a slight change of the sensor output value S(n) in the high concentration direction. As will be described later, in the subroutine of step S60, the microcomputer 16 determines whether or not a hysteresis phenomenon is occurring, by use of the data for hysteresis processing and in accordance with a predetermined method, and sets or resets a sensitization flag. Subsequently, the microcomputer 16 returns to step S20.

When the sensitization flag is in the set state, in step S20, the microcomputer 16 changes the high concentration threshold Tu to a value offset toward a sensitivity increasing direction in order to increase the detection sensitivity for concentration increase. As will be described later, when the concentration of the oxidative gas increases, the difference value D(n) increases. Further, the high concentration threshold Tu is compared with the difference value D(n), and a determination is made as to whether or not the high concentration flag is to be set. Therefore, in the first embodiment, rendering the high concentration threshold Tu to a smaller value corresponds to changing the high concentration threshold Tu in the sensitivity increasing direction.

Thus, during a sensitized period, the high concentration threshold Tu is changed to a value toward the sensitivity increasing direction; i.e., the high concentration threshold Tu is changed to a smaller value, as compared with other periods, to thereby increase the detection sensitivity for concentration increase.

Notably, the above also applies in second to eighth embodiments and first to ninth modifications.

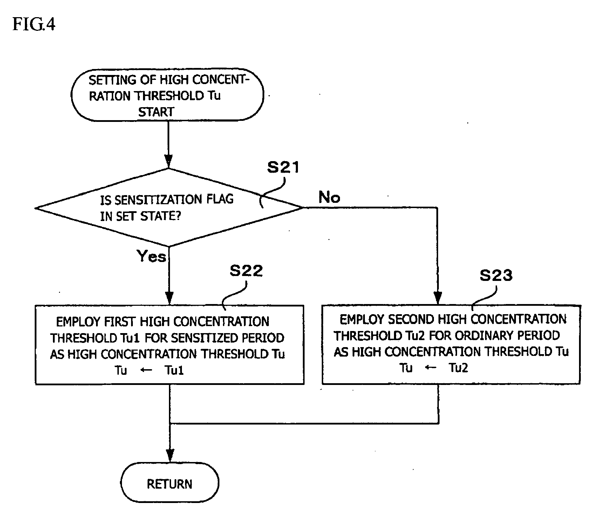

Next, there will be described the subroutine of step S20, which is shown in FIG. 4 and is adapted to set the high concentration threshold Tu. In this subroutine, in first step S21, the microcomputer 16 judges whether or not the sensitization flag is in the set state. In the case where the result of determination is No; i.e., in the case where the sensitization flag is in the reset state, the microcomputer 16 proceeds to step S23 in order to employ, as the high concentration threshold Tu, a second high concentration threshold Tu2 for ordinary period, and then returns to the main routine. In the case where the result of determination is Yes; i.e., in the case where the sensitization flag is in the set state, the microcomputer 16 proceeds to step S22 in order to employ, as the high concentration threshold Tu, a first high concentration threshold Tu1 for sensitized periods. Subsequently, the microcomputer 16 returns to the main routine. The first high concentration threshold Tu1 is a value offset from the second high concentration threshold Tu2 in the sensitivity increasing direction; i.e., a value lower than the second high concentration threshold Tu2.

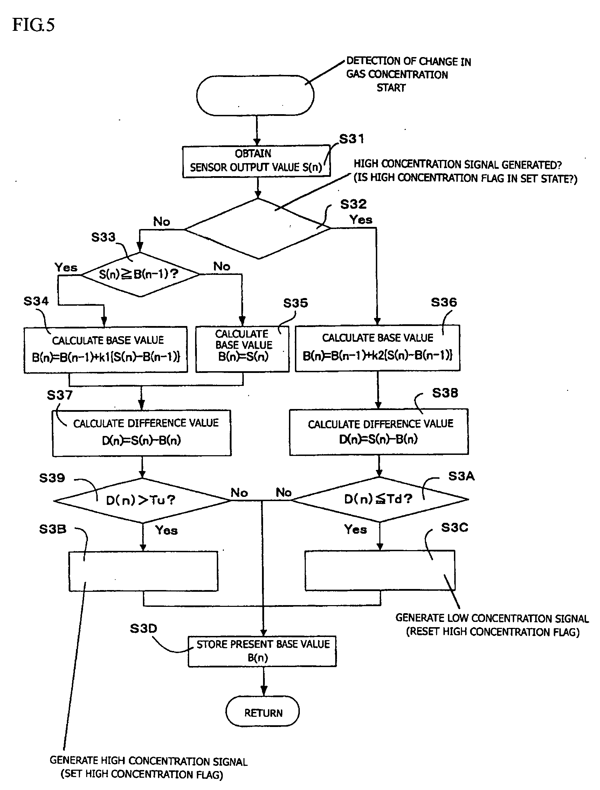

Next, there will be described the subroutine of step S30, which is shown in FIG. 5 and is adapted to detect a change in gas concentration. In the first step S31, the microcomputer 16 obtains a sensor output value S(n). Specifically, the microcomputer 16 sequentially reads, at predetermined cycle intervals, a sensor output value S(n) obtained through A/D conversion of the sensor output electric potential Vs. Subsequently, in step S32, the microcomputer 16 determines whether or not the level of the concentration signal LV is high; i.e., whether or not generation of the high concentration signal continues. Specifically, the microcomputer 16 determines whether or not the high concentration flag is in the set state, the high concentration flag being set or reset simultaneously with switching of the concentration signal LV in step S3B or step S3C, which will be described later. In the case where the result of determination is No; i.e., in the case where the high concentration flag has been reset because of the low level of the concentration signal LV (i.e., generation of a low concentration signal), the microcomputer 16 proceeds to step S33. In contrast, in the case where the result of determination is Yes; i.e., in the case where the high concentration flag has been set because of the high level of the concentration signal LV (i.e., generation of a high concentration signal), the microcomputer 16 proceeds to step S36.

In step S33, the microcomputer 16 determines whether or not the sensor output value S(n) is equal to or greater than a base value B(n-1) calculated in the preceding cycle. In the case where S(n) ≥ B(n-1) (the result of determination is Yes), the microcomputer 16 proceeds to step S34. In contrast, in the case where S(n) < B(n-1) (the result of determination is No), the microcomputer 16 proceeds to step S35.

In step S34, the microcomputer 16 calculates a present base value B(n) from the preceding base value B(n-1) and the present sensor output value S(n) by use of the following Expression (1), and then proceeds to step S37. Expression (1) : B(n) = B(n-1) + k1{S(n) - B(n-1)}, where the first coefficient k1 satisfies the relation: 0 < k1 < 1.

When the coefficient k1 satisfies the relation 0 < k1 < 1, the present base value B(n) calculated by use of the above Expression (1) changes to follow the present sensor output value S(n), and changes more slowly than does the sensor output value S(n).

As a result, a difference is produced between S(n) and B(n). This characteristics enables detection of an increase in the concentration of the specific gas by use of a difference value D(n) calculated in the below-described step S37. In other words, if the present routine is configured to generate a high concentration signal when the difference value D(n) is greater than a positive high concentration threshold Tu, an increase in the concentration of the specific gas can be detected.

In contrast, in step S35, the microcomputer 16 substitutes the present sensor output value S(n) for the base value B(n) (B(n) = S(n)), and proceeds to step S37. That is, when the present sensor output value S(n) is smaller than the preceding base value B(n-1), the microcomputer 16 renders the base value B(n) equal to the present sensor output value S(n) in order to cause the base value to follow the sensor output value without delay.

Meanwhile, in step S36, the microcomputer 16 calculates the base value B(n) from the preceding base value B(n-1) and the sensor output value S(n) by use of Expression (2), which is similar to that used in step S34, and then proceeds to step S38. Expression (2): B(n) = B (n-1) + k2{S(n) - B(n-1)}, where the second coefficient k2 satisfies the relation: 0 < k2 < k1 <1.

The speed at which the base value B(n) follows the sensor output value S(n) varies depending on the values of the coefficients k1 and k2 used. When the first coefficient k1, which assumes a relatively large value (k1 > k2), is used (step S34), the base value B(n) follows the sensor output value S(n) at relatively high speed, although with a slight delay. In contrast, when the above calculation is performed by use of the second coefficient k2, which assumes a relatively small value (k2 < k1) (step S36), the base value B(n) changes slowly, and follows the sensor output value S(n) slowly.

In step S37, subsequent to step S34 or S35, the microcomputer 16 calculates a difference value D(n) in accordance with Expression (3): D(n) = S(n) - B(n). In step S39, the microcomputer 16 compares the difference value D(n) with the high concentration threshold Tu.

When the relation D(n) > Tu is satisfied (the result of determination is Yes), the microcomputer 16 proceeds to step S3B. Since the result of determination in step S37 becomes Yes when the relation D(n) > Tu is satisfied in a state in which the low concentration signal is being generated (No in step S32), this indicates an increase in the difference between the sensor output value S(n) and the base value B(n), which follows the sensor output value S(n) with delay. In other words, the sensor output value S(n) is considered to have increased at a speed greater than the following speed of the base value B(n), because of an increase in the concentration of the specific gas (oxidative gas).

Therefore, in step S3B, the microcomputer 16 generates a high concentration signal in place of the low concentration signal. Specifically, the microcomputer 16 renders the level of the concentration signal LV high. Further, the microcomputer 16 sets the high concentration flag.

Meanwhile, the relation D(n) ≤ Tu is satisfied (the result of determination is No), the microcomputer 16 proceeds to step S3D. The result of determination in step S37 becomes No when the relation D(n) ≤ Tu is satisfied in a state in which the low concentration signal is being generated (No in step S32). This indicates only a slight increase in the difference between the sensor output value S(n) and the base value B(n), which follows the sensor output value S(n) with delay. In other words, the concentration of the specific gas (oxidative gas) is considered to remain low. Alternatively, the base value B(n) is considered to have been rendered equal to the present sensor output value S(n) in step S35, because the gas concentration decreases continuously.

Therefore, the microcomputer 16 proceeds to step S3D, while maintaining the low concentration signal.

In step S38, subsequent to step S36, the microcomputer 16 also calculates a difference value D(n) in accordance with Expression (3): D(n) = S(n) - B(n). In step S3A, the microcomputer 16 compares the difference value D(n) with the low concentration threshold Td. Notably, the low concentration threshold Td is smaller than the high concentration threshold Tu used in step S39 (Tu > Td).

When the relation D(n) ≤ Td is satisfied (the result of determination is Yes), the microcomputer 16 proceeds to step S3C. The reason for use of two thresholds; i.e., high concentration threshold Tu and low concentration threshold Td, is to prevent generation of chatter, which would otherwise occur at time of signal switching between the low concentration signal and the high concentration signal. The result of determination in step S38 becomes Yes when the relation D(n) ≤ Td is satisfied in a state in which the high concentration signal is being generated (Yes in step S32). This indicates a decrease in the difference between the sensor output value S(n) and the base value B(n), which was calculated in step S36 and reflects, to some degree, the past state (i.e., the state before the concentration of the oxidative gas has increased); i.e., a sufficient decrease in the concentration of the oxidative gas.

Therefore, in step S3C, the microcomputer 16 generates a low concentration signal in place of the high concentration signal. Specifically, the microcomputer 16 renders the level of the concentration signal LV low. Further, the microcomputer 16 resets the high concentration flag.

In contrast, the result of determination in step S3A becomes No when the relation D(n) > Td is satisfied in a state in which the high concentration signal is being generated (Yes in step S32). In this case, the difference between the present sensor output value S(n) and the base value B(n) is still large, and the concentration of the oxidative gas is considered to remain high. Therefore, the microcomputer 16 proceeds to step S3D, while maintaining the high concentration signal.

The microcomputer 16 proceeds from step S39, S3A, S3B, or S3C to step S3D in order to store the present base value B(n) calculated in step S34, S35, or S36, and then returns to the main routine.

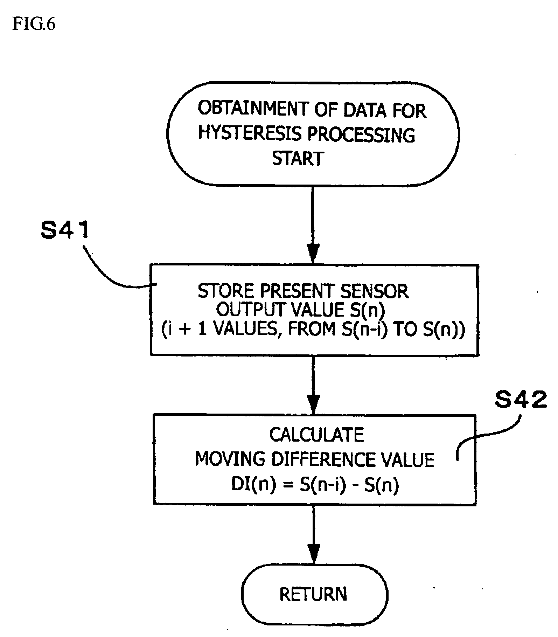

Next, there will be described the subroutine of step S40, which is shown in FIG. 6 and is adapted to obtain data for hysteresis processing. In the first step, S41, the microcomputer 16 stores the present sensor output value S(n) obtained in the above-described step S31. Notably, in the first embodiment, i+1 storage areas are provided in the microcomputer 16, and the microcomputer 16 stores i+1 sensor output values in total; i.e., S(n-i) to S(n). The operation of storing the newly obtained present sensor output value S(n) is preferably performed through overwriting the present sensor output value S(n) in the storage area which stores a sensor output value S(n-(i+1)) obtained in a past cycle that is (i+1) cycles before the present cycle.

In subsequent step S42, the microcomputer 16 calculates an alternation-determining moving difference value DI(n) in accordance with the following Expression (4). Expression (4): DI(n) = S(n-i) - S(n). This moving difference value DI(n) represents the difference between the present sensor output value S(n) and a sensor output value S(n-i) obtained in a past cycle that is i cycles before the present cycle; i.e., a change in the sensor output value during that period. In the first embodiment, by use of this moving difference value DI(n), the microcomputer 16 determines a timing for resetting the sensitization flag in the sensitized period processing of step S60, which will be described in detail below.

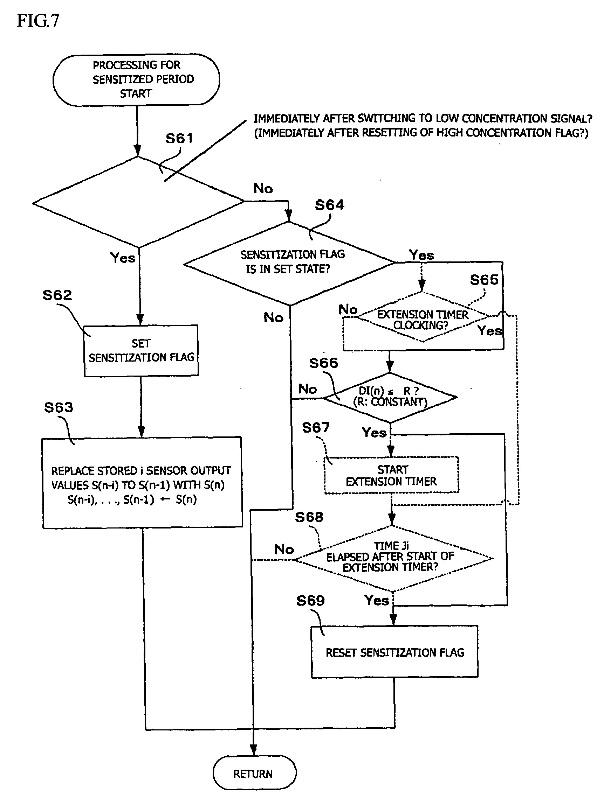

When the microcomputer 16 returns to the main routine after calculating the moving difference value DI(n) in step S42, as described above, in step S50 the microcomputer 16 checks whether or not a high concentration signal is being generated. In the case where the result of determination is Yes; i.e., a high concentration signal is being generated, the microcomputer 16 returns to step S20. In contrast, in the case where the result of determination is No; i.e., a low concentration signal is being generated, the microcomputer 16 proceeds to the subroutine of step S60 shown in FIG. 7, because there is a possibility that the present time is within a hysteresis period.

Next, there will be described the subroutine of step S60, which is shown in FIG. 7 and is adapted to perform processing for sensitized periods. Notably, steps S65, S67, and S68 indicated by broken lines are used in a first modification, which will be described later. In the first step, S61, the microcomputer 16 determines whether the present time is immediately after generation of the low concentration signal in the above-described step S3C in place of the high concentration signal; specifically, whether the present time is immediately after the high concentration flag has been reset.

In the case where the result of determination is Yes; i.e., the present time is immediately after the high concentration flag has been reset in step S3C, the microcomputer 16 proceeds to step S62 in order to set the sensitization flag. In other words, in the first embodiment, the sensitization flag is set unconditionally immediately after the switching from the high concentration signal to the low concentration signal is effected in step S30, because a hysteresis phenomenon is considered to occur, to a greater or lesser extent, immediately after the switching from the high concentration signal to the low concentration signal.

In subsequent step S63, the microcomputer 16 replaces with the present sensor output value S(n) all the past i sensor output values S(n-i) to S(n-1), among the i+1 sensor output values stored in the above-described step S41. Subsequently, the microcomputer 16 returns to the main routine. The step S63 is provided for the following reason. In an example case where the concentration of the specific gas increases and decreases during a period which is about the same as a period required for a predetermined number i of cycles, even through the sensor output value actually fluctuates up and down, in some cases the difference (moving difference value) DI(n) between the present sensor output value S(n) and the sensor output value S(n-i) obtained in a past cycle that is i cycles before the present cycle does not becomes sufficiently large (greater than R). In such a case, even though the detection sensitivity has been lowered for reasons of a hysteresis phenomenon, the result of determination in the below-described step S66 becomes Yes, and the sensitization flag is reset immediately. As a result, the sensitized period cannot be set to a proper length, and thus detection sensitivity may decrease.

In contrast, in the first embodiment, as described above, all the past i sensor output values S(n-i) to S(n-1) are replaced with the present sensor output value S(n). Thus, the moving difference value DI(n) can be calculated by use of sensor output values that are obtained after the time of switching from the high concentration signal to the low concentration signal. Therefore, the above-described problem does not occur, and a decrease in detection sensitivity stemming from a hysteresis phenomenon can be reliably prevented.

When the sensitization flag is in the set state, the result of determination in the above-described step S21 becomes Yes; and in step S22, a first high concentration threshold Tu1 to be used for sensitized periods is employed as the high concentration threshold Tu, to thereby increase the sensitivity in detecting a concentration increase in the gas concentration change detection subroutine of step S30.

In contrast, in the case where the result of determination in step S61 is No; i.e., the present time is not immediately after the high concentration flag has been reset, the microcomputer 16 proceeds to step S64 in order to determine whether the sensitization flag is in the set state. In the case where the sensitization flag is in the set state (the result of the determination is Yes); i.e., the present time is in a sensitized period, the microcomputer 16 proceeds to step S66. In step S66, the microcomputer 16 determines whether the moving difference value DI(n) calculated in step S42 has become smaller than a predetermined value (constant R). As described above, the moving difference value DI(n) represents a change in the sensor output value, from i cycles in the past until the present.

Incidentally, in a case where a hysteresis phenomenon occurs when the concentration of the specific gas (oxidative gas) has decreased, in general, the difference between a theoretical sensor output value corresponding to an actual concentration of the specific gas and an actual sensor output value, which difference is produced stemming from a hysteresis phenomenon, gradually decreases with lapse of time, and finally, the values become equal. Conceivably, molecules of the specific gas adsorbed by the gas sensor element separate from the element with lapse of time, and the number of adsorbed molecules decreases. Therefore, the moving difference value DI(n) that represents a change in the sensor output value still being large means that a hysteresis phenomenon has not ended. Therefore, in the case where the moving difference value DI(n) is greater than the constant R (the result of the determination is No), the microcomputer 16 returns to the main routine. In contrast, in the case where the moving difference value DI(n) is equal to or smaller than the constant R (the result of the determination is Yes), the microcomputer 16 proceeds to step S69 in order to reset the sensitization flag, because the change in the sensor output value decreases, and the hysteresis phenomenon is considered to have ended. Thus, the sensitization flag is reset in order to lower to an ordinary level the detection sensitivity for concentration increase, through the operation in step S23, in which the second high concentration threshold Tu2 to be used in ordinary periods is employed as the high concentration threshold Tu.

In the case where the result of the determination in step S64 is No; i.e., where the sensitization flag is in a reset state, the hysteresis phenomenon is considered to have ended, and the microcomputer 16 returns to the main routine.

In the above-described manner, the gas detection apparatus 10 of the first embodiment can detect reliably, or can detect quickly, an increase in the concentration of the specific gas irrespective of the occurrence of a hysteresis phenomenon.

Next, there will be described the manner in which the respective values are obtained when the sensor output value S(n) changes as indicated by a solid line in FIG. 8(1). The description is provided for the case where the sensor output value S(n) increases, decreases, and then increases again as shown in FIG. 8(1) in response to changes in the concentration of the oxidative gas; i.e., an increase, a decrease, and another increase in concentration. When the sensor output value S(n) changes in such a manner, the base value B(n) changes as indicated by a broken line, and the difference D(n) between these values (= S(n) - B(n)) changes as shown in FIG. 8(2). Notably, it is assumed that at the beginning, a low concentration signal is being generated; i.e., the concentration signal LV is at the LO level.

When the sensor output value S(n) increases in such a state, before time t1, the base value B(n) is calculated in step S34 (see FIG. 5), so that the base value B(n) increases to follow the increase in the sensor output value S(n) with delay. Thus, the difference value D(n) calculated in step S37 increases, and finally exceeds the high concentration threshold Tu (see FIG. 8(2)) at time t1. As a result, the result of the determination in step S39 becomes Yes, and in step S3B, a high concentration signal is generated as shown in FIG. 8(3). Specifically, the high concentration flag is set, whereby the concentration signal LV output from the gas detection apparatus 10 is switched to the HI level. After time t1, the base value B(n) is calculated in step S36 (see FIG. 5), so that the base value B(n) increases to slowly follow the increase in the sensor output value S(n) with delay.

When the sensor output value S(n) starts to decrease after having reached the maximum, the difference (difference value D(n)) between the sensor output value S(n) and the base value B(n) that increases slowly decreases, and finally, at time t0, the relation D(n) ≤ Td is satisfied. Notably, in the first embodiment, the low concentration threshold Td is set to zero. Therefore, the result of the determination in step S3A becomes Yes, and as shown in FIG. 8(2), a low concentration signal is generated after time t0. Specifically, the high concentration flag is reset, whereby the concentration signal LV output from the gas detection apparatus 10 is switched to the LO level. In the next cycle and cycles subsequent thereto, since the result of the determination in step S33 becomes No, in step S35 (see FIG. 5), the base value B(n) is made equal to the sensor output value S(n) (B(n) = S(n)). Therefore, the base value B(n) is made equal to the sensor output value S(n) during a period between time t0 and time t2 at which the sensor output value S(n) starts to increase again.

After time t2, since the sensor output value S(n) increases, the result of the determination in step 33 becomes Yes, and the base value B(n) is calculated in step S34. In this case, the sensor output value S(n) increases, and the base value B(n) increases with delay. Therefore, the difference between the two values (the difference value D(n)) increases gradually. After time t3 at which the difference value D(n) exceeds the high concentration threshold Tu, the result of the determination in step S39 again becomes Yes, and in step S3B, a high concentration signal is generated (see FIGS. 8(2) and 8(3)).

Incidentally, since the concentration of the oxidative gas decreases after having increased once, the oxidative gas remains on the surface of the gas sensor element 11 even after the concentration has decreased, and the above-described hysteresis phenomenon may have occurred. If such a hysteresis phenomenon has occurred at time t0, even when the concentration of the oxidative gas increases in a hysteresis period subsequent to time t0, the sensor output value S(n) changes only slightly, so that such a concentration increase cannot be detected properly, if a detection process which is the same as that employed in ordinary periods (no-hysteresis periods) is performed. Therefore, a delay may occur in the timing of switching to the high concentration signal (setting of the high concentration flag and switching the concentration signal LV to the HI level).

In the first embodiment, after time t0 at which switching from the high concentration signal to the low concentration signal is effected, the sensitized period processing (see step S60 and FIG. 7) is performed in order to increase the switching sensitivity, to thereby enable the switching to the high concentration signal to be performed in response to a slight increase in the sensor output value S(n).

For this purpose, the gas detection apparatus 10 of the first embodiment continuously calculates the moving difference value DI(n) in order to determine whether the present time is in a hysteresis period in which a hysteresis phenomenon is occurring (see step S42, FIG. 6, and FIG. 8(4)). The moving difference value DI(n) is a difference between two sensor output values S(n-16) and S(n), which are separated from each other by i cycles (= 16 cycles). Therefore, the moving difference value DI(n) represents a change in the sensor output value between two points in time which are separated from each other by 16T (T represents a period of time of a single cycle). Incidentally, in the case where the present time is in a hysteresis period, if the oxidative gas maintains a reduced concentration, the sensor output value S(n) decreases gradually. Therefore, the hysteresis period can be determined to continue from time t0 at which the switching from the high concentration signal to the low concentration signal is effected to a point in time at which the moving difference value DI(n) becomes equal to or smaller than the predetermined value R (DI(n) ≤ R, see step S66).

Therefore, the sensitization flag is maintained in the set state between a point in time at which the sensitization flag is set (see step S62) immediately after the switching to the low concentration signal (time t0, see step S61), and a point in time at which the flag is reset in step S69 when the moving difference value DI(n) becomes equal to or smaller than the predetermined value R. During the period in which the sensitization flag is in the set state, the first high concentration threshold Tu1 is employed as the high concentration threshold Tu (see step S22). Thus, as shown in FIG. 8(5), Tu1, which is smaller than Tu2 (Tu1 < Tu2), is used as the high concentration threshold Tu in place of Tu2 during the sensitized period between time t0 and time t4 (see FIG. 8(4)), at which the moving difference value DI(n) becomes equal to or smaller than the predetermined value R.

As has already been described with reference to FIG. 8(2), when the difference value D(n) exceeds the high concentration threshold Tu, the result of the determination in step S39 becomes Yes, and a high concentration signal is generated. Accordingly, by virtue of the operation of rendering the high concentration threshold Tu smaller only in the sensitized period between time t0 to time t4, the difference value D(n) easily exceeds the high concentration threshold Tu (= Tu1) when the sensor output value S(n) increases only slightly, and the result of the determination in step S39 becomes Yes, whereby the high concentration signal is generated easily. Therefore, when a hysteresis phenomenon occurs, the gas detection apparatus of the first embodiment detects a slight increase in the sensor output value S(n), and generates a high concentration signal (i.e., sets the high concentration flag), to thereby switch the concentration signal LV to the HI level. Therefore, it is possible to compensate a decrease in the detection sensitivity for concentration increase of the oxidative gas, which decrease would otherwise be caused by the hysteresis phenomenon.

Notably, in the example illustrated in FIG. 8(5), the sensor output value S(n) did not increase during the sensitized period (time t0 to time t4), and therefore, the sensitization flag was reset at time t4 at which DI(n) became equal to or smaller than R (see step S69). Therefore, at time t3 in an ordinary period, for which the processing in step S23 employs the second high concentration threshold Tu2 as the high concentration threshold Tu, the difference value D(n) exceeds the high concentration threshold Tu (= Tu2), and after t3, the high concentration flag is set, whereby the concentration signal LV is switched to HI level.

Further, in the first embodiment, as described above, immediately after the switching from the high concentration signal to the low concentration signal (time t0), in step S63, all the past i (= 16) sensor output values S(n-16) to S(n-1) of the 17 (= 16 + 1) sensor output values stored in step S41 are replaced with the present sensor output value S(n). This replacement is performed for the following reason. Depending on the manner of change in the sensor output value S(n), there may arise a phenomenon that even though the sensor output value S(n) actually decreases after having increased once, the moving difference value DI(n) calculated in the vicinity of time t0 becomes very small, because the present sensor output value S(n) and the sensor output value S(n-16) obtained 16 cycles prior to the present assume substantially the same value, whereby in step S66 the moving difference value DI(n) is determined to be equal to or smaller than R, and the hysteresis period is erroneously determined to have ended.

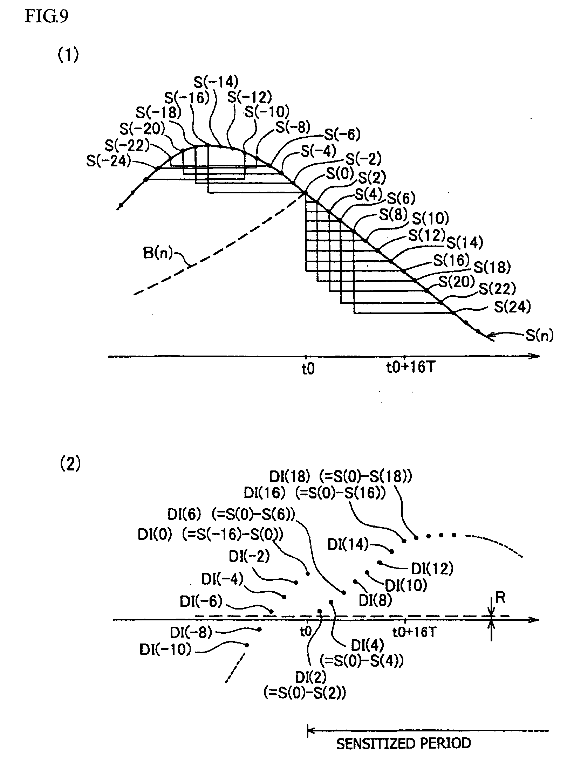

In view of the foregoing, when the moving difference value DI(n) is calculated in the above-described step S41 after time t0, an influence of sensor output values S(n) before time t0 is eliminated. When the sensor output value S(n) and the moving difference value DI(n) at each point in time are represented, while the sensor output value obtained at time t0 is represented by S(0), the sensor output value S(n) changes as shown in FIG. 9(1). Notably, in FIG. 9, values obtained every two cycles are shown for the sake of simplicity.

Here, before time t0, a moving difference value is obtained from the difference between two sensor output values obtained at points in time separated by 16 cycles; i.e., S(0) and S(-16), S(-2) and S(-18) (see FIG. 9(2)).

Meanwhile, in a period between t0 and t0+16T, because the sensor output values S(-16) to S(-1) have been replaced with the sensor output value S(0) in step S41, the sensor output value S(2), S(4), S(6), etc., at each point in time is subtracted from S(0) to obtain a corresponding moving difference value; e.g., DI(2) = S(0) - S(2), DI(4) = S(0) - S(4). Therefore, after time t0, the magnitude of the moving difference value DI(n) changes abruptly. Notably, in a period after t0+16T, the moving difference value is calculated in an ordinary manner; e.g., DI(18) = S(2) - S(18).

When a proper value is selected as the predetermined value R, DI(n) becomes greater than R after time t0, and the result of the determination in step S66 becomes No, whereby the sensitized period can be continued.

Notably, in the first embodiment, the sensor output acquisition circuit 19 corresponds to the acquisition means; and the microcomputer 16 corresponds the respective function realizing means such as the concentration detection means, the concentration increase detection means, and the concentration decrease detection means. Among the functions realized by the microcomputer 16, steps S3B and S3C correspond to the concentration detection means; and of these steps, step S3B corresponds to the concentration increase detection means, and step S3C corresponds to the concentration decrease detection means. Further, step S42 corresponds to the alternation-determining moving-difference-value calculation means; step S66 corresponds to the moving-difference-value determining means or end-timing determining means; and steps S34, S35, and S36 correspond to the reference value calculation means. These are examples of the respective means.

Next, a gas detection apparatus according to a first modification will be described. In the above-described first embodiment, when the result of the determination in step S66 of the subroutine for sensitized period processing (see FIG. 7) is Yes; i.e., DI(n) is determined to be equal to or smaller than R, the sensitization flag is immediately reset in step S69, the second high concentration threshold Tu2 is employed as the high concentration threshold Tu, and the sensitized period is ended. When the above is described with reference to FIG. 8, the sensitized period is immediately ended at time t4 at which DI(n) becomes equal to or smaller than R.

In contrast, in the gas detection apparatus according to the first modification, even when DI(n) is determined to be equal to or smaller than R, the sensitized period is continued for a short period of time. The remaining portions are the same as those of the first embodiment. Therefore, descriptions for the identical portions will be omitted or simplified, and portions different from those of the first embodiment will mainly be described.

In the first modification, as indicated by broken lines in FIG. 7, the sensitized period processing subroutine further includes steps S65, S67, and S68. Specifically, in the case where the result of the determination in step S64 becomes Yes because of the sensitization flag being in the set state, the microcomputer 16 proceeds to step S65 in order to determine whether an extension timer is clocking. When the extension timer has not been started, the result of the determination in step S65 becomes No, and the microcomputer 16 proceeds to step S66. In contrast, in the case where the result of the determination in step S65 becomes Yes, the microcomputer 16 proceeds to step S68, which will be described later. In step S66, the microcomputer 16 determines whether the moving difference value DI(n) has becomes smaller than the predetermined value (constant R). In the case where the result of the determination in step 66 is No, the microcomputer 16 returns to the main routine. In contrast, in the case where the moving difference value DI(n) is equal to or smaller than the constant R (the result of the determination in step 66 is Yes), unlike the first embodiment in which the microcomputer 16 proceeds to step S69, the microcomputer 16 proceeds to step S67 in order to start the extension timer, and then proceeds to step S68.

In step S68, the microcomputer 16 determines whether the extension timer has counted a time Ji from the start thereof. In the case where the time Ji has not elapsed (the result of the determination in step 68 is No), the microcomputer 16 returns to the main routine. In contrast, in the case where the time Ji has elapsed (the result of the determination in step 68 is Yes), the microcomputer 16 proceeds to step S69 in order to reset the sensitization flag. As a result, the result of the determination in step 21 becomes No, and thus the second high concentration threshold Tu2 is employed as the high concentration threshold Tu.

Next, the first modification will be described the manner in which the respective values are obtained when the sensor output value S(n) changes as indicated by a solid line in FIG. 8(1). FIGS. 8(1) to 8(4) are identical with those in the case of the first embodiment, and at time t4, the moving difference value DI(n) becomes equal to or smaller than the predetermined value. However, as shown in FIG. 8(6), Tu1 is employed as the high concentration threshold Tu during a period that starts at time t0, passes through time t4, and ends at time t5 at which the extension period of time Ji has elapsed from time t4. As is easily understood from comparison between FIGS. 8(5) and 8(6), in the first modification, the sensitized period (a period during which the sensitization flag is in the set state) is extended by the period of time Ji.

Therefore, as indicated by broken lines in FIGS. 8(2) and 8(3), in the first modification, in response to an increase in the sensor output value S(n) after time t4, the high concentration flag can be set and the concentration signal LV can be switched to the HI level at time t6, which is earlier than time t3 at which the high concentration flag is set in the first embodiment.

Next, a gas detection apparatus according to a second modification will be described. In the above-described first embodiment, immediately after the switching from the high concentration signal to the low concentration signal, in step S63, all the past i sensor output values S(n-i) to S(n-1) of the stored i+1 sensor output values are replaced with the present sensor output value S(n), and subsequently, the moving difference value DI(n) is calculated in step S42 of the subroutine for obtaining data for hysteresis processing (see FIG. 6). Therefore, as shown in, for example, FIGS. 8(3) and 9(2), the calculated moving difference value DI(n) is likely to assume a relatively small value during a period that starts at the time of switching from the high concentration signal to the low concentration signal and ends when a time corresponding to i-1 cycles has elapsed (time t0 to t0+15T in FIG. 8, etc.). This phenomenon occurs because the time difference between two points in time at which two sensor output values are obtained to be used for calculation of the moving difference value DI(n) is shorter than a time period corresponding to i cycles (16 cycles). In particular, moving difference values DI(1), DI(2), etc., immediately after the switching from the high concentration signal to the low concentration signal each assume a small value (see FIG. 9(2). Therefore, a restriction is imposed on selection of the predetermined value R which is compared with the moving difference value DI(n) so as to determine the end of the sensitized period.

In contrast, in the gas detection apparatus according to the second modification, the moving difference value is calculated by use of a different calculation formula until i cycles elapse after the switching from the high concentration signal to the low concentration signal. The remaining portions are the same as those of the first embodiment. Therefore, descriptions for the identical portions will be omitted or simplified, and portions different from those of the first embodiment will mainly be described.

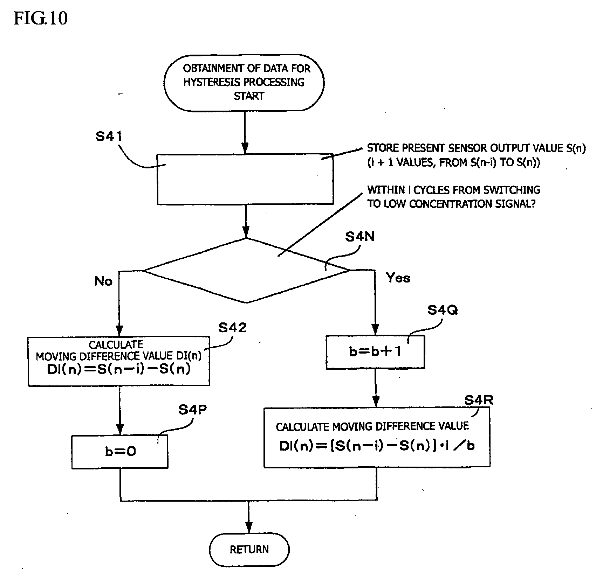

In the second modification, in place of the subroutine for obtaining data for hysteresis processing (see FIG. 6) used in the first embodiment, a subroutine for obtaining data for hysteresis processing shown in FIG. 10 is used. Specifically, as in the first embodiment, in step S41, the microcomputer 16 stores the present sensor output value S(n) obtained in step S31. Thus, i+1 sensor output values in total; i.e., S(n-i) to S(n), are stored. Subsequently, the microcomputer 16 proceeds to step S4N in order to determine whether the present cycle is within i cycles after the switching to the low concentration signal.

In the case where the present cycle is not within i cycles (the result of the determination is No), the microcomputer 16 proceeds to step S42 so as to calculate the moving difference value DI(n) in an ordinary manner. Subsequently, the microcomputer 16 sets an auxiliary value b to zero.

In contrast, in the case where the present cycle is within i cycles (the result of the determination is Yes), the microcomputer 16 proceeds to step S4Q so as to increment the auxiliary value b by one. In the next step S4R, the microcomputer 16 calculates the moving difference value DI(n) in accordance with the following Expression (5). Expression (5): DI(n) = {S(n-i) - S(n)}·i/b. As in the case of the first embodiment, in step S63, all the past i sensor output values, among the stored i+1 sensor output values, have been replaced with the sensor output value S(n) at the time of switching to the low concentration signal. Therefore, the moving difference value DI(n) calculated in step S4R in accordance with Expression (5) is i/b times the moving difference value obtained in the first embodiment. That is, in the case where time difference between two points in time at which two sensor output values are obtained to be used for calculation of the moving difference value DI(n) is shorter than a time period corresponding to i cycles (the result of the determination in step S4N is Yes), the difference is extended to a value which will be obtained when the time difference is increased to a value corresponding to i cycles.

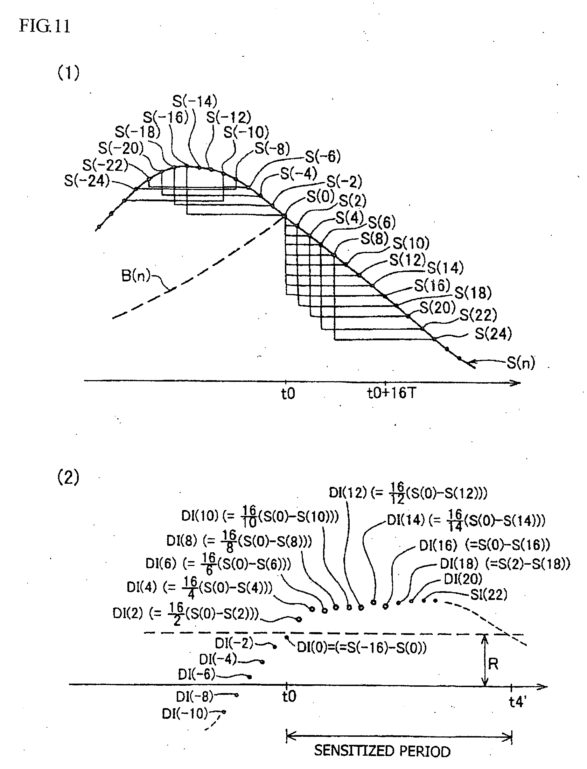

Therefore, when the sensor output value S(n) changes as shown in FIG. 11(1), the moving difference value DI(n) obtained in the second modification changes as shown in FIG. 11(2). Notably, in FIG. 11(2), each open circle represents a moving difference value calculated in step S4R.

As is easily understood from comparison with the case of the first embodiment (see FIG. 9), the moving difference values DI(n) calculated during the period between time t0 and t0+16T are greater than those in the case of the first embodiment. Therefore, instead of the predetermined value R used in step S66 in the first embodiment, a greater value can be used as the predetermined value R. Thus, the predetermined value R can be selected from a more appropriate range.

As shown in FIG. 11(2), since DI(n) becomes equal to or smaller than R at time t4', which is earlier than time t4 in the first embodiment, the sensitized period continues from time t0 to time t4'.

Next, a gas detection apparatus according to a second embodiment will be described. In the above-described first embodiment, a sensitized period during which the sensitization flag is in the set state is ended when the condition shown in step S66 is satisfied. The second embodiment differs from the first embodiment in that a sensitized period is ended when a predetermined duration time BJ1 has elapsed after the time of switching from the high concentration signal to the low concentration signal.

Further, in the first embodiment, in such a sensitized period, the predetermined first high concentration threshold Tu1 is used unconditionally as the high concentration threshold Tu. In contrast, in the second embodiment, the first high concentration threshold Tu1 is determined from the magnitude of a moving difference value obtained immediately after the switching to the low concentration signal. Therefore, the present embodiment differs from the first embodiment in that the high concentration threshold Tu used in the sensitized period changes in accordance with the obtained sensor output value S(n).

Therefore, portions different from those of the first embodiment will mainly be described, and descriptions of identical portions will be omitted or simplified.

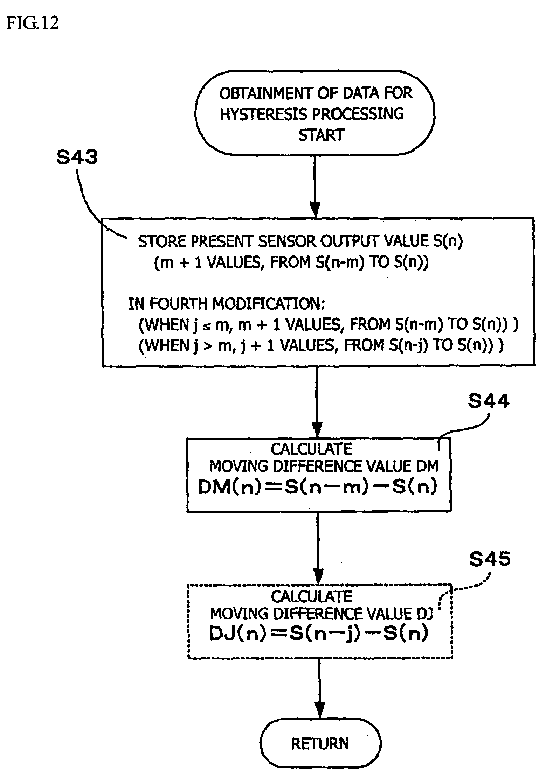

In the second embodiment, in place of the subroutine for obtaining data for hysteresis processing (see FIG. 6) used in the first embodiment, a subroutine for obtaining data for hysteresis processing shown in FIG. 12 is used. Notably, step S45 indicated by a broken line is used in a fourth modification, which will be described later. In first step S43, the microcomputer 16 stores the present sensor output value S(n) obtained in step S31. Notably, in the second embodiment, m+1 storage areas are provided in the microcomputer 16, and the microcomputer 16 stores m+1 sensor output values in total; i.e., S(n-m) to S(n). The operation of storing the newly obtained present sensor output value S(n) is preferably performed through overwriting the present sensor output value S(n) in the storage area which stores a sensor output value S(n-(m+1)) obtained in a previous cycle that precedes the present cycle by (m+1) cycles.

In subsequent step S44, the microcomputer 16 calculates a moving difference value DM(n) in accordance with the following Expression (6). Expression (6): DM(n) = S(n-m) - S(n). This moving difference value DM(n) represents the difference between the present sensor output value S(n) and a sensor output value S(n-m) obtained in a previous cycle that precedes the present cycle by m cycles; i.e., a change in the sensor output value during that period. In the second embodiment, by use of this moving difference value DM(n), the microcomputer 16 determines the magnitude of the first concentration threshold Tu1 in the sensitized period processing of step S60, which will be described later (step S6A).

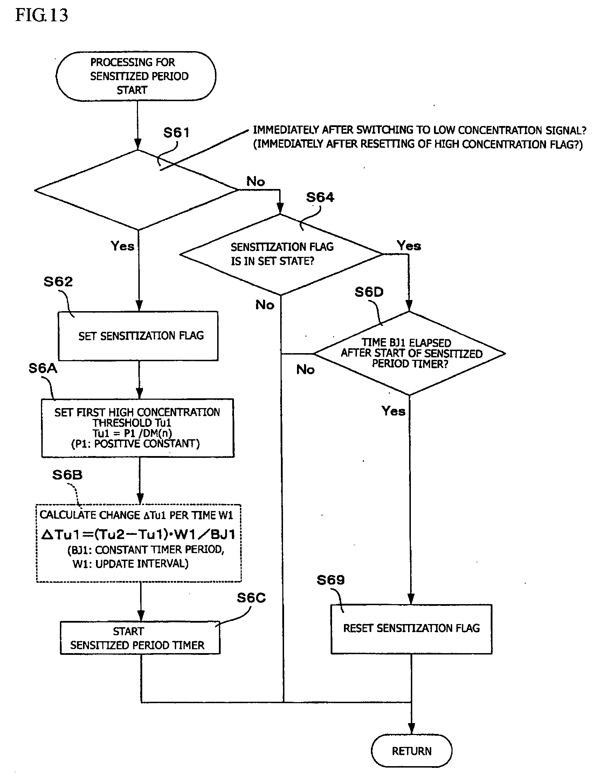

When the microcomputer 16 returns to the main routine (see FIG. 3) after calculating the moving difference value DM(n) in step S44, as in the case of the first embodiment, in step S50 the microcomputer 16 checks whether a high concentration signal is being generated. In the case where the result of determination is Yes; i.e., a high concentration signal is being generated, the microcomputer 16 returns to step S20. In contrast, in the case where the result of determination is No; i.e., a low concentration signal is being generated, the microcomputer 16 proceeds to the subroutine of step S60 shown in FIG. 13, because the present time may be in a hysteresis period.

Next, there will be described the subroutine of step S60, which is shown in FIG. 13 and is adapted to perform processing for sensitized periods. Steps S61, S62, S64, and S69 of this subroutine are identical with those in the first embodiment. Notably, step S6B indicated by a broken line is used in a third modification, which will be described later.

In first step S61, the microcomputer 16 determines whether the present time is immediately after the low concentration signal has been generated in place of the high concentration signal.

In the case where the result of determination is Yes, the microcomputer 16 proceeds to step S62 in order to set the sensitization flag, as in the first embodiment.

Subsequently, in step S6A, the microcomputer 16 sets the first concentration threshold Tu1 in accordance with the following Expression (7). Expression (7): Tu1 = P1/DM(n). Since P1 is a positive constant, the first high concentration threshold Tu1 assumes a smaller value; i.e., shifts toward the sensitivity increasing direction, as the moving difference value DM(n) obtained at the timing of the switching to the low concentration signal increases. The first high concentration threshold Tu1 is employed as the high concentration threshold Tu in step S22 (see FIG. 4) during a sensitized period in which the sensitization flag is in the set state. Therefore, the greater the moving difference value DM(n), the smaller the high concentration threshold Tu used in the sensitized period. Accordingly, even when the difference value D(n) increases slightly because of a slight increase in the sensor output value S(n), the difference value D(n) exceeds the high concentration threshold Tu. In this manner, the greater the moving difference value DM(n), the higher the sensitivity in detecting an increase in the sensor output value, and thus the higher the sensitivity in detecting an increase in gas concentration.

Notably, the microcomputer 16 performs step S6A only immediately after the low concentration signal is generated.

The microcomputer 16 proceeds from step S6A to step S6C in order to start a sensitized period timer.

In contrast, in the case where the result of the determination in step S61 is No, the microcomputer 16 proceeds to step S64 so as to determine whether the sensitization flag is in the set state. In the case where the sensitization flag is not in the set state (the result of the determination is No), the microcomputer 16 returns to the main routine. In contrast, in the case where the sensitization flag is in the set state (the result of the determination is Yes), the microcomputer 16 proceeds to step S6D.

In step S6D, the microcomputer 16 determines whether the sensitized period timer has clocked the predetermined duration time BJ1 after having started in step S6C. Before the predetermined duration time BJ1 has elapsed after the start of the sensitized period timer (the result of the determination is No), the microcomputer 16 returns to the main routine. As a result, as described above, in step S22, the first high concentration threshold Tu1 set in step S6A is employed as the high concentration threshold Tu. In contrast, after the predetermined duration time BJ1 has elapsed (the result of the determination is Yes), the microcomputer 16 proceeds to step S69 to thereby reset the sensitization flag, and then returns to the main routine. In the main routine, the result of the determination in step S21 (see FIG. 4) becomes No, and in step S23, the second high concentration threshold Tu2 is employed as the high concentration threshold Tu.

As described above, in the second embodiment, the sensitization flag is reset after the predetermined duration time BJ1 has elapsed from the switching to the low concentration signal. In other words, the end of the sensitized period in which the sensitization flag is in the set state is fixed to the point in time at which the predetermined duration time BJ1 has elapsed from the switching to the low concentration signal. Although the length of a hysteresis period over which a hysteresis phenomenon continues differs from time to time, the sensitivity drops greatly only within a period at the beginning of the hysteresis period. Therefore, even when the sensitized period has a fixed length, the sensitivity in detecting an increase in gas concentration can be increased within the period in which the sensitivity decreases greatly. Accordingly, a decrease in sensitivity stemming from a hysteresis phenomenon can conceivably be prevented substantially.

In this manner, the gas detection apparatus of the second embodiment can detect an increase in concentration of the specific gas reliably or quickly, irrespective of occurrence of a hysteresis phenomenon.

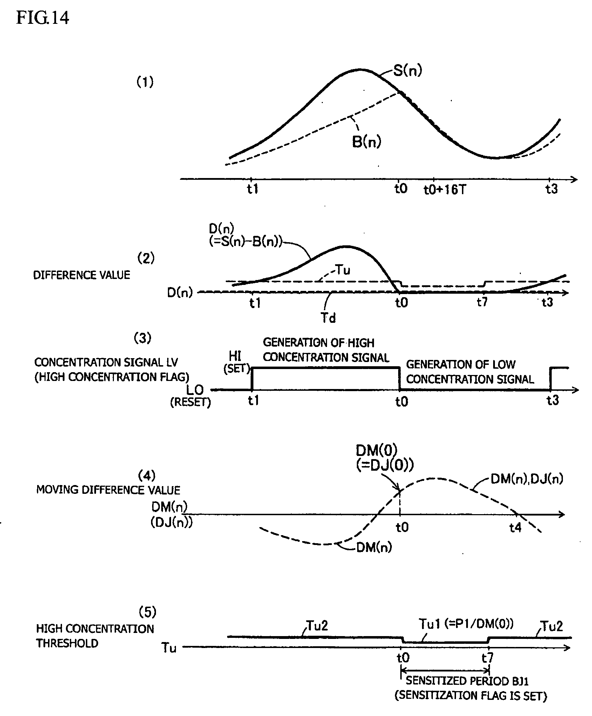

Next, with reference to FIGS. 14(1) to 14(5), there will be described the manner in which the respective values are obtained when the sensor output value S(n) changes as indicated by a solid line in FIG. 14(1). The profile of S(n) shown in FIG. 14(1) is identical with that in the case of the first embodiment. Therefore, the profiles of B(n) shown in FIG. 14(1) and D(n) shown in FIG. 14(2) are identical with those in the case of the first embodiment.

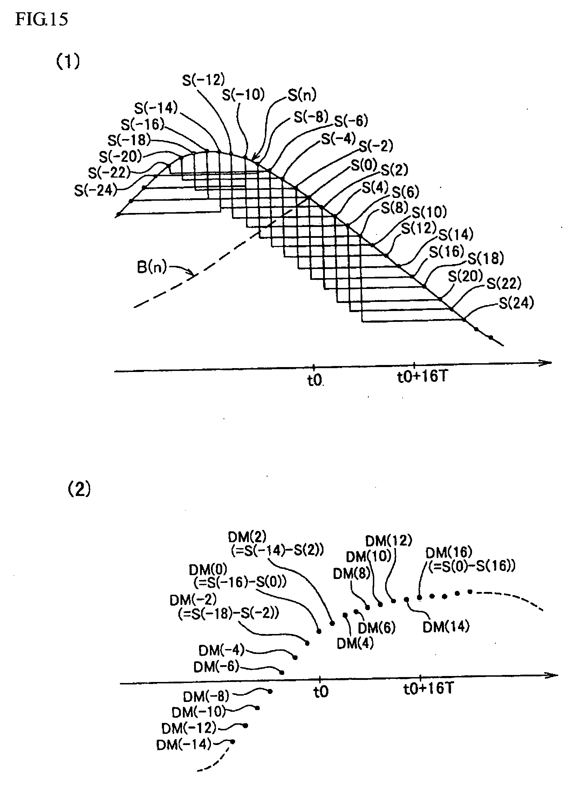

Meanwhile, the moving difference value DM(n) calculated in step S44 changes as shown in FIG. 14(4). The relation between the moving difference value DM(n) and the sensor output value S(n) will be described with reference to FIG. 15. FIG. 15(1) shows the sensor output value S(n) and the moving difference value DI(n) at each point in time, where the sensor output value obtained at time t0 is represented by S(0). Notably, this graph is identical to FIG. 9(1) in the first embodiment. Further, in FIG. 15, values obtained every two cycles are shown for the sake of simplicity.

In the second embodiment, a moving difference value is obtained from the difference between two sensor output values obtained points in time separated by 16 cycles (m = 16); i.e., S(-2) and S(-18), S(0) and S(-16), and S(2) and S(-14) (see FIG. 15(2)). As described above, in the second embodiment, since the replacement of past sensor output values, which in the first embodiment is performed in step S63, is not performed, the moving difference value DM(n) changes smoothly in the vicinity of time t0, unlike the moving difference value DI(n) in the first embodiment (see FIG. 9(2)).

Since D(n) has become equal to or smaller than Td at t0 (see FIG. 14(2)), at time t0, the high concentration flag is reset, whereby the concentration signal LV is switched to the LO level (see FIG. 14(3)). Further, in step S44, a moving difference value DM(0) at time t0 is calculated (see FIG. 14(4)). Moreover, in step S6A, the first high concentration value Tu1 is calculated, and employed as the high concentration value Tu for the sensitized period (see FIG. 14(5)). As described above, the high concentration value Tu used in the sensitized period changes in accordance with the moving difference value DM(0) at time t0, so that the larger the moving difference value DM(0), the smaller the high concentration value Tu. It was found that the greater the moving difference value DM(0); i.e., the greater the change in the sensor output value S(n) at the time (time t0) of switching from the high concentration signal to the low concentration signal, the greater the degree of a hysteresis phenomenon. In view of the above, as the moving difference value DM(0) increases, the value of the high concentration threshold Tu is decreased in order to compensate a decrease in detection sensitivity for concentration increase in the oxidative gas stemming from a hysteresis phenomenon.

In the second embodiment, the sensitized period during which the sensitization flag is in the set state ends at time t7 at which the predetermined duration time BJ1 has elapsed from time t0 (see FIG. 14(5)). Accordingly, after time t7, the second high concentration threshold Tu2 of a predetermined value is employed as the high concentration threshold Tu. The degree of the decrease in detection sensitivity for concentration increase in the oxidative gas stemming from a hysteresis phenomenon is the greatest immediately after the time (time t0) of switching from the high concentration signal to the low concentration signal, and decreases gradually with time. Therefore, when the sensitized period is unconditionally determined to continue over the period of the predetermined duration time BJ1 starting at time t0, the sensitivity can be increased at least within a period in which the sensitivity decreases greatly. Therefore, the above-described sensitization operation is considered to be sufficient in many cases.

Thus, the high concentration threshold Tu changes as indicated by a solid line in FIG. 14(5) and a broken line in FIG. 14(2). In step S39, through comparison between the high concentration threshold Tu and the difference value D(n) (see FIG. 5), a determination is made as to whether the high concentration signal is to be generated (the high concentration flag is to be set, the concentration signal LV is to be switched to the HI level). Notably, in the case of the change in the sensor output value S(n) shown in FIG. 14(1), D(n) becomes greater than Tu at time t3, and after time t3, the high concentration flag is set, and the concentration signal LV is switched to the HI level.

In the second embodiment, the moving difference value DM(n) obtained immediately after the switching to the low concentration signal; i.e., the moving difference value DM(0) at time t0, serves as hysteresis strength information. Among the functions realized by the microcomputer 16, step S44 corresponds to the threshold-determining moving-difference-value calculation means; and step S6A corresponds to the first-high-concentration-threshold determining means. These are examples of the respective means.

Next, a gas detection apparatus according to a third modification will be described. In the above-described first and second embodiments, etc., when the result of the determination in step S21 of the subroutine for setting the high concentration threshold Tu (see FIG. 4) is Yes; i.e., when the sensitization flag is determined to be in the set state, in step S22, the first high concentration threshold Tu1 is employed as the high concentration threshold Tu. In the first embodiment, the first high concentration threshold Tu1 is always constant. In the second embodiment, in step S6A the first high concentration threshold Tu1 is determined in accordance with the moving difference value DM(n). However, in either embodiment, the first high concentration threshold Tu1 is maintained in a continued sensitized period, and the first high concentration threshold Tu1 and the high concentration threshold Tu do not change within the continued sensitized period.

In contrast, in the gas detection apparatus of the third modification, the first high concentration threshold Tu1 is changed in a sensitized period, and thus the high concentration threshold Tu is also changed. The remaining portions are the same as those of the second embodiment. Therefore, descriptions of identical portions will be omitted or simplified, and portions different from those of the second embodiment will mainly be described.

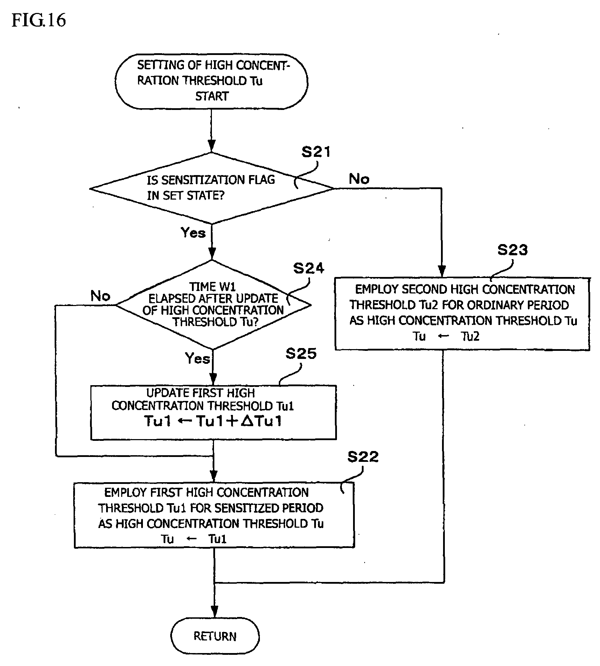

As is easily understood from comparison with the subroutine for setting the high concentration threshold Tu in the first and second embodiments, etc. (shown in FIG. 4), the subroutine for setting the high concentration threshold Tu in the third modification (shown in FIG. 16) has new steps S24 and S25 between steps S21 and S22.

Specifically, in the case where in step S21 the sensitization flag is determined to be in the set state (the result of the determination is Yes), the microcomputer 16 proceeds to step S24 in order to determine whether a time W1 has elapsed after the high concentration threshold Tu was updated. In the case where the result of the determination is No, the microcomputer 16 skips step S25, and proceeds to step S22. In contrast, in the case where the result of the determination is Yes, the microcomputer 16 proceeds to step S25 so as to update the first high concentration threshold Tu1. Specifically, a value obtained through addition of a change value ΔTu1 to the present high concentration threshold Tu1 is employed as a new high concentration threshold Tu1. By virtue of this operation, the first high concentration threshold Tu1 gradually increases every time the time W1 elapses. In subsequent step S22, the first high concentration threshold Tu1 is employed as the high concentration threshold Tu. Therefore, during a sensitized period in which the sensitization flag is in the set state, the high concentration threshold Tu also gradually increases by the change value ΔTu1 every time the time W1 elapses.

In the case where in step S21 the sensitization flag is determined not to be in the set state (the result of the determination is No), the second high concentration threshold Tu2 is employed as the high concentration threshold Tu as in the case of the first and second embodiments.

Further, in the third modification, the subroutine for sensitized period processing shown in FIG. 13 has a step S6B between steps S6A and S6C. In step S6B, the microcomputer 16 calculates the change value ΔTu1 of the first high concentration threshold Tu1 for every update interval W1. In the third modification, as in the case of the second embodiment, the magnitude of the first high concentration threshold Tu1 changes in step S6A in accordance with the moving difference value DM(n) obtained at the time (time t0) of the switching. Meanwhile, the first high concentration threshold Tu1 is preferably determined so as not to exceed the second high concentration threshold Tu2 and to gradually approach the second high concentration threshold Tu2. In consideration of the above, the change value ΔTu1 is properly calculated in step S6B. Specifically, the change value ΔTu1 is calculated in accordance with the following Expression (8). Expression (8): ΔTu1 = (Tu2 - Tu1)·W1/BJ1.

In this manner, irrespective of occurrence of a hysteresis phenomenon, the gas detection apparatus of the third modification can set a proper high concentration threshold at each point in time, and can detect an increase in concentration of the specific gas reliably or quickly.

In the third modification, the update interval W1 is rendered constant. Further, since the change value ΔTu1 is calculated in step S6B, the change value ΔTu1 can be a different value in each sensitized period, although the change value ΔTu1 is maintained constant in a single continuous sensitized period. However, the update interval W1 and the change value ΔTu1 can be properly determined at each point in time. Preferably, these value are determined in consideration of the duration time BJI of each sensitized period and the magnitude of the first high concentration threshold Tu1 at the beginning of each sensitized period in such a manner that the first high concentration threshold Tu1 used in each sensitized period does not exceed the second high concentration threshold Tu2.

Moreover, in the third modification, the update interval W1 is maintained constant, and the change value ΔTu1 is maintained constant in a single continuous sensitized period. However, the change value ΔTu1 may be changed with time, e.g., decreased with time, whereby a higher degree of sensitization is effected only in an early stage of the continuous sensitized period, and the first high concentration threshold Tu1 changes to gradually approach the second high concentration threshold Tu2. Moreover, the interval W1 for updating the high concentration threshold may be changed with time; e.g., the update interval W1 gradually increases, whereby a higher degree of sensitization is effected only in an early stage of the continuous sensitized period.

In the third modification, among the functions realized by the microcomputer 16, step S6B corresponds to the first-high-concentration-threshold update means. This is an example of the means.

Next, a gas detection apparatus according to a fourth modification will be described. In the above-described gas detection apparatus according to the second embodiment and the third modification, immediately after the switching to the low concentration signal, in step S6A the first high concentration threshold Tu1 is determined, whereby the first high concentration threshold Tu1 changes in accordance with the moving difference value DM(n). Meanwhile, for each sensitized period, the duration time BJ1 is imparted unconditionally in step S6D (see FIG. 13). Further, in the third modification, in each sensitized period, the first high concentration threshold Tu1 is gradually increased, and thus the high concentration threshold Tu is also gradually increased.

In contrast, in the fourth modification, the first high concentration threshold Tu1 immediately after the switching to the low concentration signal is changed in accordance with the moving difference value DM(n) as in the case of the second embodiment, and further, the length of each sensitized period is changed. The remaining portions are the same as those of the third modification. Therefore, descriptions of identical portions will be omitted or simplified, and portions different from those of the third modification will mainly be described.

As shown in FIG. 12, in the fourth modification, in step S44 of the subroutine for obtaining data for hysteresis processing, the microcomputer 16 calculates the moving difference value DM(n) to be used for determination of the first high concentration threshold Tu1, as in the cases of the second embodiment and the third modification. In addition, as indicated by a broken line in FIG. 12, in step S45 the microcomputer 16 calculates a moving difference value DJ(n) to be used for determining the end of each sensitized period, from the present sensor output value S(n) and the sensor output value S(n-j) in a previous cycle that precedes the present cycle by j cycles, in accordance with the following Expression (9). Expression (9): DJ(n) = S(n-j) - S(n). Notably, in step S43, preceding steps S44 and S45, the microcomputer 16 stores m+1 sensor output values when j ≤ m, and stores j+1 sensor output values when j > m. In other words, the microcomputer 16 stores sensor output values whose number is equal to a value obtained through addition of 1 to m or j, whichever is greater.

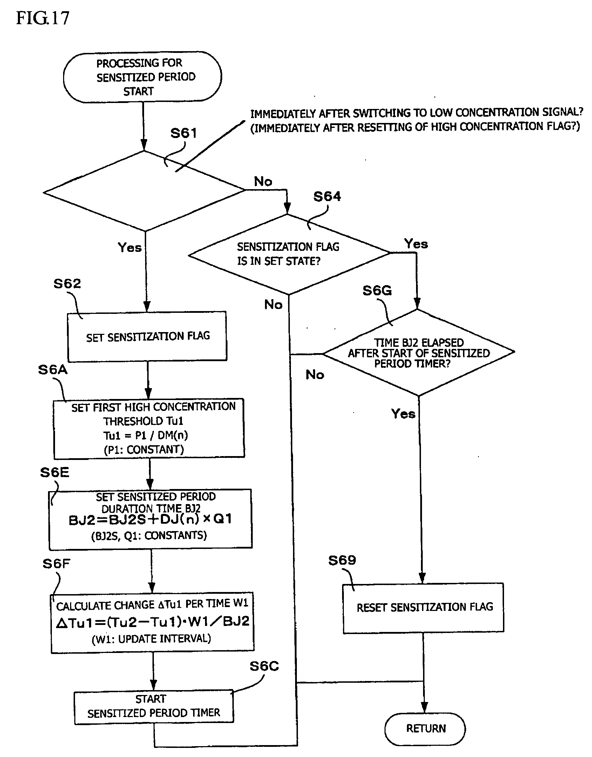

Moreover, as is easily understood by comparison with the subroutine for sensitized period processing in the second embodiment and the third modification (shown in FIG. 13), the subroutine for sensitized period processing in the fourth modification (shown in FIG. 17) has new steps S6E and S6F between steps S6A and S6C.

Specifically, as in the case of the second embodiment and the third modification, immediately after the switching to the low concentration signal, in step S62 the microcomputer 16 sets the sensitization flag, and then in step S6A sets the first concentration threshold Tu1 by use of the moving difference value DM(n). Further, in the fourth embodiment, in step S6E the microcomputer 16 sets a sensitized period duration time BJ2 in accordance with the following Expression (10). Expression (10): BJ2 = BJ2S + DJ(n) × Q1, where BJ2S and Q1 are each a positive constant. Therefore, the greater the moving difference value DJ(n) obtained immediately after the switching to the low concentration signal, the longer the sensitized period duration time BJ2.

Further, in step S6F, the microcomputer 16 calculates the change value ΔTu1 for every update interval W1. In the fourth modification, as in the case of the second embodiment and the third modification, the magnitude of the first high concentration threshold Tu1 obtained in step S6A changes in accordance with the moving difference value DM(n) obtained at the time (time t0) of the switching. Meanwhile, the first high concentration threshold Tu1 used in the sensitized period is preferably determined so as not to exceed the second high concentration threshold Tu2 and to gradually approach the second high concentration threshold Tu2. In consideration of the above, the change value ΔTu1 is properly calculated in step S6F. In the fourth embodiment, since the sensitized period duration time BJ2 was set in step S6E, the change value ΔTu1 is calculated in accordance with the following Expression (11) by use of the sensitized period duration time BJ2. Expression (11): ΔTu1 = (Tu2 - Tu1)·W1/BJ2.

In contrast, in the case where the result of the determination in step S61 is No and the result of the determination in step S64 is Yes (the case where the present time is within a sensitized period), in step S6G the microcomputer 16 determines whether the sensitized period timer has clocked the duration time BJ2 after having started in step S6C. In the case where the result of the determination in step S6G is No, the microcomputer 16 returns to the main routine. In the case where the result of the determination is Yes, in step S69 the microcomputer 16 resets the sensitization flag. By virtue of this operation, the sensitized period ends when the duration time BJ2 has elapsed after the start of the timer.

The above-described operation is performed for the following reason. That is, it was found that the greater the degree of a hysteresis phenomenon (accordingly, the greater the change in the sensor output value immediately after the switching to the low concentration signal), the longer the hysteresis period in which the hysteresis phenomenon continues. In view of this, in the fourth embodiment, the sensitized period duration time BJ2 is set in accordance with Expression (10). In other words, the sensitized period duration time BJ2 is set in accordance with the moving difference value DJ(n) obtained at the time of the switching, which corresponds to the change in the sensor output value immediately after the switching, in such a manner that the sensitized period duration time BJ2 increases as the moving difference value DJ(n) increases. This operation causes the length of a sensitized period to increase with the length of a hysteresis period, whereby a decrease in detection sensitivity for gas concentration increase stemming from a hysteresis phenomenon can be compensated over an appropriate period.

In the fourth modification, as in the case of the third modification, the first high concentration threshold Tu1 is caused to increase gradually.

In this manner, irrespective of occurrence of a hysteresis phenomenon, the gas detection apparatus of the fourth modification can set an appropriate high concentration threshold Tu at each point in time, and can detect an increase in concentration of the specific gas reliably or quickly.

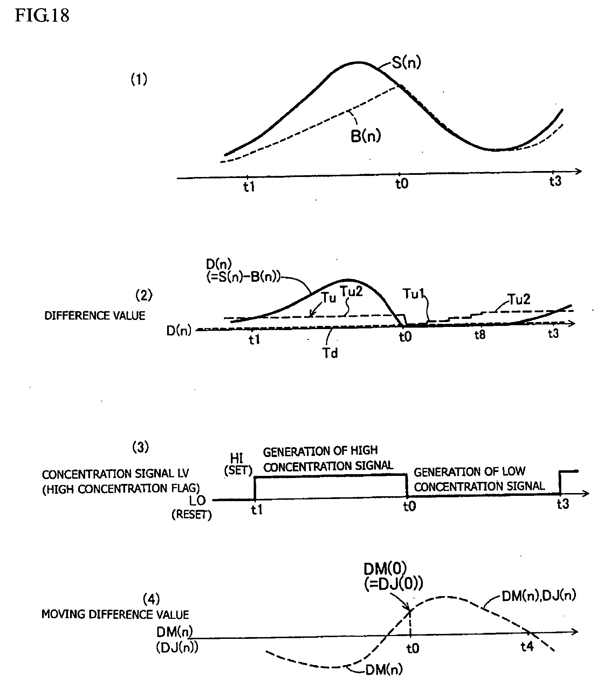

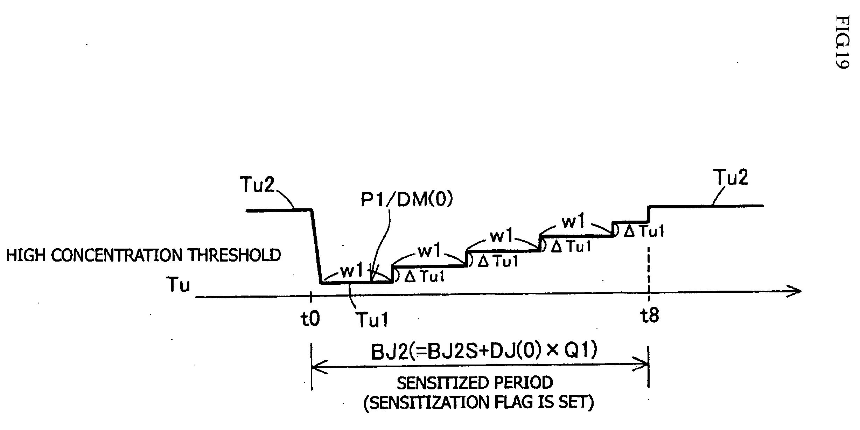

Next, with reference to FIGS. 18(1) to 18(4) and FIG. 19, there will be described the manner in which the respective values are obtained when the sensor output value S(n) changes as indicated by a solid line in FIG. 18(1). The profile of S(n) shown in FIG. 18(1) is identical with that in the case of the first and second embodiments and the third modification. Therefore, the profiles of B(n) shown in FIG. 18(1), D(n) shown in FIG. 18(2), and the change of the high concentration flag shown in FIG. 18(3) are identical with those in the case of the first and second embodiments and the third modification. Notably, in the example shown in FIGS. 18 and 19, for the sake of simplification, j and m are set to 16 (j = m = 16). Therefore, the moving difference value DM(n) to be used for determination of the first high concentration threshold Tu1 and the moving difference value DJ(n) to be used for determination of the end of each sensitized period are equal to each other (see FIG. 18(4)).

Meanwhile, the first high concentration threshold Tu1 is employed as the high concentration threshold Tu indicated by a solid line in FIG. 19 and by a broken line in FIG. 18(2), during a sensitized period between time t0 at which switching from the high concentration signal to the low concentration signal is effected and time t8 at which the sensitized period duration time BJ2 has elapsed. In addition, as shown in FIG. 19, the high concentration threshold Tu (the first high concentration threshold Tu1) is set to increase by the change value ΔTu1 every time the time W1 elapses. The first high concentration threshold Tu1 and the high concentration threshold Tu set immediately after time t0 are calculated in step S6A by use of the moving difference value DM(0) obtained at time t0.

Notably, as shown in FIG. 19, the length of the sensitized period duration time BJ2 is set in step S6E by use of the moving difference value DJ(0) obtained at time t0. Further, the change value ΔTu1 is set in step S6F by use of the duration time BJ2.

In the fourth modification, as in the case of the third modification, the update interval W1 and the change value ΔTu1 may be properly determined at each point in time. Preferably, these value are determined in consideration of the duration time BJ2 of each sensitized period and the magnitude of the first high concentration threshold Tu1 at the beginning of each sensitized period in such a manner that, in each sensitized period, the first high concentration threshold Tu1 does not exceed the second high concentration threshold Tu2.

Further, the change value ΔTu1 may be changed with time, e.g., decreased with time, whereby a higher degree of sensitization is effected only in an early stage of the sensitized period. Moreover, the interval W1 for updating the high concentration threshold may be changed with time; e.g., the update interval W1 gradually increases, whereby a higher degree of sensitization is effected only in an early stage of the sensitized period.

In the fourth modification, the moving difference value DJ(n) obtained immediately after the switching to the low concentration signal; i.e., the moving difference value DJ(0) at time t0, serves as hysteresis period information. Among the functions realized by the microcomputer 16, step S45 corresponds to the end-timing-determining moving-difference-value calculation means; and steps S6E and S6G correspond to the end-timing advance determining means. These are examples of the respective means.

Further, in the fourth modification, the microcomputer 16 determines the length of the duration time BJ2 in step S6E by use of the moving difference value DJ(n) obtained at the time of switching from the high concentration signal to the low concentration signal (by use of the moving difference value DJ(0) obtained at time t0 in the example shown in FIGS. 18 and 19). However, the end timing may be determined from a moving difference value obtained at a point in time which is a predetermined time earlier or later than the time of switching. In the example shown in FIGS. 18 and 19, the microcomputer 16 may determine the length of the duration time BJ2 in step S6E by use of a moving difference value (e.g., DJ(-2), DJ(4)) obtained at a point in time which is earlier or later than time t0 by a predetermined time.

Next, a gas detection apparatus according to a third embodiment will be described. In the above-described second embodiment, the first high concentration threshold Tu1 is determined by the processing in step S6A in accordance with the moving difference value DM(n) obtained immediately after the switching to the low concentration signal (see FIG. 13). In contrast, in the third embodiment, the first high concentration threshold Tu1 is determined in accordance with the maximum value of the difference value D(n) in a high-concentration-signal generation period immediately prior to the switching to the low concentration signal.

Therefore, portions different from those of the second embodiment will mainly be described, and descriptions of the identical portions will be omitted or simplified.

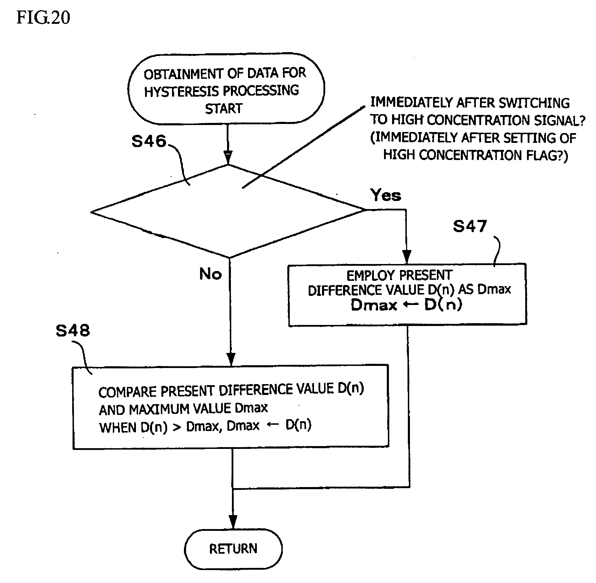

In the third embodiment, in place of the subroutine for obtaining data for hysteresis processing (see FIGS. 6 and 12) used in the first and second embodiments, a subroutine for obtaining data for hysteresis processing shown in FIG. 20 is used. In this subroutine, the microcomputer 16 obtains the maximum value Dmax of the difference value D(n) in a high-concentration-signal generation period immediately prior to the switching to the low concentration signal. In first step S46, the microcomputer 16 determines whether the present time is immediately after the switching to the high concentration signal; i.e., immediately after the high concentration flag is set.

In the case where the result of the determination is Yes, the microcomputer 16 proceeds to step S47 so as to store the present difference value D(n) as Dmax. This operation is performed in order to set an initial value for obtaining the maximum value of the difference value after the time when the switching to the high concentration signal is effected.

In contrast, in the case where the result of the determination is No, the microcomputer 16 proceeds to step S48. In this case, since Dmax has already been held, the microcomputer 16 compares the present difference value D(n) and Dmax, and, when D(n) > Dmax, replaces Dmax with the present difference value D(n). Since the above-described operation is repeated during the high-concentration-signal generation period, the maximum difference value Dmax in the high-concentration-signal generation period can be obtained.

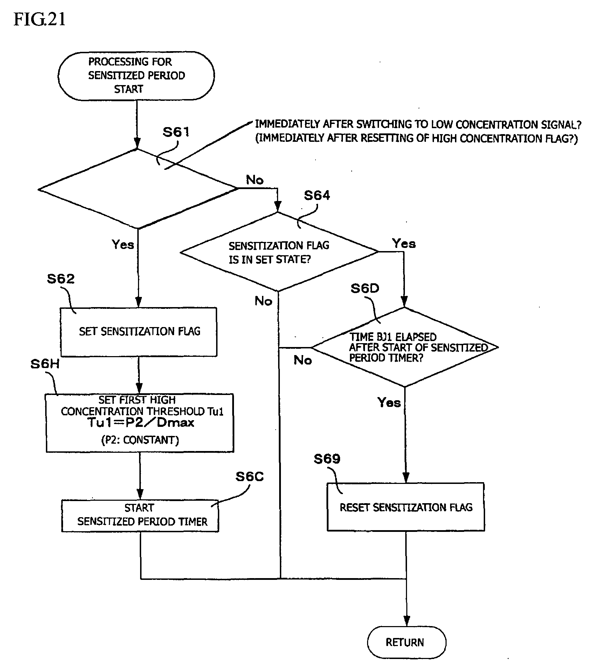

Next, there will be described the subroutine for sensitized period processing shown in FIG. 21. This subroutine is the same as the subroutine for sensitized period processing according to the second embodiment (shown in FIG. 13), except that step S6H is used in place of step S6A. Specifically, in the case where the present time is immediately after the switching to the low concentration signal (the result of determination in step S61 is Yes), after setting the sensitization flag in step S62, in step S6H the microcomputer 16 sets the first concentration threshold Tu1, by use of the maximum difference value Dmax previously obtained in the above-described subroutine for obtaining data for hysteresis processing, in accordance with the following Expression (12). Expression (12): Tu1 = P2/Dmax. Since P2 is a positive constant, as the maximum difference value Dmax increases, the calculated first high concentration threshold Tu1 assumes a smaller value. As in the case of the first and second embodiments, the first high concentration threshold Tu1 is employed as the high concentration threshold Tu in step S22 (see FIG. 4) during a sensitized period in which the sensitization flag is in the set state. Therefore, the greater the maximum difference value Dmax, the smaller the high concentration threshold Tu used in the sensitized period. Accordingly, an increase in the sensor output value can be detected more sensitively, and therefore an increase in gas concentration can also be detected more sensitively.

A hysteresis phenomenon is considered to occur because of adsorption of molecules of the specific gas to the gas sensor element. Therefore, the degree of the hysteresis phenomenon is considered to depend on the maximum concentration of the specific gas to which the gas sensor element is exposed in a high-concentration-signal generation period immediately before the time of switching from the high concentration signal to the low concentration signal.

In contrast, the difference value D(n), which is the difference between the sensor output value S(n) and the base value B(n), is considered to reflect, to some degree, the gas concentration at each point in time. Therefore, the maximum difference value Dmax is considered to assume a magnitude corresponding to the maximum concentration of the specific gas to which the gas sensor element is exposed in a high-concentration-signal generation period immediately before the time of switching from the high concentration signal to the low concentration signal.

In view of the foregoing, in the third embodiment, the first high concentration threshold Tu1 is determined on the basis of the maximum difference value Dmax. In addition, since the degree of a hysteresis phenomenon that occurs after the switching from the high concentration signal to the low concentration signal is predicted to increase with the maximum difference value Dmax, the first high concentration threshold Tu1 is made smaller in order to compensate a decrease in detection sensitivity for an increase in gas concentration.

Notably, the processing in step S6H is performed only immediately after the switching to the low concentration signal. Further, as in the case of the second embodiment, in the third embodiment, a sensitized period is ended when the predetermined duration time BJ1 has elapsed after the start of the sensitized period timer in step S6C. Subsequently, the second high concentration threshold Tu2 is used as the high concentration threshold Tu.

In this manner, the gas detection apparatus of the third embodiment can detect reliably, or can detect quickly, an increase in the concentration of the specific gas, irrespective of occurrence of a hysteresis phenomenon.

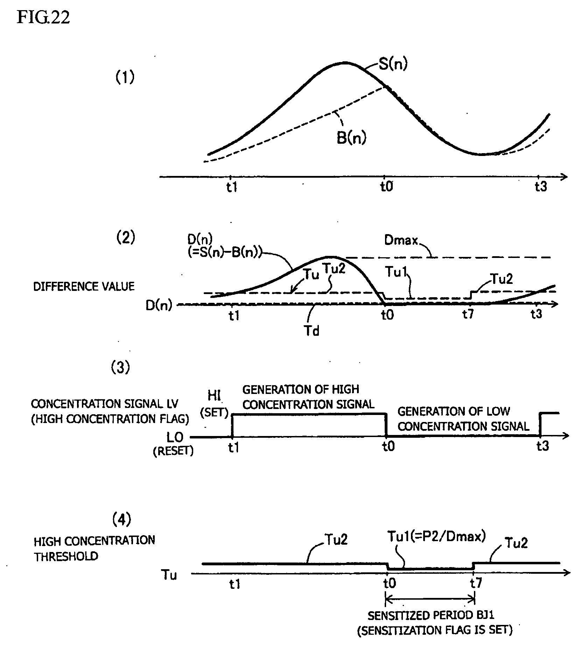

Next, with reference to FIGS. 22(1) to 22(4), there will be described the manner in which the respective values are obtained when the sensor output value S(n) changes as indicated by a solid line in FIG. 22(1). The profile of S(n) shown in FIG. 22(1) is identical with that in the case of the first and second embodiments. Therefore, the profiles of B(n) shown in FIG. 22(1), D(n) shown in FIG. 22(2), and the change of the high concentration flag shown in FIG. 22(3) are identical with those in the case of the first and second embodiments.

Meanwhile, the first high concentration threshold Tu1 is used as the high concentration threshold Tu indicated by a broken line in FIG. 22(2) and a solid line in FIG. 22(4), during a sensitized period between time t0 at which switching from the high concentration signal to the low concentration signal is effected and time t7 at which the predetermined duration time BJ1 has elapsed. In step S6H the first high concentration threshold Tu1 is determined in accordance with Expression (12) and by use of the maximum difference value Dmax (see FIG. 22(2)) among difference values D(n) obtained in the high-concentration-signal generation period (time t1 to t0) immediately before the time (time t0) of switching from the high concentration signal to the low concentration signal. Therefore, the first high concentration threshold Tu1 is rendered to decrease as the maximum difference value Dmax increases. In this manner, as the degree of the decrease in detection sensitivity stemming from a hysteresis phenomenon increases, the first high concentration threshold Tu1 is decreased, whereby an increase in the sensor output value S(n) and thus an increase in gas concentration can be detected more sensitively.

Next, a gas detection apparatus according to a fifth modification will be described. In the above-described gas detection apparatus according to the third embodiment, immediately after the switching to the low concentration signal, in step S6H, the first high concentration threshold Tu1 is determined in accordance with Expression (12), so that the first high concentration threshold Tu1 assumes a value corresponding to the maximum difference value Dmax. Meanwhile, in step S6D, each sensitized period is set to the constant duration time BJ1 (see FIG. 21).

In the fifth modification, as in the case of the third embodiment, the first high concentration threshold Tu1 is determined in accordance with the maximum difference value Dmax, immediately after the switching to the low concentration signal. However, in addition thereto, the length of each sensitized period (end of each sensitized period) is also set in accordance with the maximum difference value Dmax. The remaining portions are the same as those in the third embodiment. Therefore, descriptions for the identical portions will be omitted or simplified, and portions different from those of the third embodiment will mainly be described.

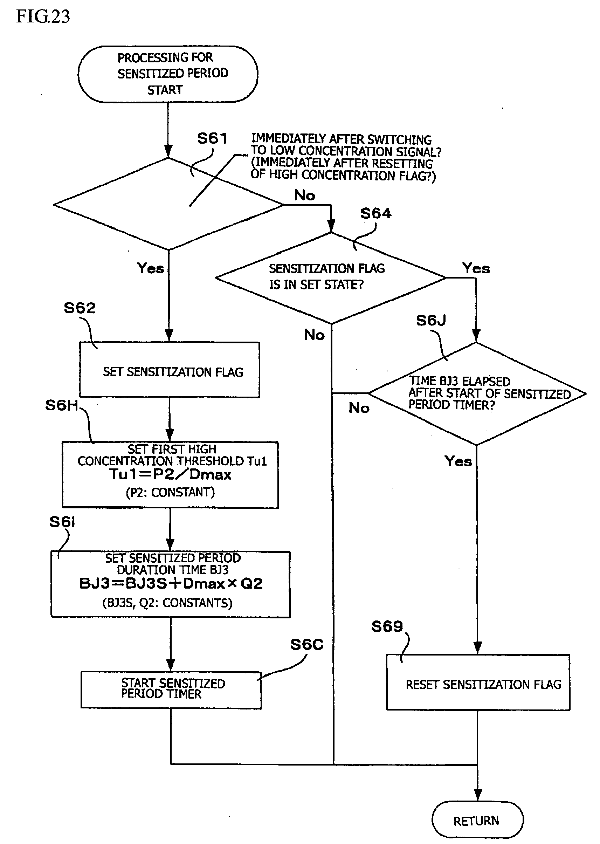

As is easily understood from comparison with the subroutine for sensitized period processing in the third embodiment (shown in FIG. 21), the subroutine for sensitized period processing in the fifth modification (shown in FIG. 23) has a new step S6I between steps S6H and S6C.

Specifically, as in the case of the third embodiment, immediately after the switching to the low concentration signal, after setting the sensitization flag in step S62, the microcomputer 16 sets the first concentration threshold Tu1 in step S6H by use of the maximum difference value Dmax. Further, in step S6I, the microcomputer 16 sets a sensitized period duration time BJ3 by use of the following Expression (13). Expression (13): BJ3 = BJ3S + Dmax × Q2. Here, BJ3S and Q2 are each a positive constant. Therefore, the sensitized period duration time BJ3 assumes a larger value as the maximum difference value Dmax increases.

In contrast, in the case where the result of the determination in step S61 is No and the result of the determination in step S64 is Yes (the case where the present time is within a sensitized period), in step S6J, the microcomputer 16 determines whether the sensitized period timer has clocked the duration time BJ3 after having started in step S6C. In the case where the result of the determination in step S6J is No, the microcomputer 16 returns to the main routine. In the case where the result of the determination is Yes, the microcomputer 16 resets the sensitization flag in step S69. The end of each sensitized period is determined in this manner, so that the duration time of each sensitized period coincides with the length of the sensitized period duration time BJ3 set in step S6I.

The above-described operation is performed because of the following reason. That is, it was found that the higher the maximum concentration of the oxidative gas to which the gas sensor element is exposed in a high-concentration-signal generation period immediately before the time of switching the low concentration signal; i.e., the maximum difference value Dmax obtained in that period, the longer a hysteresis period in which a hysteresis phenomenon continues. In view of this, in the fifth modification, the sensitized period duration time BJ3 is set in accordance with Expression (13). In other words, the sensitized period duration time BJ3 is set in accordance with the maximum difference value Dmax in such a manner that the sensitized period duration time BJ3 increases as the maximum difference value Dmax increases. This operation causes the length of a sensitized period to increase with the length of a hysteresis period, whereby a decrease in detection sensitivity for gas concentration increase stemming from a hysteresis phenomenon can be compensated over a proper period.

In this manner, irrespective of occurrence of a hysteresis phenomenon, the gas detection apparatus of the fifth modification can set a proper high concentration threshold Tu, and can detect an increase in concentration of the specific gas reliably or quickly.

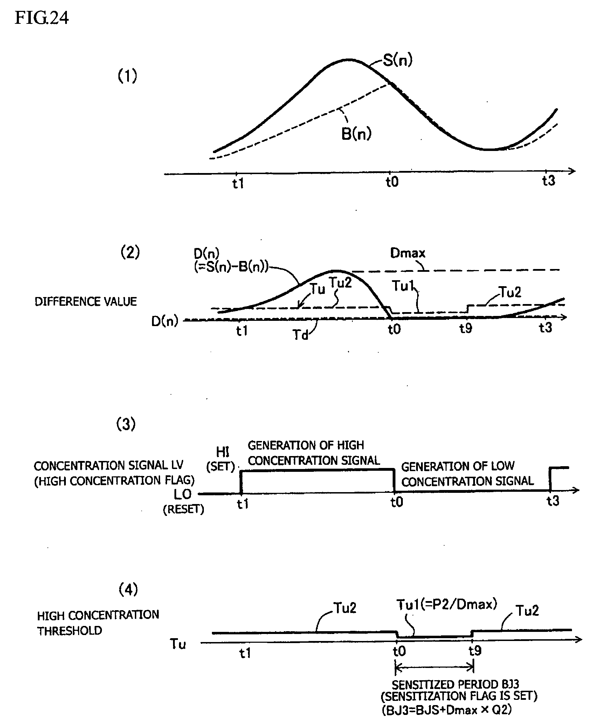

Next, with reference to FIGS. 24(1) to 24(4), there will be described the manner in which the respective values are obtained when the sensor output value S(n) changes as indicated by a solid line in FIG. 24(1). The profile of S(n) shown in FIG. 24(1) is identical with that in the case of the third embodiment. Therefore, the profiles of B(n) shown in FIG. 24(1), D(n) shown in FIG. 24(2), and the change of the high concentration flag shown in FIG. 24(3) are identical with those in the case of the third embodiment.

Meanwhile, the first high concentration threshold Tu1 is used as the high concentration threshold Tu shown in FIG. 24(4) during a sensitized period between time t0 at which switching from the high concentration signal to the low concentration signal is effected and time t9 at which the sensitized period duration time BJ3 has elapsed. As in the case of the third embodiment, the first high concentration threshold Tu1 and the high concentration threshold Tu are determined in accordance with Expression (12) and by use of the maximum difference value Dmax (see FIG. 24(2)) obtained in the high-concentration-signal generation period (time t1 to t0). Therefore, the first high concentration threshold Tu1 is rendered to decrease as the maximum difference value Dmax increases.

Further, in the fifth modification, the length of the sensitized period duration time BJ3 is also determined in accordance with Expression (13) and by use of the maximum difference value Dmax. The sensitized period duration time BJ3 is rendered to increase as the maximum difference value Dmax increases.

The difference value D(n), which is the difference between the sensor output value S(n) and the base value B(n), is considered to reflect, in some degree, a change in the concentration of the oxidative gas. Therefore, the maximum difference value Dmax is considered to reflect the maximum concentration of the oxidative gas to which the gas sensor element 11 is exposed in the high-concentration-signal generation period (time t1 to t0). Meanwhile, it was found that the length of a hysteresis period in which a hysteresis phenomenon continues increases as the maximum concentration of the oxidative gas to which the gas sensor element 11 is exposed. Therefore, by use of sensitized period duration time BJ3 corresponding to the maximum difference value Dmax, the length of each sensitized period can be increased with the length of a hysteresis period, to thereby compensate a decrease in detection sensitivity for an increase in the concentration of the oxidative gas.

Next, a gas detection apparatus according to a fourth embodiment will be described. In the above-described third embodiment, the first high concentration threshold Tu1 is determined by the processing in step S6H in accordance with the maximum difference value Dmax obtained in a high-concentration-signal generation period (see FIG. 21).

In contrast, in the fourth embodiment, the first high concentration threshold Tu1 is determined in accordance with the duration time of the high-concentration-signal generation period (high-concentration duration time) GH.

Therefore, portions different from those of the third embodiment will mainly be described, and descriptions for the identical portions will be omitted or simplified.

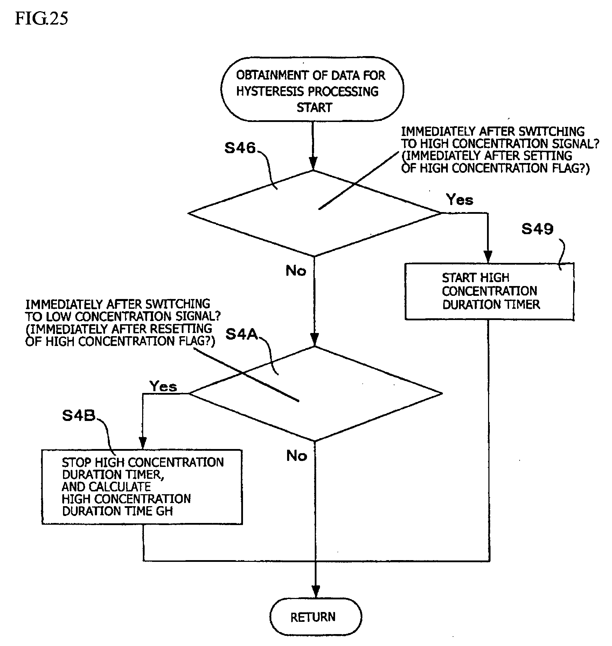

In the fourth embodiment, in place of the subroutine for obtaining data for hysteresis processing (see FIG. 20) used in the third embodiment, a subroutine for obtaining data for hysteresis processing shown in FIG. 25 is used. In this subroutine, the microcomputer 16 obtains a high-concentration duration time GH, which is the length of the high-concentration-signal generation period immediately before the switching to the low concentration signal.

First, as in the case of the third embodiment, in step S46, the microcomputer 16 determines whether the present time is immediately after the switching to the high concentration signal; i.e., immediately after the high concentration flag is set. This operation is performed in order to detect the start of the high-concentration-signal generation period.

In the case where the result of the determination is Yes, the microcomputer 16 proceeds to step S49 so as to start a high concentration duration timer, and then returns to the main routine.

In contrast, in the case where the result of the determination is No, the microcomputer 16 proceeds to step S4A. Contrary to the processing in step S46, in step S4A, the microcomputer 16 determines whether the present time is immediately after the switching to the low concentration signal; i.e., immediately after the high concentration flag is reset. This operation is performed in order to detect the end of the high-concentration-signal generation period. In the case where the result of the determination is No, the microcomputer 16 returns to the main routine. In contrast, in the case where the result of the determination is Yes, the microcomputer 16 proceeds to step S4B so as to stop the high concentration duration timer, and obtain the time clocked by the timer as the high-concentration duration time GH.

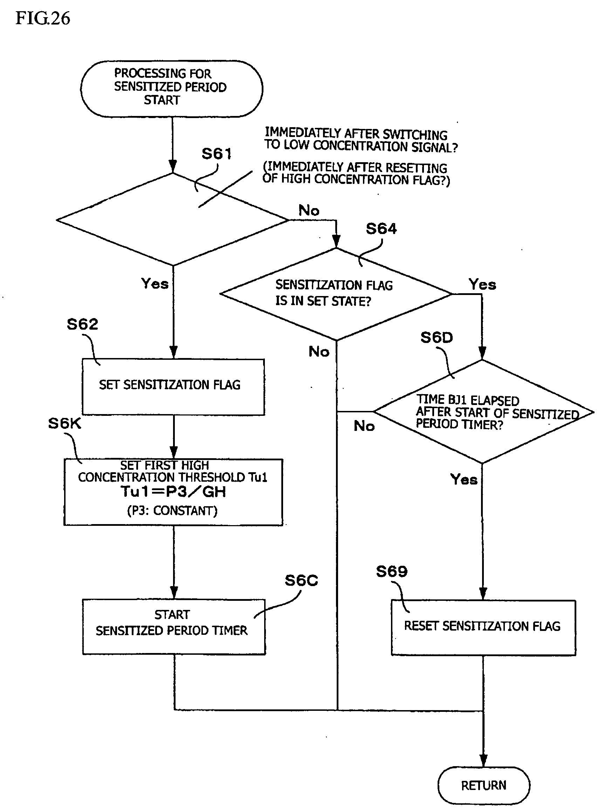

Next, there will be described the subroutine for sensitized period processing shown in FIG. 26. This subroutine is the same as the subroutine for sensitized period processing according to the third embodiment (shown in FIG. 21) except that step S6K is used in place of step S6H. Specifically, in the case where the present time is immediately after the switching to the low concentration signal (the result of determination in step S61 is Yes), after setting the sensitization flag in step S62, the microcomputer 16 sets the first concentration threshold Tu1 in step S6K, by use of the high-concentration duration time GH previously obtained in the above-described subroutine for obtaining data for hysteresis processing, in accordance with the following Expression (14). Expression (14): Tu1 = P3/GH. Since P3 is a positive constant, the calculated first high concentration threshold Tu1 assumes a smaller value, as the high-concentration duration time GH increases. As in the case of the first trough third embodiments, the first high concentration threshold Tu1 is employed as the high concentration threshold Tu in step S22 (see FIG. 4) during a sensitized period. Therefore, the longer the high-concentration duration time GH, the smaller the high concentration threshold Tu used in the sensitized period. Accordingly, an increase in the sensor output value, and thus an increase in gas concentration can be detected more sensitively.

A hysteresis phenomenon is considered to occur stemming from adsorption of molecules of the specific gas to the gas sensor element. Therefore, the degree of the hysteresis phenomenon is considered to depend on the length of a period between a point in time at which the concentration of the specific gas (oxidative gas) increased and a point in time at which the concentration of the specific gas (oxidative gas) decreases. This is because, the amount of gas molecules adsorbed by the gas sensor element is considered to increase with the length of a period in which the gas sensor element is exposed to the specific gas.

Meanwhile, the period in which the high concentration flag is in the set state, and the concentration signal LV assumes the HI level; i.e., the high-concentration-signal generation period, is considered to generally corresponds to a period in which the gas sensor element is exposed to the specific gas of high concentration. Therefore, the high-concentration duration time GH, which is the length of the high-concentration-signal generation period, is considered to correspond to the degree of the hysteresis phenomenon.

In view of the foregoing, in the fourth embodiment, the first high concentration threshold Tu1 is determined on the basis of the high-concentration duration time GH. In addition, since the degree of a hysteresis phenomenon that occurs after the switching from the high concentration signal to the low concentration signal is predicted to increase with the length of the high-concentration duration time GH, the first high concentration threshold Tu1 is made smaller in order to compensate a decrease in detection sensitivity for gas concentration increase.

Notably, the processing in step S6K is performed only immediately after the switching to the low concentration signal. Further, as in the case of the second and third embodiments, in the fourth embodiment, the sensitized period is ended when the predetermined duration time BJ1 has elapsed after the start of the sensitized period timer in step S6C. After that, the second high concentration threshold Tu2 is used as the high concentration threshold Tu.

In this manner, the gas detection apparatus of the fourth embodiment can detect reliably, or can detect quickly, an increase in the concentration of the specific gas irrespective of occurrence of a hysteresis phenomenon.

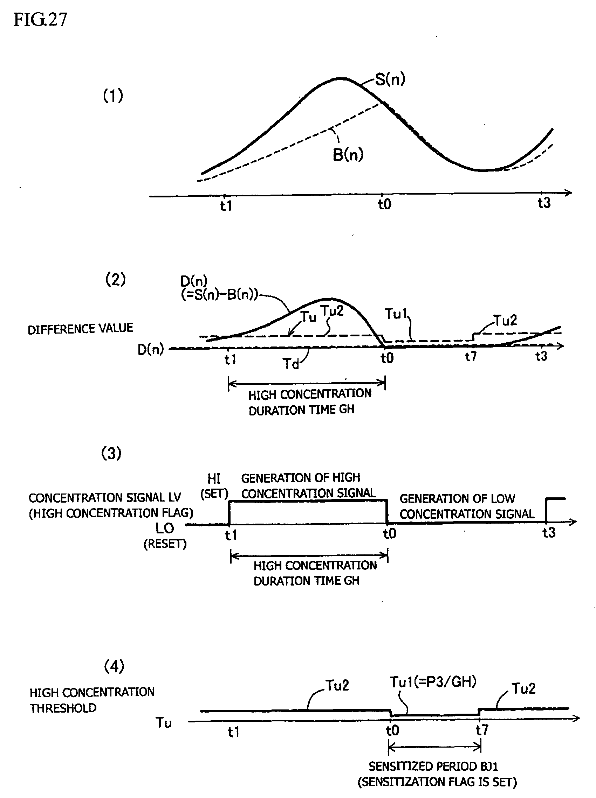

Next, with reference to FIGS. 27(1) to 27(4), there will be described the manner in which the respective values are obtained when the sensor output value S(n) changes as indicated by a solid line in FIG. 27(1). The profile of S(n) shown in FIG. 27(1) is identical with that in the case of the first through third embodiments. Therefore, the profiles of B(n) shown in FIG. 27(1), D(n) shown in FIG. 27(2), and the change of the high concentration flag shown in FIG. 27(3) are identical with those in the case of the first through third embodiments.

Meanwhile, the first high concentration threshold Tu1 is used as the high concentration threshold Tu indicated by a broken line in FIG. 27(2) and a solid line in FIG. 27(4), during a sensitized period between time t0 at which switching from the high concentration signal to the low concentration signal is effected and time t7 at which the predetermined duration time BJ1 has elapsed. The first high concentration threshold Tu1 is determined in step S6K in accordance with Expression (14) and by use of the high-concentration duration time GH (see FIG. 27(2), which is the duration time of the high-concentration-signal generation period (time t1 to t0) immediately before the time (time t0) when the switching from the high concentration signal to the low concentration signal is effected. Therefore, the first high concentration threshold Tu1 is rendered to decrease as the high-concentration duration time GH increases. In this manner, as the degree of the decrease in detection sensitivity stemming from a hysteresis phenomenon increases, the first high concentration threshold Tu1 is decreased, to thereby enable an increase in the sensor output value S(n), and thus an increase in the concentration of the oxidative gas to be detected more sensitively.

Next, a gas detection apparatus according to a sixth modification will be described. In the above-described gas detection apparatus according to the fourth embodiment, immediately after the switching to the low concentration signal, in step S6K, the first high concentration threshold Tu1 is determined in accordance with Expression (14), so that the first high concentration threshold Tu1 assumes a value corresponding to the high-concentration duration time GH. Meanwhile, in step S6D, each sensitized period is set to the constant duration time BJ1 (see FIG. 27).

In the sixth modification, as in the case of the fourth embodiment, the first high concentration threshold Tu1 is determined in accordance with the high-concentration duration time GH, immediately after the switching to the low concentration signal. However, in addition thereto, the length of each sensitized period is also set in accordance with the high-concentration duration time GH. The remaining portions are the same as those in the fourth embodiment. Therefore, descriptions for the identical portions will be omitted or simplified, and portions different from those of the fourth embodiment will mainly be described.

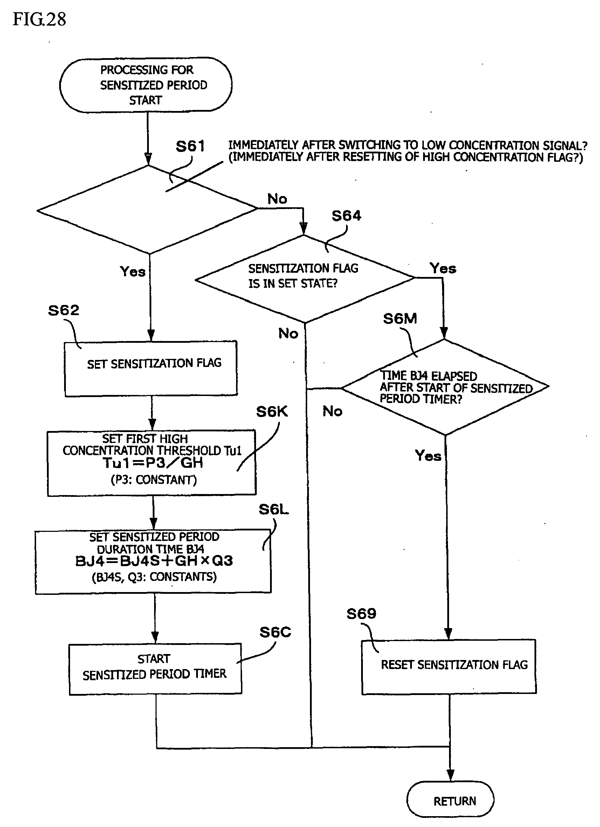

As is easily understood from comparison with the subroutine for sensitized period processing in the fourth embodiment (shown in FIG. 26), the subroutine for sensitized period processing in the sixth modification (shown in FIG. 28) has a new step S6L between steps S6K and S6C.

Specifically, as in the case of the fourth embodiment, immediately after the switching to the low concentration signal, after setting the sensitization flag in step S62, the microcomputer 16 sets the first concentration threshold Tu1 by use of the high-concentration duration time GH in step S6K. Further, in step S6L, the microcomputer 16 sets a sensitized period duration time BJ4 by use of the following Expression (15). Expression (15): BJ4 = BJ4S + GH × Q3. Here, BJ4S and Q3 are each a positive constant. Therefore, the sensitized period duration time BJ4 assumes a larger value as the high-concentration duration time GH increases.

In contrast, in the case where the result of the determination in step S61 is No and the result of the determination in step S64 is Yes (the case where the present time is within a sensitized period), in step S6M, the microcomputer 16 determines whether the sensitized period timer has clocked the duration time BJ4 after having started in step S6C. In the case where the result of the determination is No, the microcomputer 16 returns to the main routine. In the case where the result of the determination is Yes, the microcomputer 16 resets the sensitization flag in step S69. The end of each sensitized period is determined in this manner, so that the duration time of each sensitized period coincides with the length of the sensitized period duration time BJ4 set in step S6L.

The above-described operation is performed because of the following reason. That is, it was found that the longer the period in which the gas sensor element is exposed to the specific gas; i.e., the longer the high-concentration-signal generation period, the longer the length of a hysteresis period. In view of this, in the sixth modification, the sensitized period duration time BJ4 is set in accordance with Expression (15) in such a manner that the sensitized period duration time BJ4 increases as the length of the high-concentration duration time GH increases. This operation causes the length of a sensitized period to increase with the length of a hysteresis period, whereby a decrease in detection sensitivity for gas concentration increase stemming from a hysteresis phenomenon can be compensated over a proper period.

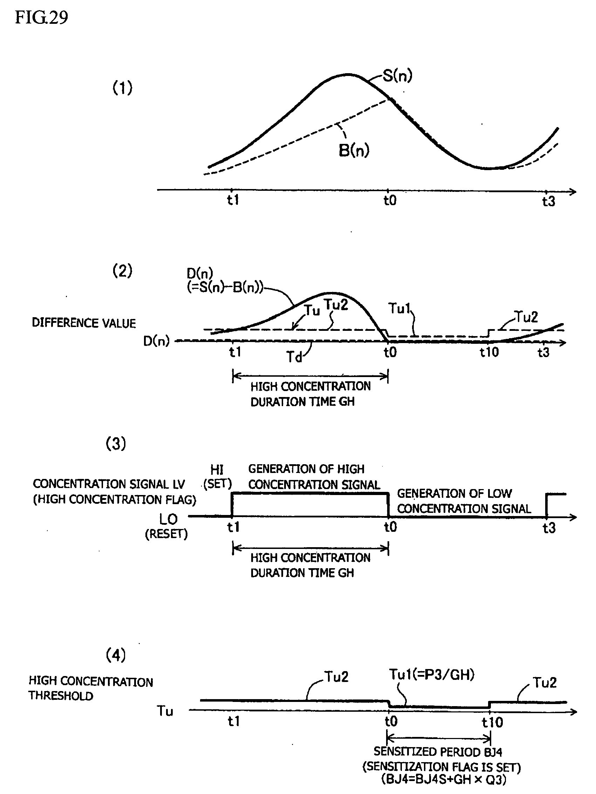

Next, with reference to FIGS. 29(1) to 29(4), there will be described the manner in which the respective values are obtained when the sensor output value S(n) changes as indicated by a solid line in FIG. 29(1). The profile of S(n) shown in FIG. 29(1) is identical with that in the case of the first through fourth embodiments. Therefore, the profiles of B(n) shown in FIG. 29(1), D(n) shown in FIG. 29(2), and the change of the high concentration flag shown in FIG. 29(3) are identical with those in the case of the fourth embodiment.

Meanwhile, the first high concentration threshold Tu1 is used as the high concentration threshold Tu shown in FIG. 29(4) during a sensitized period between time t0 at which switching from the high concentration signal to the low concentration signal is effected and time t10 at which the sensitized period duration time BJ4 has elapsed. As in the case of the fourth embodiment, the first high concentration threshold Tu1 and the high concentration threshold Tu are determined in accordance with Expression (14) and by use of the high-concentration duration time GH (see FIG. 29(3)), which the length of the high-concentration-signal generation period (time t1 to t0). Therefore, the first high concentration threshold Tu1 is rendered to decrease as the length of the high-concentration duration time GH increases.

Further, in the sixth modification, the length of the sensitized period duration time BJ4 is also determined in accordance with Expression (15) and by use of the high-concentration duration time GH. The sensitized period duration time BJ4 is rendered to increase as the length of the high-concentration duration time GH increases.

The length of a hysteresis period is considered to increase as the duration time of the specific gas to which the gas sensor element is exposed. Therefore, by use of the sensitized period duration time BJ4 corresponding to the high-concentration duration time GH, which is the length of the high-concentration-signal generation period (time t1 to t0), the length of each sensitized period can be increased with the length of the corresponding hysteresis period, whereby a decrease in detection sensitivity for an increase in the concentration of the oxidative gas can be compensated over a proper period.

Next, a gas detection apparatus according to a fifth embodiment will be described. In the above-described third embodiment, the first high concentration threshold Tu1 is determined by the processing in step S6H in accordance with the maximum difference value Dmax obtained in a high-concentration-signal generation period (see FIG. 21).

In contrast, in the fifth embodiment, the first high concentration threshold Tu1 is determined in accordance with the sum SD of difference values D(n) obtained in a high-concentration-signal generation period.

Therefore, portions different from those of the third embodiment will mainly be described, and descriptions for the identical portions will be omitted or simplified.

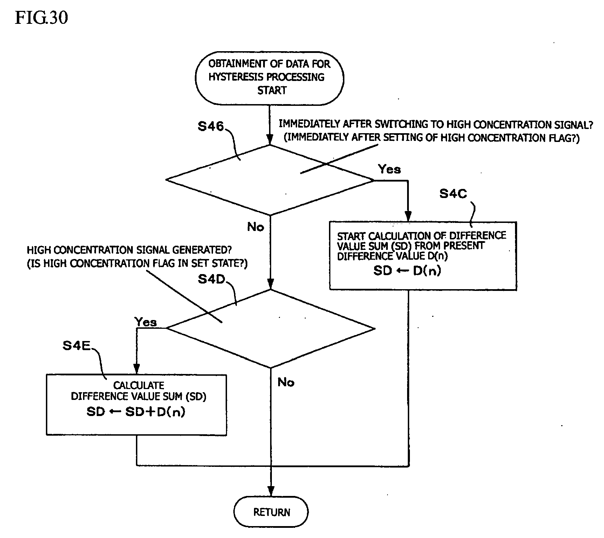

In the fifth embodiment, in place of the subroutine for obtaining data for hysteresis processing (see FIG. 20) used in the third embodiment, a subroutine for obtaining data for hysteresis processing shown in FIG. 30 is used. In this subroutine, the microcomputer 16 obtains the difference value sum SD.

First, as in the case of the third embodiment, in step S46, the microcomputer 16 determines whether the present time is immediately after the switching to the high concentration signal; i.e., immediately after the high concentration flag is set. This operation is performed to detect the start of a high-concentration-signal generation period.

In the case where the result of the determination is Yes, the microcomputer 16 proceeds to step S4C so as to start calculation of the difference value sum SD by use of the present difference value D(n). Specifically, the microcomputer 16 stores the present difference value D(n) as the difference value sum SD, and then returns to the main routine.

In contrast, in the case where the result of the determination is No, the microcomputer 16 proceeds to step S4D. In step S4D, the microcomputer 16 determines whether a high concentration signal is presently being generated. Specifically, the microcomputer 16 determines whether the high concentration is in the set state. This operation is performed in order to update the difference value sum SD only in the high-concentration-signal generation period. In the case where the result of the determination is No, the microcomputer 16 returns to the main routine. In contrast, in the case where the result of the determination is Yes, the microcomputer 16 proceeds to step S4E so as to store, as a new difference value sum SD, the value obtained through addition of the present difference value D(n) to the difference value sum SD already obtained. Since the difference value sum SD is obtained in the above-described manner, the difference value sum SD gradually increases during the high-concentration-signal generation period.

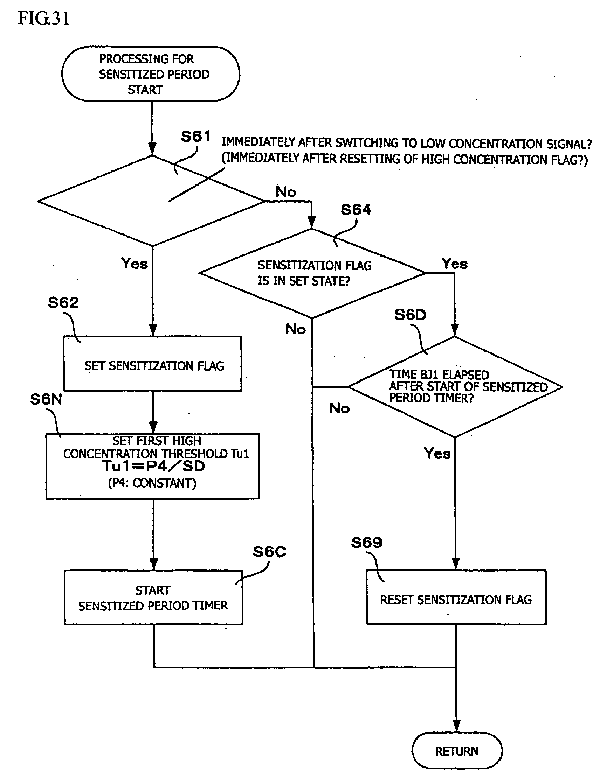

Next, there will be described the subroutine for sensitized period processing shown in FIG. 31. This subroutine is the same as the subroutine for sensitized period processing according to the third embodiment (shown in FIG. 21) except that step S6N is used in place of step S6H. Specifically, in the case where the present time is immediately after the switching to the low concentration signal (the result of determination in step S61 is Yes), after setting the sensitization flag in step S62, the microcomputer 16 sets the first concentration threshold Tu1 in step S6N, by use of the difference value sum SD previously obtained in the above-described subroutine for obtaining data for hysteresis processing, in accordance with the following Expression (16). Expression (16): Tu1 = P4/SD. Since P4 is a positive constant, the calculated first high concentration threshold Tu1 assumes a smaller value, as the difference value sum SD increases. As in the case of the third embodiment, the first high concentration threshold Tu1 is employed as the high concentration threshold Tu in step S22 (see FIG. 4) during a sensitized period in which the sensitized flag is in the set state. Therefore, the greater the difference value sum SD, the smaller the high concentration threshold Tu used in the sensitized period. Accordingly, an increase in the sensor output value, and thus an increase in gas concentration can be detected more sensitively.

The degree of the hysteresis phenomenon is considered to depend on the concentration of the specific gas in the high-concentration-signal generation period, and the duration of that period. This is because, the amount of gas molecules adsorbed by the gas sensor element is considered to increase with the concentration of the specific gas to which the gas sensor element is exposed as well as the length of the exposure time.

Meanwhile, the difference value D(n) is considered to correspond, in some degree, to a change in the concentration of the oxidative gas, whereas the high-concentration-signal generation period is considered to generally correspond to the length of a period in which the gas sensor element is exposed to the specific gas of high concentration. Therefore, the difference value sum SD, which is the sum of difference values D(n) in the high-concentration-signal generation period, is considered to assume a value corresponding to the degree of a hysteresis phenomenon. In view of the foregoing, in the fifth embodiment, the first high concentration threshold Tu1 is determined on the basis of the difference value sum SD. In addition, since the degree of a hysteresis phenomenon that occurs after the switching from the high concentration signal to the low concentration signal is predicted to increase as the difference value sum SD increases, the first high concentration threshold Tu1 is made smaller in order to compensate a decrease in detection sensitivity for gas concentration increase.

Notably, the processing in step S6N is performed only immediately after the switching to the low concentration signal. Further, as in the case of the second to fourth embodiments, in the fifth embodiment, the sensitized period is ended when the predetermined duration time BJ1 has elapsed after the start of the sensitized period timer in step S6C. After that, the second high concentration threshold Tu2 is used as the high concentration threshold Tu.

In this manner, the gas detection apparatus of the fifth embodiment can detect reliably, or can detect quickly, an increase in the concentration of the specific gas irrespective of occurrence of a hysteresis phenomenon.

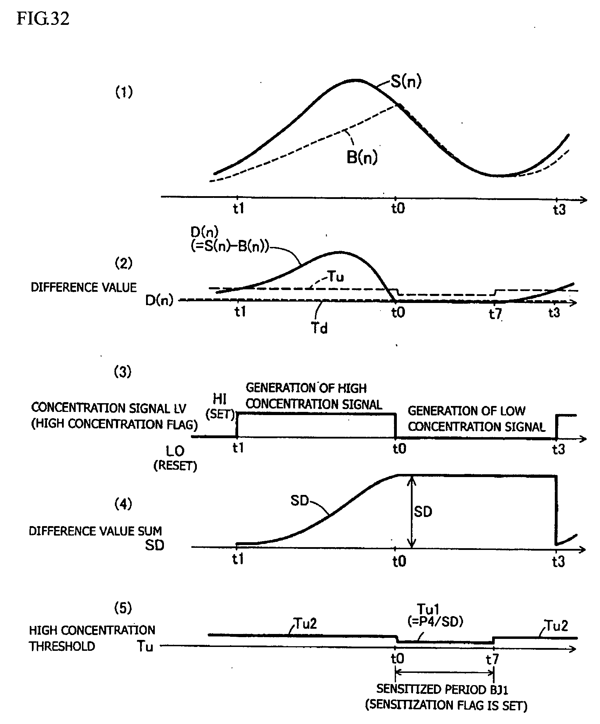

Next, with reference to FIGS. 32(1) to 32(5), there will be described the manner in which the respective values are obtained when the sensor output value S(n) changes as indicated by a solid line in FIG. 32(1). The profile of S(n) shown in FIG. 32(1) is identical with that in the case of the first through fourth embodiments. Therefore, the profiles of B(n) shown in FIG. 32(1), D (n) shown in FIG. 32(2), and the change of the high concentration flag shown in FIG. 32(3) are identical with those in the case of the first through fourth embodiments.

Meanwhile, the first high concentration threshold Tu1 is used as the high concentration threshold Tu indicated by a broken line in FIG. 32(2) and a solid line in FIG. 32(5), during the sensitized period between time t0 at which switching from the high concentration signal to the low concentration signal is effected and time t7 at which the predetermined duration time BJ1 has elapsed.

The first high concentration threshold Tu1 is determined in step S6N in accordance with Expression (16) and by use of the difference value sum SD at time t0 on the graph (see FIG. 32(4)) of the difference value sum SD, which indicates the sum of difference values D(n) in the high-concentration-signal generation period (time t1 to t0). The difference value sum SD at time t0 is the sum of difference values D(n) in a high-concentration-signal generation period between time t1 and time t0 (a period in which the high concentration flag is in the set state). The first high concentration threshold Tu1 is rendered to decrease as the difference value sum SD at time t0 increases. In this manner, as the degree of the decrease in detection sensitivity stemming from a hysteresis phenomenon increases, the first high concentration threshold Tu1 is decreased, to thereby enable an increase in the sensor output value S(n), and thus an increase in the concentration of the oxidative gas to be detected more sensitively.

In the fifth embodiment, the first high concentration threshold Tu1 is determined in accordance with the difference value sum SD immediately after the switching to the low concentration signal, whereas the length of each sensitized period (end of the sensitized period) is rendered constant (set to the predetermined duration time BJ1). However, the length of each sensitized period (end of the sensitized period) may be set in accordance with the difference value sum SD in such a manner that the length of the sensitized period increases as the difference value sum SD increases. For example, the length of each sensitized period may be determined by a sensitized-period duration time BJ5, which is calculated by Expression (17): BJ5 = BJ5S + SD × Q4, where BJ5S and Q4 are each a positive constant.

Moreover, although, in the fifth embodiment, the first high concentration threshold Tu1 is determined in accordance with the difference value sum SD, the first high concentration threshold Tu1 may be determined from an output value sum SS, which is the sum of differences between a start-point sensor output value which is obtained at the beginning of the high-concentration-signal generation period, and sensor output values at respective points in time during that period. In addition, the length of the sensitized period may be set to increase as the output value sum SS increases.

Next, a gas detection apparatus according to a sixth embodiment will be described. In the above-described third embodiment, the first high concentration threshold Tu1 is determined by the processing in step S6H in accordance with the maximum difference value Dmax obtained in a high-concentration-signal generation period (see FIG. 21).

In contrast, in the sixth embodiment, the first high concentration threshold Tu1 is determined in accordance with a maximum difference MD, which is the difference between a start-point sensor output value which is obtained at the beginning of a high-concentration-signal generation period and the maximum sensor output value (peak sensor output value) obtained in that period.

Therefore, portions different from those of the third embodiment will mainly be described, and descriptions for the identical portions will be omitted or simplified.

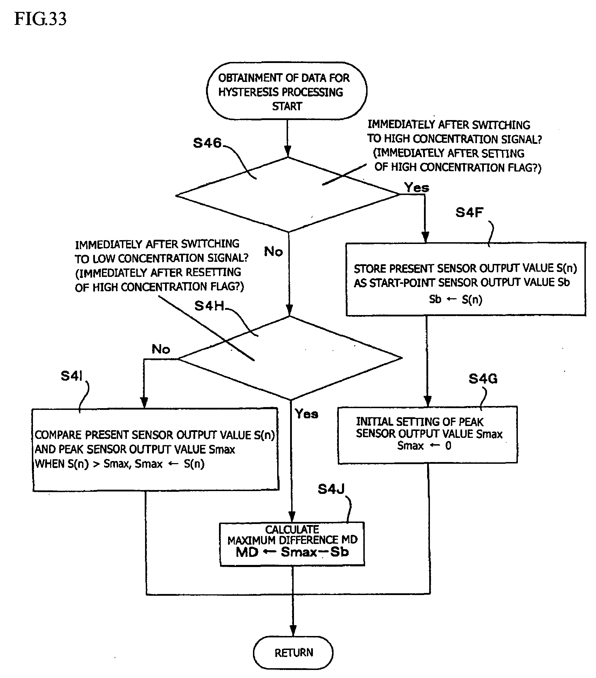

In the sixth embodiment, in place of the subroutine for obtaining data for hysteresis processing (see FIG. 20) used in the third embodiment, a subroutine for obtaining data for hysteresis processing shown in FIG. 33 is used. In this subroutine, the microcomputer 16 obtains the maximum difference MD.

First, as in the case of the third embodiment, in step S46, the microcomputer 16 determines whether the present time is immediately after the switching to the high concentration signal; i.e., immediately after the high concentration flag is set. This operation is performed to detect the start of a high-concentration-signal generation period.

In the case where the result of the determination is Yes, the microcomputer 16 proceeds to step S4F so as to store the present sensor output value S(n) as a start-point sensor output value Sb. Further, in step S4G, the microcomputer 16 sets a peak sensor output value Smax to zero, to thereby perform initial setting, and then returns to the main routine.

In contrast, in the case where the result of the determination is No, the microcomputer 16 proceeds to step S4H. Contrary to the processing in step S46, in step S4H, the microcomputer 16 determines whether the present time is immediately after the switching to the low concentration signal; i.e., immediately after the high concentration flag is reset.

In the case where the result of the determination is No, the microcomputer 16 proceeds to step S4I. In this case, since the peak sensor output value Smax has already been held, the microcomputer 16 compares the present sensor output value S(n) and Smax. When S(n) > Smax, the microcomputer 16 replaces Smax with the present sensor output value S(n). Since the above-described operation is repeated during the high-concentration-signal generation period, the peak sensor output value Smax in the high-concentration-signal generation period can be obtained. In contrast, in the case where the result of the determination in step S4H is Yes, the microcomputer 16 proceeds to step S4J. In this case, since the high-concentration-signal generation period has ended, the start-point sensor output value Sb is subtracted from the peak sensor output value Smax already obtained, to thereby obtain the maximum difference MD (= Smax - Sb).

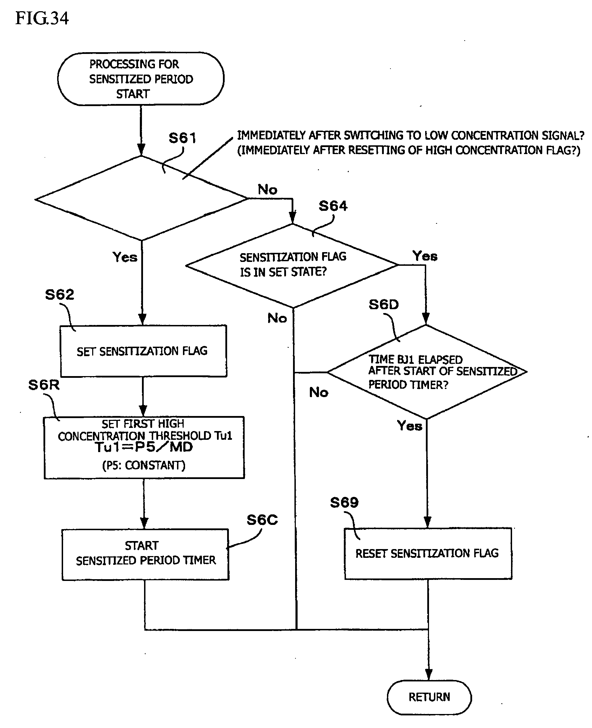

Next, there will be described the subroutine for sensitized period processing shown in FIG. 34. This subroutine is the same as the subroutine for sensitized period processing according to the third embodiment (shown in FIG. 21) except that step S6R is used in place of step S6H. Specifically, in the case where the present time is immediately after the switching to the low concentration signal (the result of determination in step S61 is Yes), after setting the sensitization flag in step S62, the microcomputer 16 sets the first concentration threshold Tu1 in step S6R, by use of the maximum difference MD previously obtained in the above-described subroutine for obtaining data for hysteresis processing, in accordance with the following Expression (18). Expression (18): Tu1 = P5/MD. Since P5 is a positive constant, the calculated first high concentration threshold Tu1 assumes a smaller value, as the maximum difference MD increases. As in the case of the third embodiment, the first high concentration threshold Tu1 is employed as the high concentration threshold Tu in step S22 (see FIG. 4) during a sensitized period in which the sensitized flag is in the set state. Therefore, the greater the maximum difference MD, the smaller the high concentration threshold Tu used in the sensitized period. Accordingly, an increase in the sensor output value, and thus an increase in gas concentration can be detected more sensitively.

The degree of the hysteresis phenomenon is considered to depend on the concentration of the specific gas in the high-concentration-signal generation period. This is because, the amount of gas molecules adsorbed by the gas sensor element is considered to increase with the concentration of the specific gas to which the gas sensor element is exposed.

Meanwhile, the start-point sensor output value Sb is considered to relate to the state in which the concentration of the specific gas is low, whereas the peak sensor output value Smax is considered to relate to the maximum value of the concentration of the specific gas. Therefore, the maximum difference MD, which is the difference between the peak sensor output value Smax and the start-point sensor output value Sb, is considered to assume a value corresponding to the degree of the hysteresis phenomenon. In view of the foregoing, in the sixth embodiment, the first high concentration threshold Tu1 is determined on the basis of the maximum difference MD. In addition, since the degree of the hysteresis phenomenon that occurs after the switching from the high concentration signal to the low concentration signal is predicted to increase as the maximum difference MD increases, the first high concentration threshold Tu1 is made smaller in order to compensate a decrease in detection sensitivity for gas concentration increase.

Notably, the processing in step S6R is performed only immediately after the switching to the low concentration signal. Further, as in the case of the second to fifth embodiments, in the sixth embodiment, the sensitized period is ended when the predetermined duration time BJ1 has elapsed after the start of the sensitized period timer in step S6C. After that, the second high concentration threshold Tu2 is used as the high concentration threshold Tu.

In this manner, the gas detection apparatus of the fifth embodiment can detect reliably, or can detect quickly, an increase in the concentration of the specific gas irrespective of occurrence of a hysteresis phenomenon.

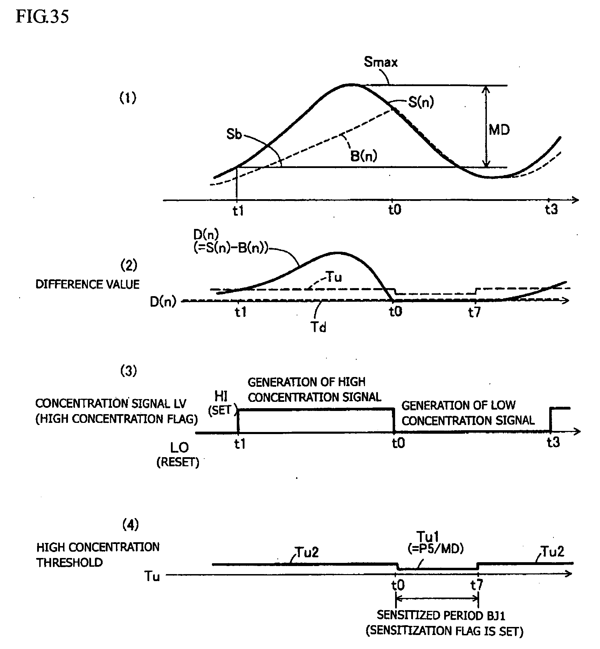

Next, with reference to FIGS. 35(1) to 35(4), there will be described the manner in which the respective values are obtained when the sensor output value S(n) changes as indicated by a solid line in FIG. 35(1). The profile of S(n) shown in FIG. 35(1) is identical with that in the case of the first through fourth embodiments. Therefore, the profiles of B(n) shown in FIG. 35(1), D(n) shown in FIG. 35(2), and the change of the high concentration flag shown in FIG. 35(3) are identical with those in the case of the first through fourth embodiments.

Meanwhile, the first high concentration threshold Tu1 is used as the high concentration threshold Tu indicated by a broken line in FIG. 35(2) and a solid line in FIG. 35(4), during the sensitized period between time t0 at which switching from the high concentration signal to the low concentration signal is effected and time t7 at which the predetermined duration time BJ1 has elapsed.

The first high concentration threshold Tu1 is determined in step S6R in accordance with Expression (18) and by use of the maximum difference MD (FIG. 35(1), which is the difference between the peak sensor output value Smax in the high-concentration-signal generation period, and the start-point sensor output value Sb, which is obtained at time t1, which is the start point of the high-concentration-signal generation period (time t1 to t0). The first high concentration threshold Tu1 is rendered to decrease as the maximum difference MD increases. In this manner, as the degree of the decrease in detection sensitivity stemming from a hysteresis phenomenon increases, the first high concentration threshold Tu1 is decreased, to thereby enable an increase in the sensor output value S(n), and thus an increase in the concentration of the oxidative gas to be detected more sensitively.

Next, a gas detection apparatus according to a seventh modification will be described. In the above-described gas detection apparatus according to the sixth embodiment, immediately after the switching to the low concentration signal, in step S6R, the first high concentration threshold Tu1 is determined in accordance with Expression (18), so that the first high concentration threshold Tu1 assumes a value corresponding to the maximum difference MD. Meanwhile, in step S6D, each sensitized period is set to the constant duration time BJ1 (see FIG. 34).

In the seventh modification, as in the case of the sixth embodiment, the first high concentration threshold Tu1 is determined in accordance with the maximum difference MD, immediately after the switching to the low concentration signal. However, in addition thereto, the length of each sensitized period (end of the sensitized period) is also set in accordance with the maximum difference MD. The remaining portions are the same as those in the sixth embodiment. Therefore, descriptions for the identical portions will be omitted or simplified, and portions different from those of the sxith embodiment will mainly be described.

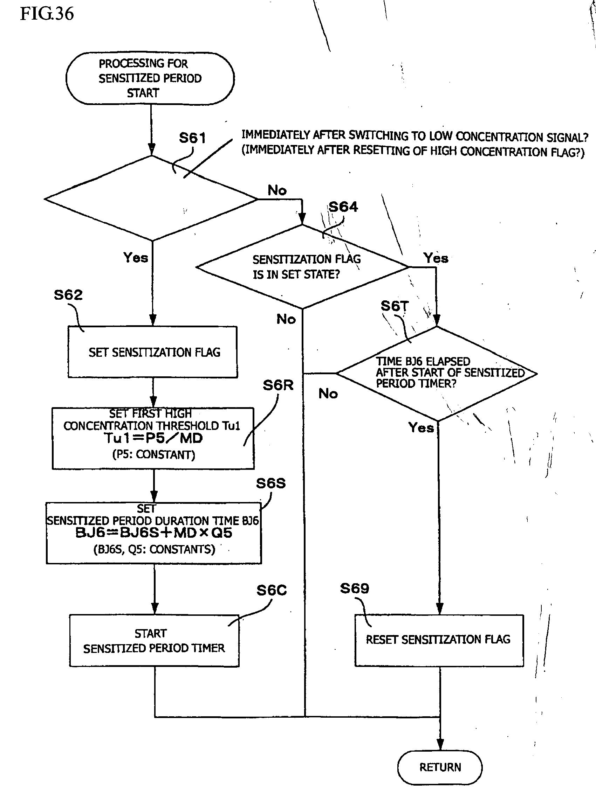

As is easily understood from comparison with the subroutine for sensitized period processing in the sixth embodiment (shown in FIG. 34), the subroutine for sensitized period processing in the seventh modification (shown in FIG. 36) has a new step S6S between steps S6R and S6C.

Specifically, as in the case of the fourth embodiment, immediately after the switching to the low concentration signal, after setting the sensitization flag in step S62, the microcomputer 16 sets the first concentration threshold Tu1 in step S6T by use of the maximum difference MD. Further, in step S6S, the microcomputer 16 sets a sensitized period duration time BJ6 by use of the following Expression (19). Expression (19): BJ6 = BJ6S + MD × Q5. Here, BJ6S and Q5 are each a positive constant. Therefore, the sensitized period duration time BJ6 assumes a larger value as the maximum difference MD increases.

In contrast, in the case where the result of the determination in step S61 is No and the result of the determination in step S64 is Yes (the case where the present time is within a sensitized period), in step S6T, the microcomputer 16 determines whether the sensitized period timer has clocked the duration time BJ6 after having started in step S6C. In the case where the result of the determination is No, the microcomputer 16 returns to the main routine. In the case where the result of the determination is Yes, the microcomputer 16 resets the sensitization flag in step S69. The end of each sensitized period is determined in this manner, so that the duration time of each sensitized period coincides with the length of the sensitized period duration time BJ6 set in step S6S.

The above-described operation is performed because of the following reason. That is, it was found that the length of a hysteresis period increases with the concentration of the specific gas to which the gas sensor element is exposed; i.e., with the maximum difference MD, which is the difference between the peak sensor output value Smax and the start-point sensor output value Sb obtained at the beginning of the high-concentration-signal generation period. In view of this, in the seventh modification, the sensitized period duration time BJ6 is set in accordance with Expression (19) in such a manner that the sensitized period duration time BJ6 increases as the maximum difference MD increases. This operation causes the length of a sensitized period to increase with the length of a hysteresis period, whereby a decrease in detection sensitivity for gas concentration increase stemming from a hysteresis phenomenon can be compensated over a proper period.

In this manner, the gas detection apparatus of the seventh modification can set a proper high concentration threshold Tu irrespective of occurrence of a hysteresis phenomenon, whereby an increase in the concentration of the specific gas can be detected reliably or quickly.

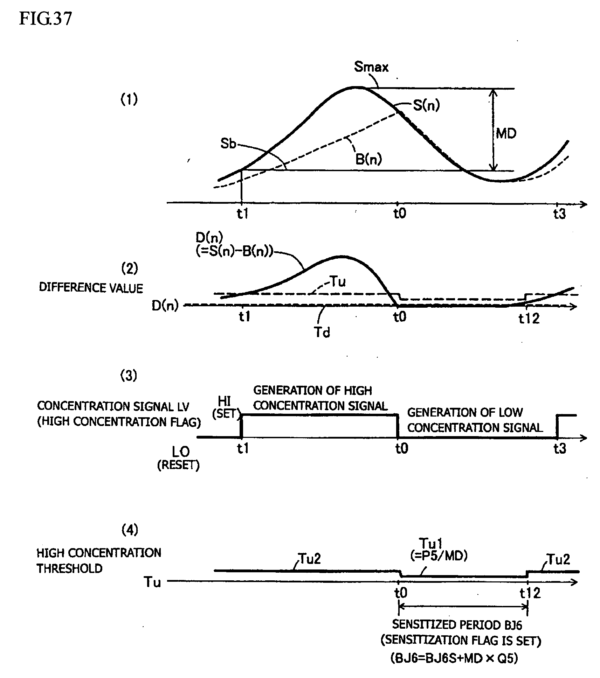

Next, with reference to FIGS. 37(1) to 37(4), there will be described the manner in which the respective values are obtained when the sensor output value S(n) changes as indicated by a solid line in FIG. 37(1). The profile of S(n) shown in FIG. 37(1) is identical with that in the case of the first through sixth embodiments. Therefore, the profiles of B(n) shown in FIG. 37(1), D (n) shown in FIG. 37(2), and the change of the high concentration flag shown in FIG. 37(3) are identical with those in the case of the sixth embodiment.

Meanwhile, the first high concentration threshold Tu1 is used as the high concentration threshold Tu shown by a solid line in FIG. 37(4) and a broken line in FIG. 37(2) during a sensitized period between time t0 at which switching from the high concentration signal to the low concentration signal is effected and time t12 at which the sensitized period duration time BJ6 has elapsed. As in the case of the sixth embodiment, the first high concentration threshold Tu1 and the high concentration threshold Tu are determined by the processing in step S6R in accordance with Expression (18) and by use of the maximum difference MD (see FIG. 37(1)), which is the difference between the peak sensor output value Smax in a high-concentration-signal generation period, and a start-point sensor output value Sb obtained at time t1, which is the start point of the high-concentration-signal generation period (time t1 to t0). The first high concentration threshold Tu1 is rendered to decrease as the maximum difference MD increases.

Further, in the seventh modification, the length of the sensitized period duration time BJ6 is also determined in accordance with Expression (19) and by use of the maximum difference MD obtained in the high-concentration-signal generation period (time t1 to t0). The sensitized period duration time BJ6 is rendered to increase as the maximum difference MD increases.

In this manner, by use of the sensitized period duration time BJ6 corresponding to the maximum difference MD, the length of each sensitized period can be increased with the length of a hysteresis period, whereby a decrease in detection sensitivity for an increase in the concentration of the oxidative gas can be compensated over a proper period.

Next, a gas detection apparatus according to a seventh embodiment will be described. In the above-described sixth embodiment, by use of the subroutine for obtaining data for hysteresis processing shown in FIG. 33, the microcomputer 16 obtains a maximum difference MD, which is the difference between a start-point sensor output value which is obtained at the beginning of a high-concentration-signal generation period and a peak sensor output value, which is the maximum sensor output value obtained in that period. Further, the microcomputer 16 determines the first high concentration threshold Tu1 from the maximum difference MD, which is the difference between the start-point sensor output value which is obtained at the beginning of the high-concentration-signal generation period and the maximum sensor output value (peak sensor output value) obtained in that period (see step S6R in FIG. 34). Meanwhile, the length (end) of each sensitized period is previously determined in step S6D by use of the constant duration time BJ1.

Moreover, in the seventh modification, as in the case of the sixth embodiment, the first high concentration threshold Tu1 is set in accordance with the maximum difference MD, immediately after the switching to the low concentration signal, and the length of each sensitized period (end of the sensitized period) is also set in accordance with the maximum difference MD.

In the seventh embodiment, as in the case of the sixth embodiment and the seventh modification, the first high concentration threshold Tu1 is set in accordance with the maximum difference MD, immediately after the switching to the low concentration signal. However, the end of each sensitized period is not set previously, and each sensitized period is ended when the sensor output value S(n) becomes equal to or smaller than a predetermined cancel threshold. Specifically, each sensitized period is ended when the sensor output value S(n) becomes equal to or smaller than a predetermined cancel threshold which is an intermediate value between a start-point sensor output value which is obtained at the beginning of a high-concentration-signal generation period immediately before the switching to the low concentration signal, and the maximum sensor output value (peak sensor output value) obtained in that period.

Therefore, portions different from those of the sixth embodiment will mainly be described, and descriptions for the identical portions will be omitted or simplified.

In the seventh embodiment, as in the case of the sixth embodiment, the maximum difference MD is previously obtained by use of the subroutine for obtaining data for hysteresis processing shown in FIG. 33.

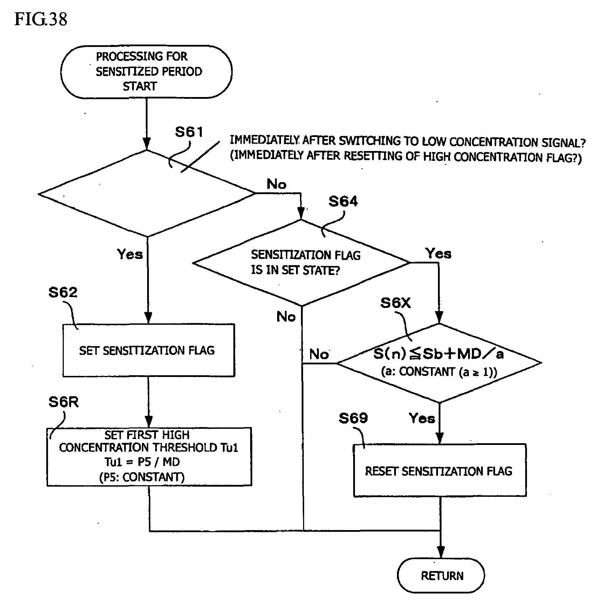

Next, there will be described the subroutine for sensitized period processing shown in FIG. 38. This subroutine is the same as the subroutine for sensitized period processing according to the sixth embodiment (shown in FIG. 34) except that step S6C is removed, and step S6X is used in place of step S6D. Specifically, in the case where the present time is immediately after the switching to the low concentration signal (the result of determination in step S61 is Yes), after setting the sensitization flag in step S62, the microcomputer 16 sets the first concentration threshold Tu1 in step S6R, by use of the maximum difference MD previously,obtained in the above-described subroutine for obtaining data for hysteresis processing, in accordance with the following Expression (18). Expression (18): Tu1 = P5/MD. Since P5 is a positive constant, the calculated first high concentration threshold Tu1 assumes a smaller value, as the maximum difference MD increases. As in the case of the sixth embodiment, the first high concentration threshold Tu1 is employed as the high concentration threshold Tu in step S22 (see FIG. 4) during a sensitized period in which the sensitized flag is in the set state. Therefore, the greater the maximum difference MD, the smaller the high concentration threshold Tu used in the sensitized period. Accordingly, an increase in the sensor output value, and thus an increase in gas concentration can be detected more sensitively.

Notably, the processing in step S6R is performed only immediately after the switching to the low concentration signal.

In contrast, in the case where the result of determination in step S61 is No and the result of determination in step S64 is Yes (the case where the present time is in a sensitized period), in step S6X the microcomputer 16 determines whether the present sensor output value S(n) becomes equal to or smaller than the cancel threshold. An intermediate value between the start-point sensor output value Sb and the peak sensor output value Smax is selected as a cancel threshold. Specifically, the cancel threshold is calculated by use of the start-point sensor output value Sb, the maximum difference MD (= Smax - Sb), and a constant a (Sb + MD/a). Here, a is a constant greater than 1 (a > 1). In the case where the result of determination in step S6W is No, the microcomputer 16 returns to the main routine. In the case where the result of determination in step S6W is Yes, in step S69, the microcomputer 16 resets the sensitization flag. Since the end of the sensitized period is determined in this manner, the duration time of the sensitized period changes in response to changes in the sensor output value S(n).

The cancel threshold (Sb + MD/a) obtained in the above-described manner is used because of the following reason. If the sensor output value is assumed to involve no drift stemming from, for example, a change in humidity or temperature of the environment, a hysteresis phenomenon is considered to end when, after passage of a high-concentration-signal generation period at the beginning of which the gas concentration has been determined to increase, the sensor output value returns to the same value as the sensor output value (start-point sensor output value Sb) obtained at the beginning of that period. Meanwhile, if the sensor output value drifts toward the increasing direction stemming from, for example, a change in temperature or humidity, the sensor output value obtained when the hysteresis phenomenon has ended becomes greater than the start-point sensor output value Sb. Therefore, if the start-point sensor output value Sb is used, as a threshold for ending the sensitized period, in the case where the sensor output value may possibly drift, the sensitized period may unnecessarily continues for a long time because the sensor output value fails to become smaller than the start-point sensor output value Sb. In order to solve this problem, in the seventh embodiment, a value (Sb + MD/a) which is greater than the start-point sensor output value Sb by MD/a is used as a cancel threshold. Thus, the sensitized period can be ended properly even when the sensor output value involves drift. Meanwhile, each sensitized period can be continued until a hysteresis phenomenon ends substantially, a drop in sensitivity for gas concentration increase stemming from a hysteresis phenomenon can be compensated properly.

Notably, as in the case of the second through sixth embodiments, in the seventh embodiment, after the sensitized period ends, the second high concentration threshold Tu2 is used as the high concentration threshold Tu.

In this manner, the gas detection apparatus of the seventh embodiment can detect reliably, or can detect quickly, an increase in the concentration of the specific gas irrespective of occurrence of a hysteresis phenomenon.

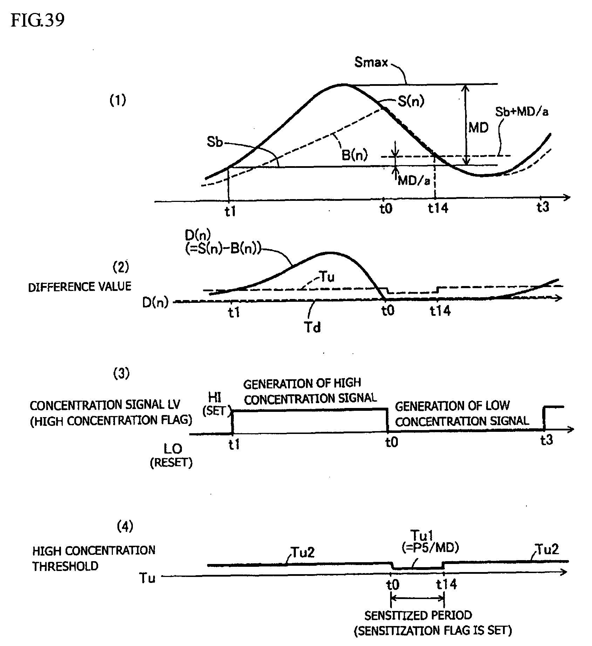

Next, with reference to FIGS. 39(1) to 39(4), there will be described the manner in which the respective values are obtained when the sensor output value S(n) changes as indicated by a solid line in FIG. 39(1). The profile of S(n) shown in FIG. 39(1) is identical with that in the case of the first through sixth embodiments. Therefore, the profiles of B(n) shown in FIG. 39(1), D(n) shown in FIG. 39(2), and the change of the high concentration flag shown in FIG. 39(3) are identical with those in the case of the first through sixth embodiments.

Meanwhile, the first high concentration threshold Tu1 is used as the high concentration threshold Tu indicated by a solid line in FIG. 39(4) and a broken line in FIG. 39(2), during a sensitized period between time t0 at which switching from the high concentration signal to the low concentration signal is effected and time t14 at which a sensitized period ends. As in the case of the sixth embodiment, the first high concentration threshold Tu1 and the high concentration threshold Tu are determined in step S6R in accordance with Expression (18) and by use of the maximum difference MD (FIG. 39(1), which is the difference between the peak sensor output value Smax in a high-concentration-signal generation period, and a start-point sensor output value Sb, which is obtained at time t1, which is the start point of the high-concentration-signal generation period (time t1 to t0). The first high concentration threshold Tu1 is rendered to decrease as the maximum difference MD increases.

Further, in the seventh embodiment, the time (t14) at which the sensitized period ends is determined by use of the cancel threshold. Specifically, as shown in FIG. 39(1), at time t14 at which S(n) ≤ Sb + MD/a is satisfied, the sensitized period is ended, and in place of the first high concentration threshold Tu1, the second high concentration threshold Tu2 is used as the high concentration threshold Tu (see FIG. 39(4)). Notably, the end point (time t14) of the sensitized period can be adjusted by changing the constant a. The greater the constant a, the more the end point of the sensitized period delays, whereby the length of the sensitized period increases.

When the sensor output value S(n)' decreases to a small value that is about the same as the start-point sensor output value Sb, the hysteresis phenomenon is considered to have ended substantially. Meanwhile, when the sensor output value S(n) drifts after time t0 stemming from, for example, a change in humidity or temperature, the sensor output value S(n) may fail to decrease to the start-point sensor output value Sb. In view of the above, a value which is greater than the start-point sensor output value Sb by MD/a is used as the cancel threshold, whereby the sensitized period can be ended properly even when the sensor output value drifts. Further, in the case where the hysteresis phenomenon continues, the sensor output value is considered to maintain a relatively large magnitude, and the sensor output value does not quickly decrease to the cancel threshold or smaller. Therefore, the length of the sensitized period is increased with the length of the hysteresis period, whereby a drop in detection sensitivity for an increase in the concentration of the oxidative gas can be compensated.

Notably, the seventh embodiment uses the cancel threshold (Sb + MD/a), which is calculated by use of the start-point sensor output value Sb and the maximum difference MD.

However, the cancel threshold may be a value (Sb + SD/a1), which is calculated by use of the start-point sensor output value Sb and the difference value sum SD, which is used in the fifth embodiment and is obtained at time t0 immediately after the switching to the low concentration signal. Here, a1 is a constant that is greater than 1 (a1 > 1).

The present invention has been described with reference to the first through seventh embodiments and the first through seventh modifications. However, needless to say, the present invention is not limited to the above-described embodiments and modifications, and may be practiced in a freely modified form, without departing from the scope of the invention.

For example, in the second to sixth embodiments, etc., the first high concentration threshold Tu1 to be used in a sensitized period is set by Expression (7), (12), (14), (16), or (18). In these expressions, the first high concentration threshold Tu1 is set by use of a single piece of hysteresis strength information, such as the moving difference value DM(n) or the maximum difference value Dmax. However, the first high concentration threshold Tu1 may be set by use of two ore more pieces of hysteresis strength information.

Similarly, in the fourth to ninth modifications, etc., the length (end point) of each sensitized period is determined on the basis of the sensitized-period duration time BJ2, BJ3, etc., which is obtained by Expression (10), (13), (15), (17), or (19). In these expressions, the sensitized-period duration time BJ2, BJ3, etc. is set by use of a single piece of hysteresis period information, such as the moving difference value DM(n) or the maximum difference value Dmax. However, the sensitized-period duration time BJ2, BJ3, etc. may be set by use of two or more pieces of hysteresis period information.

In the first through seventh embodiments and the first through seventh modifications, there is used the gas sensor element 11 which is formed of an oxide semiconductor and which reacts to an oxidative gas, so that its sensor resistance Rs increases as the concentration of the oxidative gas component increases. Further, there is used the sensor resistance conversion circuit 14 whose sensor output potential Vs increases as the sensor resistance Rs increases. Therefore, there is used the gas detection apparatus 10 designed such that the increase direction of the sensor output value S(n) corresponds to the high concentration direction in relation to the sensor output value S(n).

However, there may be used a gas detection apparatus designed such that the decrease direction of the sensor output value S(n) corresponds to the high concentration direction in relation to the sensor output value S(n). Further, there is used a sensor resistance conversion circuit whose sensor output potential Vs decreases as the sensor resistance Rs increases. Moreover, there may be used a conversion circuit which converts the sensor resistance to voltage or the like by a circuit configuration and a principle, which differ from those of the sensor resistance conversion circuit 14 shown in FIG. 1. Furthermore, there may be used a gas sensor element which is formed of an oxide semiconductor and which reacts to a reducing gas, so that its sensor resistance Rs decreases as the concentration of the reducing gas component increases. Further, there may be used a gas sensor element which reacts to both an oxidative gas and a reducing gas.

In the first through seventh embodiments and the first through seventh modifications, the base value B(n) is obtained from the obtained sensor output value S(n) by use of Expression (1), (2), etc. However, instead of the base value B(n) obtained by use of these expressions, other expressions may be used to obtain a reference value.

Further, needless to say, as in the case of the first embodiment, the gas detection apparatus of the second through seventh embodiments and the first through seventh modifications may be applied to an automatic ventilation system for a vehicle.

## Claims

1. A gas detection apparatus using a gas sensor element (11) whose sensor resistance changes in accordance with concentration of a specific gas, comprising:
acquisition means (19) for acquiring a sensor output value at predetermined cycle intervals, the sensor output value corresponding to the sensor resistance; and
concentration detection means for detecting a change in the concentration of the specific gas and for generating a low concentration signal when the concentration of the specific gas is low and a high concentration signal when the concentration of the specific gas is high,
wherein the concentration detection means (S3B) comprises concentration increase detection means for generating the high concentration signal in place of the low concentration signal when, during a period in which the low concentration signal is generated, the sensor output value shifts toward a high concentration direction with respect to a reference value (B(n)) by an amount equal to or greater than a high concentration threshold (Tu, Tu1, Tu2), and concentration decrease detection means (S3C) for generating the low concentration signal in place of the high concentration signal when, during a period in which the high concentration signal is generated, the sensor output value shifts toward a low concentration direction with respect to the reference value by an amount equal to or greater than a low concentration threshold (Td)
wherein the concentration increase detection means uses
a first high concentration threshold (Tu1) as the high concentration threshold during a sensitized period of the period in which the low concentration signal is generated, the sensitized period following a switching from the high concentration signal to the low concentration signal, and
a second high concentration threshold (Tu2) as the high concentration threshold during the remaining portion of the period in which the low concentration signal is generated, and
wherein the first high concentration threshold is a value on a sensitivity-increasing-direction side with respect to the second high concentration threshold.

2. A gas detection apparatus according to claim 1, comprising first-high-concentration-threshold update means for causing, in the sensitized period, the first high concentration threshold (Tu1) to gradually approach the second high concentration threshold (Tu2) with elapse of time.

3. A gas detection apparatus according to claim 1 or 2, comprising first-high-concentration-threshold determining means for determining the first high concentration threshold on the basis of hysteresis strength information.

4. A gas detection apparatus according to claim 3, comprising:
threshold-determining moving-difference-value calculation means for calculating a first-high-concentration-threshold-determining moving difference value from a present sensor output value and an m^{th} past sensor output value which is a sensor output value obtained m cycles before the present sensor output value (m is an integer not less than 1),
wherein the hysteresis strength information includes the first-high-concentration-threshold-determining moving difference value which is obtained at the time of switching from the high concentration signal to the low concentration signal, a point in time a predetermined time earlier than the time of switching, or a point in time a predetermined time later than the time of switching, and
wherein the first-high-concentration-threshold update means shifts the first high concentration threshold (Tu1) in the sensitivity increasing direction in accordance with the absolute value of the first-high-concentration-threshold-determining moving difference value, which is used as the hysteresis strength information.

5. A gas detection apparatus according to claim 3 or 4, wherein the hysteresis strength information includes a maximum difference which is a difference between a peak sensor output value and a start-point sensor output value, the peak sensor output value being a value that shifts in the high concentration direction to the greatest degree among sensor output values obtained in a high-concentration-signal generation period immediately before the time of switching from the high concentration signal to the low concentration signal, and the start-point sensor output value being a sensor output value obtained at the beginning of the high-concentration-signal generation period, and
wherein the first-high-concentration-threshold update means shifts the first high concentration threshold in the sensitivity increasing direction as the absolute value of the maximum difference increases.

6. A gas detection apparatus according to claim 3 or 4, wherein the hysteresis strength information includes a maximum difference value which is a difference selected from differences between the reference value and respective sensor output values obtained in a high-concentration-signal generation period immediately before the time of switching from the high concentration signal to the low concentration signal, the selected difference having the highest absolute value among the differences, and
wherein the first-high-concentration-threshold update means shifts the first high concentration threshold (Tu1) in the sensitivity increasing direction as the absolute value of the maximum difference increases.

7. A gas detection apparatus according to any one of claims 3 to 6, wherein the hysteresis strength information includes duration time of a high-concentration-signal generation period immediately before the time of switching from the high concentration signal to the low concentration signal, and
wherein the first-high-concentration-threshold update means shifts the first high concentration threshold in the sensitivity increasing direction as the length of the duration time increases.

8. A gas detection apparatus according to any one of claims 1 to 7, comprising end means for ending the sensitized period when a first predetermined period of time has passed after the time of switching from the high concentration signal to the low concentration signal.

9. A gas detection apparatus according to any one of claims 1 to 7, comprising end-timing advance determining means for previously determining the end of the sensitized period on the basis of hysteresis period information.

10. A gas detection apparatus according to claim 9, comprising:
end-timing-determining moving-difference-value calculation means for calculating an end-timing-determining moving difference value from a present sensor output value and an j^{th} past sensor output value which is a sensor output value obtained j cycles before the present sensor output value (j is an integer not less than 1),
wherein the hysteresis period information includes the end-timing-determining moving difference value which is obtained at the time of switching from the high concentration signal to the low concentration signal, a point in time a predetermined time earlier than the time of switching, or a point in time a predetermined time later than the time of switching, and
wherein the end-timing advance determining means delays the end of the sensitized period to a greater degree with increasing absolute value of the end-timing-determining moving difference value, which is used as the hysteresis period information.

11. A gas detection apparatus according to claim 9 or 10, wherein the hysteresis period information includes a maximum difference which is a difference between a peak sensor output value and a start-point sensor output value, the peak sensor output value being a value that shifts in the high concentration direction to the greatest degree among sensor output values obtained in a high-concentration-signal generation period immediately before the time of switching from the high concentration signal to the low concentration signal, and the start-point sensor output value being a sensor output value obtained at the beginning of the high-concentration-signal generation period, and
wherein the end-timing advance determining means delays the end of the sensitized period to a greater degree with increasing absolute value of the maximum difference.

12. A gas detection apparatus according to claim 9 or 10, wherein the hysteresis period information includes a maximum difference value which is a difference selected from differences between the reference value and respective sensor output values obtained in a high-concentration-signal generation period immediately before the time of switching from the high concentration signal to the low concentration signal, the selected difference having the largest absolute value among the differences,
wherein the end-timing advance determining means delays the end of the sensitized period to a greater degree with increasing absolute value of the maximum difference.

13. A gas detection apparatus according to any one of claims 9 to 12, wherein the hysteresis period information includes duration time of a high-concentration-signal generation period immediately before the time of switching from the high concentration signal to the low concentration signal, and
wherein the end-timing advance determining means delays the end of the sensitized period to a greater degree as the length of the duration time increases.

14. A gas detection apparatus according to any one of claims 1 to 7, comprising:
alternation-determining moving-difference-value calculation means for calculating an alternation-determining moving difference value from a present sensor output value and an i^{th} past sensor output value which is a sensor output value obtained i cycles before the present sensor output value (i is an integer not less than 1); and
moving-difference-value determining means for determining whether the alternation-determining moving difference value is located on the side toward 0 with respect to a predetermined value serving as a reference,
wherein the concentration increase detection means uses, in place of the first high concentration threshold (Tu1), the second high concentration threshold (Tu2) as the high concentration threshold after the alternation-determining moving difference value has shifted to the side toward 0.

15. A gas detection apparatus according to any one of claims 1 to 7, comprising:
alternation-determining moving-difference-value calculation means for calculating an alternation-determining moving difference value from a present sensor output value and an i^{th} past sensor output value which is a sensor output value obtained i cycles before the present sensor output value (i is an integer not less than 1); and
moving-difference-value determining means for determining whether the alternation-determining moving difference value is located on the side toward 0 with respect to a predetermined value serving as a reference,
wherein the concentration increase detection means uses, in place of the first high concentration threshold (Tu1), the second high concentration threshold (Tu2) as the high concentration threshold after elapse of a predetermined period of time after the alternation-determining moving difference value has shifted to the side toward 0.

16. A gas detection apparatus according to claim 14 or 15, wherein from the time of switching from the high concentration signal to the low concentration signal, the alternation-determining moving-difference-value calculation means starts the calculation of the alternation-determining moving difference value, or resumes the calculation of the alternation-determining moving difference value without use of sensor output values obtained before the time of switching, and
wherein during a period from the time of switching up to a point in time at which obtainment of sensor output values corresponding to the predetermined number (i) of cycles is completed, the difference between a sensor output value obtained at the time of switching and a sensor output value obtained in each subsequent cycle is used as the alternation-determining moving difference value in respective cycles.

17. A gas detection apparatus according to claim 14 or 15, wherein from the time of switching from the high concentration signal to the low concentration signal, the alternation-determining moving-difference-value calculation means starts the calculation of the alternation-determining moving difference value, or resumes the calculation of the alternation-determining moving difference value without use of sensor output values obtained before the time of switching, and
wherein during a period from the time of switching up to a point in time at which obtainment of sensor output values corresponding to the predetermined number (i) of cycles is completed, a value obtained through multiplying, by i/b, the difference between a sensor output value obtained at the time of switching and a sensor output value obtained b cycles after the time of switching is used as the alternation-determining moving difference value in respective cycles.

18. A gas detection apparatus according to any one of claims 1 to 7, comprising:
determination means for determining whether the present sensor output value has shifted to the side toward the low concentration direction with respect to a cancel threshold which is an intermediate value between a start-point sensor output value and a peak sensor output value, the start-point sensor output value being a sensor output value obtained at the beginning of a high-concentration-signal generation period immediately before the switching from the high concentration signal to the low concentration signal, and the peak sensor output value being a value that shifts in the high concentration direction to the greatest degree among sensor output values obtained in the high-concentration-signal generation period,
wherein the concentration increase detection means uses, in place of the first high concentration threshold (Tu1), the second high concentration threshold (Tu2) as the high concentration threshold when the present sensor output value has shifted to the side toward the low concentration direction with respect to the cancel threshold.

19. A gas detection apparatus according to claim 18, wherein the cancel threshold is a value which is shifted toward the peak sensor output value from the start-point sensor output value by an amount obtained through division by a of the difference between the peak sensor output value and the start-point sensor output value, where a > 1.

20. A gas detection apparatus according to claim 1,
wherein the concentration detection means comprises end-timing determining means for determining the end of said sensitized period.

21. An automatic ventilation system for a vehicle comprising a gas detection apparatus (10) according to any of claims 1 to 20.

22. A method of operating a gas detection apparatus with a gas sensor element whose sensor resistance changes in accordance with concentration of a specific gas, acquisition means for acquiring a sensor output value at predetermined cycle intervals, the sensor output value corresponding to the sensor resistance and with concentration detection means for detecting a change in the concentration of the specific gas and for generating a low concentration signal when the concentration of the specific gas is low and a high concentration signal when the concentration of the specific gas is high,
comprising the steps of:
generating the high concentration signal in place of the low concentration signal with a concentration increase detection means when, during a period in which the low concentration signal is generated, the sensor output value shifts toward a high concentration direction with respect to a reference value by an amount equal to or greater than a high concentration threshold, and
generating the low concentration signal in place of the high concentration signal with a concentration decrease detection means when, during a period in which the high concentration signal is generated, the sensor output value shifts toward a low concentration direction with respect to the reference value by an amount equal to or greater than a low concentration threshold,
wherein the concentration increase detection means uses
a first high concentration threshold as the high concentration threshold during a sensitized period of the period in which the low concentration signal is generated, the sensitized period following a switching from the high concentration signal to the low concentration signal, and
a second high concentration threshold as the high concentration threshold during the remaining portion of the period in which the low concentration signal is generated, and
wherein the first high concentration threshold is a value on a sensitivity-increasing-direction side with respect to the second high concentration threshold.

23. A method of operating a gas detection apparatus according to claim 22, further comprising the step of:
causing, in the sensitized period, the first high concentration threshold to gradually approach the second high concentration threshold with elapse of time with first-high-concentration-threshold update means.

24. A method of operating a gas detection apparatus according to claim 22 or 23, further comprising the step of:
determining the first high concentration threshold on the basis of hysteresis strength information with a first-high-concentration-threshold determining means.

25. A method of operating a gas detection apparatus as described in claim 24, further comprising the step of:
calculating a first-high-concentration-threshold-determining moving difference value with a threshold-determining moving-difference-value calculation means from a present sensor output value and an m^{th} past sensor output value which is a sensor output value obtained m cycles before the present sensor output value (m is an integer not less than 1),
wherein the hysteresis strength information includes the first-high-concentration-threshold-determining moving difference value which is obtained at the time of switching from the high concentration signal to the low concentration signal, a point in time a predetermined time earlier than the time of switching, or a point in time a predetermined time later than the time of switching, and
wherein the first high concentration threshold is shifted with the first-high-concentration-threshold update means in the sensitivity increasing direction in accordance with the absolute value of the first-high-concentration-threshold-determining moving difference value, which is used as the hysteresis strength information.

26. A method of operating a gas detection apparatus according to claim 22, further comprising
determining the end of a sensitized period with a end-timing determining means.

27. A method of operating a gas detection apparatus according to claim 22, wherein the sensitized period starts at the switching from the high concentration signal to the low concentration signal, and ends at a point in time after the direction of change of the sensor output value changes from the low concentration direction to the high concentration direction.

## Patentansprüche

1. Gasdetektionsvorrichtung, welche ein Gassensorelement (11) verwendet, dessen Sensorwiderstand sich entsprechend der Konzentration eines spezifischen Gases ändert, aufweisend:
Erfassungsmittel (19) zum Erfassen eines Sensorausgangswertes in vorbestimmten Zyklusintervallen, wobei der Sensorausgangswert dem Sensorwiderstand entspricht; und
Konzentrationsdetektionsmittel zum Detektieren einer Änderung in der Konzentration des spezifischen Gases und zum Erzeugen eines Niedrigkonzentrationssignals, wenn die Konzentration des spezifischen Gases niedrig ist, und eines Hochkonzentrationssignals, wenn die Konzentration des spezifischen Gases hoch ist,
wobei das Konzentrationsdetektionsmittel (S3B) aufweist:
Konzentrationserhöhungs-Detektionsmittel zum Erzeugen des Hochkonzentrationssignals anstelle des Niedrigkonzentrationssignals, wenn während eines Zeitraums, in welchem das Niedrigkonzentrationssignal erzeugt wird, der Sensorausgangswert sich in einer Richtung hoher Konzentration in Bezug auf einen Referenzwert (B(n)) um einen Betrag verschiebt, der gleich einem oder größer als ein Hochkonzentrationsschwellwert (Tu, Tu1, Tu2) ist, und
Konzentrationsverringerungs-Detektionsmittel (S3C) zum Erzeugen des Niedrigkonzentrationssignals anstelle des Hochkonzentrationssignals, wenn während eines Zeitraums, in welchem das Hochkonzentrationssignal erzeugt wird, der Sensorausgangswert sich in einer Richtung niedriger Konzentration in Bezug auf den Referenzwert um einen Betrag verschiebt, der gleich einem oder größer als ein Niedrigkonzentrationsschwellwert (Td) ist,
wobei das Konzentrationserhöhungs-Detektionsmittel verwendet:
einen ersten Hochkonzentrationsschwellwert (Tu1) als den Hochkonzentrationsschwellwert während eines sensibilisierten Zeitabschnitts des Zeitabschnitts, in welchem das Niedrigkonzentrationssignal erzeugt wird, wobei der sensibilisierte Zeitabschnitt auf eine Umschaltung von dem Hochkonzentrationssignal zu dem Niedrigkonzentrationssignal folgt, und
einen zweiten Hochkonzentrationsschwellwert (Tu2) als den Hochkonzentrationsschwellwert während des restlichen Teils des Zeitabschnitts, in welchem das Niedrigkonzentrationssignal erzeugt wird, und
wobei der erste Hochkonzentrationsschwellwert ein Wert auf einer Seite der Richtung zunehmender Empfindlichkeit in Bezug auf den zweiten Hochkonzentrationsschwellwert ist.

2. Gasdetektionsvorrichtung nach Anspruch 1, welche Aktualisierungsmittel für den ersten Hochkonzentrationsschwellwert aufweist, zum Bewirken, in dem sensibilisierten Zeitabschnitt, dass sich der erste Hochkonzentrationsschwellwert (Tu1) im Laufe der Zeit allmählich an den zweiten Hochkonzentrationsschwellwert (Tu2) annähert.

3. Gasdetektionsvorrichtung nach Anspruch 1 oder 2, welche Bestimmungsmittel für den ersten Hochkonzentrationsschwellwert aufweist, zum Bestimmen des ersten Hochkonzentrationsschwellwertes auf der Basis von Hysteresestärke-Information.

4. Gasdetektionsvorrichtung nach Anspruch 3, welche aufweist:
Berechnungsmittel für den Wert einer gleitenden Differenz zur Schwellwertbestimmung, zum Berechnen eines Wertes einer gleitenden Differenz zur Bestimmung des ersten Hochkonzentrationsschwellwertes aus einem gegenwärtigen Sensorausgangswert und einem m-ten vergangenen Sensorausgangswert, welcher ein Sensorausgangswert ist, der m Zyklen vor dem gegenwärtigen Sensorausgangswert erhalten wurde (m ist eine ganze Zahl, die nicht kleiner als 1 ist),
wobei die Hysteresestärke-Information den Wert der gleitenden Differenz zur Bestimmung des ersten Hochkonzentrationsschwellwertes beinhaltet, welcher zum Zeitpunkt der Umschaltung von dem Hochkonzentrationssignal zu dem Niedrigkonzentrationssignal, einem Zeitpunkt, der um eine vorbestimmte Zeit früher als der Zeitpunkt der Umschaltung ist, oder einem Zeitpunkt, der um eine vorbestimmte Zeit später als der Zeitpunkt der Umschaltung ist, erhalten wird, und
wobei das Aktualisierungsmittel für den ersten Hochkonzentrationsschwellwert den ersten Hochkonzentrationsschwellwert (Tu1) entsprechend dem absoluten Betrag des Wertes der gleitenden Differenz zur Bestimmung des ersten Hochkonzentrationsschwellwertes, welcher als die Hysteresestärke-Information verwendet wird, in die Richtung zunehmender Empfindlichkeit verschiebt.

5. Gasdetektionsvorrichtung nach Anspruch 3 oder 4, wobei die Hysteresestärke-Information eine maximale Differenz beinhaltet, welche eine Differenz zwischen einem Spitzen-Sensorausgangswert und einem Anfangspunkt-Sensorausgangswert ist, wobei der Spitzen-Sensorausgangswert ein Wert ist, welcher unter Sensorausgangswerten, die in einem Zeitabschnitt der Erzeugung des Hochkonzentrationssignals unmittelbar vor dem Zeitpunkt der Umschaltung von dem Hochkonzentrationssignal zu dem Niedrigkonzentrationssignal erhalten wurden, der stärksten Verschiebung in die Richtung hoher Konzentration entspricht, und der Anfangspunkt-Sensorausgangswert ein Sensorausgangswert ist, der zu Beginn des Zeitabschnitts der Erzeugung des Hochkonzentrationssignals erhalten wurde, und
wobei das Aktualisierungsmittel für den ersten Hochkonzentrationsschwellwert den ersten Hochkonzentrationsschwellwert in die Richtung zunehmender Empfindlichkeit verschiebt, wenn sich der absolute Betrag der maximalen Differenz erhöht.

6. Gasdetektionsvorrichtung nach Anspruch 3 oder 4, wobei die Hysteresestärke-Information einen maximalen Differenzwert beinhaltet, welcher eine Differenz ist, die aus Differenzen zwischen dem Referenzwert und jeweiligen Sensorausgangswerten ausgewählt ist, die in einem Zeitabschnitt der Erzeugung des Hochkonzentrationssignals unmittelbar vor dem Zeitpunkt der Umschaltung von dem Hochkonzentrationssignal zu dem Niedrigkonzentrationssignal erhalten wurden, wobei die ausgewählte Differenz den größten absoluten Betrag unter den Differenzen hat, und
wobei das Aktualisierungsmittel für den ersten Hochkonzentrationsschwellwert den ersten Hochkonzentrationsschwellwert (Tu1) in die Richtung zunehmender Empfindlichkeit verschiebt, wenn sich der absolute Betrag der maximalen Differenz erhöht.

7. Gasdetektionsvorrichtung nach einem der Ansprüche 3 bis 6, wobei die Hysteresestärke-Information die Dauer eines Zeitabschnitts der Erzeugung des Hochkonzentrationssignals unmittelbar vor dem Zeitpunkt der Umschaltung von dem Hochkonzentrationssignal zu dem Niedrigkonzentrationssignal beinhaltet, und
wobei das Aktualisierungsmittel für den ersten Hochkonzentrationsschwellwert den ersten Hochkonzentrationsschwellwert in die Richtung zunehmender Empfindlichkeit verschiebt, wenn sich die Dauer verlängert.

8. Gasdetektionsvorrichtung nach einem der Ansprüche 1 bis 7, welche Beendigungsmittel zum Beenden des sensibilisierten Zeitabschnitts aufweist, wenn ein erster vorbestimmter Zeitabschnitt nach dem Zeitpunkt der Umschaltung von dem Hochkonzentrationssignal zu dem Niedrigkonzentrationssignal vergangen ist.

9. Gasdetektionsvorrichtung nach einem der Ansprüche 1 bis 7, welche Endzeitpunkt-Vorausbestimmungsmittel aufweist, zum Vorausbestimmen des Endes des sensibilisierten Zeitabschnitts auf der Basis von Hystereseperiode-Information.

10. Gasdetektionsvorrichtung nach Anspruch 9, welche aufweist:
Berechnungsmittel für den Wert einer gleitenden Differenz zur Endzeitpunktbestimmung, zum Berechnen eines Wertes einer gleitenden Differenz zur Endzeitpunktbestimmung aus einem gegenwärtigen Sensorausgangswert und einem j-ten vergangenen Sensorausgangswert, welcher ein Sensorausgangswert ist, der j Zyklen vor dem gegenwärtigen Sensorausgangswert erhalten wurde (j ist eine ganze Zahl, die nicht kleiner als 1 ist),
wobei die Hystereseperiode-Information den Wert der gleitenden Differenz zur Endzeitpunktbestimmung beinhaltet, welcher zum Zeitpunkt der Umschaltung von dem Hochkonzentrationssignal zu dem Niedrigkonzentrationssignal, einem Zeitpunkt, der um eine vorbestimmte Zeit früher als der Zeitpunkt der Umschaltung ist, oder einem Zeitpunkt, der um eine vorbestimmte Zeit später als der Zeitpunkt der Umschaltung ist, erhalten wird, und
wobei das Endzeitpunkt-Vorausbestimmungsmittel das Ende des sensibilisierten Zeitabschnitts mit zunehmendem absolutem Betrag des Wertes der gleitenden Differenz zur Endzeitpunktbestimmung, welcher als die Hystereseperiode-Information verwendet wird, stärker verzögert.

11. Gasdetektionsvorrichtung nach Anspruch 9 oder 10, wobei die Hystereseperiode-Information eine maximale Differenz beinhaltet, welche eine Differenz zwischen einem Spitzen-Sensorausgangswert und einem Anfangspunkt-Sensorausgangswert ist, wobei der Spitzen-Sensorausgangswert ein Wert ist, welcher unter Sensorausgangswerten, die in einem Zeitabschnitt der Erzeugung des Hochkonzentrationssignals unmittelbar vor dem Zeitpunkt der Umschaltung von dem Hochkonzentrationssignal zu dem Niedrigkonzentrationssignal erhalten wurden, der stärksten Verschiebung in die Richtung hoher Konzentration entspricht, und der Anfangspunkt-Sensorausgangswert ein Sensorausgangswert ist, der zu Beginn des Zeitabschnitts der Erzeugung des Hochkonzentrationssignals erhalten wurde, und
wobei das Endzeitpunkt-Vorausbestimmungsmittel das Ende des sensibilisierten Zeitabschnitts mit zunehmendem absolutem Betrag der maximalen Differenz stärker verzögert.

12. Gasdetektionsvorrichtung nach Anspruch 9 oder 10, wobei die Hystereseperiode-Information einen maximalen Differenzwert beinhaltet, welcher eine Differenz ist, die aus Differenzen zwischen dem Referenzwert und jeweiligen Sensorausgangswerten ausgewählt ist, die in einem Zeitabschnitt der Erzeugung des Hochkonzentrationssignals unmittelbar vor dem Zeitpunkt der Umschaltung von dem Hochkonzentrationssignal zu dem Niedrigkonzentrationssignal erhalten wurden, wobei die ausgewählte Differenz den größten absoluten Betrag unter den Differenzen hat, und
wobei das Endzeitpunkt-Vorausbestimmungsmittel das Ende des sensibilisierten Zeitabschnitts mit zunehmendem absolutem Betrag der maximalen Differenz stärker verzögert.

13. Gasdetektionsvorrichtung nach einem der Ansprüche 9 bis 12, wobei die Hystereseperiode-Information die Dauer eines Zeitabschnitts der Erzeugung des Hochkonzentrationssignals unmittelbar vor dem Zeitpunkt der Umschaltung von dem Hochkonzentrationssignal zu dem Niedrigkonzentrationssignal beinhaltet, und
wobei das Endzeitpunkt-Vorausbestimmungsmittel das Ende des sensibilisierten Zeitabschnitts stärker verzögert, wenn sich die Dauer verlängert.

14. Gasdetektionsvorrichtung nach einem der Ansprüche 1 bis 7, aufweisend:
Berechnungsmittel für den Wert einer gleitenden Differenz zur Alternationsbestimmung, zum Berechnen eines Wertes einer gleitenden Differenz zur Alternationsbestimmung aus einem gegenwärtigen Sensorausgangswert und einem i-ten vergangenen Sensorausgangswert, welcher ein Sensorausgangswert ist, der i Zyklen vor dem gegenwärtigen Sensorausgangswert erhalten wurde (i ist eine ganze Zahl, die nicht kleiner als 1 ist); und
Bestimmungsmittel für den Wert der gleitenden Differenz zum Bestimmen, ob sich der Wert der gleitenden Differenz zur Alternationsbestimmung in Bezug auf einen als Referenz dienenden vorbestimmten Wert auf der Seite zu 0 hin befindet,
wobei die Konzentrationserhöhungs-Detektionsmittel, nachdem sich der Wert der gleitenden Differenz zur Alternationsbestimmung zu der Seite zu 0 hin verschoben hat, anstelle des ersten Hochkonzentrationsschwellwertes (Tu1) den zweiten Hochkonzentrationsschwellwert (Tu2) als den Hochkonzentrationsschwellwert verwenden.

15. Gasdetektionsvorrichtung nach einem der Ansprüche 1 bis 7, aufweisend:
Berechnungsmittel für den Wert einer gleitenden Differenz zur Alternationsbestimmung, zum Berechnen eines Wertes einer gleitenden Differenz zur Alternationsbestimmung aus einem gegenwärtigen Sensorausgangswert und einem i-ten vergangenen Sensorausgangswert, welcher ein Sensorausgangswert ist, der i Zyklen vor dem gegenwärtigen Sensorausgangswert erhalten wurde (i ist eine ganze Zahl, die nicht kleiner als 1 ist); und
Bestimmungsmittel für den Wert der gleitenden Differenz zum Bestimmen, ob sich der Wert der gleitenden Differenz zur Alternationsbestimmung in Bezug auf einen als Referenz dienenden vorbestimmten Wert auf der Seite zu 0 hin befindet,
wobei das Konzentrationserhöhungs-Detektionsmittel nach Ablauf eines vorbestimmten Zeitabschnitts, nachdem sich der Wert der gleitenden Differenz zur Alternationsbestimmung zu der Seite zu 0 hin verschoben hat, anstelle des ersten Hochkonzentrationsschwellwertes (Tu1) den zweiten Hochkonzentrationsschwellwert (Tu2) als den Hochkonzentrationsschwellwert verwendet.

16. Gasdetektionsvorrichtung nach Anspruch 14 oder 15, wobei ab dem Zeitpunkt der Umschaltung von dem Hochkonzentrationssignal zu dem Niedrigkonzentrationssignal das Berechnungsmittel für den Wert einer gleitenden Differenz zur Alternationsbestimmung mit der Berechnung des Wertes der gleitenden Differenz zur Alternationsbestimmung beginnt oder mit der Berechnung des Wertes der gleitenden Differenz zur Alternationsbestimmung fortfährt, ohne Sensorausgangswerte zu verwenden, die vor dem Zeitpunkt der Umschaltung erhalten wurden, und
wobei während eines Zeitabschnitts vom Zeitpunkt der Umschaltung bis zu einem Zeitpunkt, zu dem die Gewinnung von Sensorausgangswerten entsprechend der vorbestimmten Anzahl (i) von Zyklen abgeschlossen ist, die Differenz zwischen einem Sensorausgangswert, der zum Zeitpunkt der Umschaltung erhalten wurde, und einem Sensorausgangswert, der in jedem nachfolgenden Zyklus erhalten wurde, als der Wert der gleitenden Differenz zur Alternationsbestimmung in jeweiligen Zyklen verwendet wird.

17. Gasdetektionsvorrichtung nach Anspruch 14 oder 15, wobei ab dem Zeitpunkt der Umschaltung von dem Hochkonzentrationssignal zu dem Niedrigkonzentrationssignal das Berechnungsmittel für den Wert einer gleitenden Differenz zur Alternationsbestimmung mit der Berechnung des Wertes der gleitenden Differenz zur Alternationsbestimmung beginnt oder mit der Berechnung des Wertes der gleitenden Differenz zur Alternationsbestimmung fortfährt, ohne Sensorausgangswerte zu verwenden, die vor dem Zeitpunkt der Umschaltung erhalten wurden, und
wobei während eines Zeitabschnitts vom Zeitpunkt der Umschaltung bis zu einem Zeitpunkt, zu dem die Gewinnung von Sensorausgangswerten entsprechend der vorbestimmten Anzahl (i) von Zyklen abgeschlossen ist, ein Wert, der erhalten wurde, indem die Differenz zwischen einem Sensorausgangswert, der zum Zeitpunkt der Umschaltung erhalten wurde, und einem Sensorausgangswert, der b Zyklen nach dem Zeitpunkt der Umschaltung erhalten wurde, mit i/b multipliziert wurde, als der Wert der gleitenden Differenz zur Alternationsbestimmung in jeweiligen Zyklen verwendet wird.

18. Gasdetektionsvorrichtung nach einem der Ansprüche 1 bis 7, welche aufweist:
Bestimmungsmittel zum Bestimmen, ob sich der gegenwärtige Sensorausgangswert zur Seite der Richtung niedriger Konzentration in Bezug auf einen Annullierungs-Schwellwert verschoben hat, welcher ein Zwischenwert zwischen einem Anfangspunkt-Sensorausgangswert und einem Spitzen-Sensorausgangswert ist, wobei der Anfangspunkt-Sensorausgangswert ein Sensorausgangswert ist, der zu Beginn eines Zeitabschnitts der Erzeugung des Hochkonzentrationssignals unmittelbar vor der Umschaltung von dem Hochkonzentrationssignal zu dem Niedrigkonzentrationssignal erhalten wurde, und der Spitzen-Sensorausgangswert ein Wert ist, welcher unter Sensorausgangswerten, die in dem Zeitabschnitt der Erzeugung des Hochkonzentrationssignals erhalten wurden, der stärksten Verschiebung in die Richtung hoher Konzentration entspricht,
wobei das Konzentrationserhöhungs-Detektionsmittel anstelle des ersten Hochkonzentrationsschwellwertes (Tu1) den zweiten Hochkonzentrationsschwellwert (Tu2) als den Hochkonzentrationsschwellwert verwendet, wenn sich der gegenwärtige Sensorausgangswert zur Seite der Richtung niedriger Konzentration in Bezug auf den Annullierungs-Schwellwert verschoben hat.

19. Gasdetektionsvorrichtung nach Anspruch 18, wobei der Annullierungs-Schwellwert ein Wert ist, welcher von dem Anfangspunkt-Sensorausgangswert zu dem Spitzen-Sensorausgangswert hin um einen Betrag verschoben ist, der durch Division der Differenz zwischen dem Spitzen-Sensorausgangswert und dem Anfangspunkt-Sensorausgangswert durch a erhalten wird, wobei a > 1 ist.

20. Gasdetektionsvorrichtung nach Anspruch 1,
wobei das Konzentrationsdetektionsmittel Endzeitpunkt-Bestimmungsmittel zum Bestimmen des Endes des sensibilisierten Zeitabschnitts aufweist.

21. Automatisches Belüftungssystem für ein Fahrzeug, welches eine Gasdetektionsvorrichtung (10) nach einem der Ansprüche 1 bis 20 aufweist.

22. Verfahren zum Betreiben einer Gasdetektionsvorrichtung mit einem Gassensorelement, dessen Sensorwiderstand sich entsprechend der Konzentration eines spezifischen Gases ändert, Erfassungsmitteln zum Erfassen eines Sensorausgangswertes in vorbestimmten Zyklusintervallen, wobei der Sensorausgangswert dem Sensorwiderstand entspricht, und mit Konzentrationsdetektionsmitteln zum Detektieren einer Änderung in der Konzentration des spezifischen Gases und zum Erzeugen eines Niedrigkonzentrationssignals, wenn die Konzentration des spezifischen Gases niedrig ist, und eines Hochkonzentrationssignals, wenn die Konzentration des spezifischen Gases hoch ist,
die folgenden Schritte aufweisend:
Erzeugen des Hochkonzentrationssignals anstelle des Niedrigkonzentrationssignals mit einem Konzentrationserhöhungs-Detektionsmittel, wenn während eines Zeitraums, in welchem das Niedrigkonzentrationssignal erzeugt wird, der Sensorausgangswert sich in einer Richtung hoher Konzentration in Bezug auf einen Referenzwert um einen Betrag verschiebt, der gleich einem oder größer als ein Hochkonzentrationsschwellwert ist, und
Erzeugen des Niedrigkonzentrationssignals anstelle des Hochkonzentrationssignals mit einem Konzentrationsverringerungs-Detektionsmittel, wenn während eines Zeitraums, in welchem das Hochkonzentrationssignal erzeugt wird, der Sensorausgangswert sich in einer Richtung niedriger Konzentration in Bezug auf den Referenzwert um einen Betrag verschiebt, der gleich einem oder größer als ein Niedrigkonzentrationsschwellwert ist,
wobei das Konzentrationserhöhungs-Detektionsmittel verwendet:
einen ersten Hochkonzentrationsschwellwert als den Hochkonzentrationsschwellwert während eines sensibilisierten Zeitabschnitts des Zeitabschnitts, in welchem das Niedrigkonzentrationssignal erzeugt wird, wobei der sensibilisierte Zeitabschnitt auf eine Umschaltung von dem Hochkonzentrationssignal zu dem Niedrigkonzentrationssignal folgt, und
einen zweiten Hochkonzentrationsschwellwert als den Hochkonzentrationsschwellwert während des restlichen Teils des Zeitabschnitts, in welchem das Niedrigkonzentrationssignal erzeugt wird, und
wobei der erste Hochkonzentrationsschwellwert ein Wert auf einer Seite der Richtung zunehmender Empfindlichkeit in Bezug auf den zweiten Hochkonzentrationsschwellwert ist.

23. Verfahren zum Betreiben einer Gasdetektionsvorrichtung nach Anspruch 22, welches ferner den folgenden Schritt aufweist:
Bewirken, in dem sensibilisierten Zeitabschnitt, mit Aktualisierungsmitteln für den ersten Hochkonzentrationsschwellwert, dass sich der erste Hochkonzentrationsschwellwert im Laufe der Zeit allmählich an den zweiten Hochkonzentrationsschwellwert annähert.

24. Verfahren zum Betreiben einer Gasdetektionsvorrichtung nach Anspruch 22 oder 23, welches ferner den folgenden Schritt aufweist:
Bestimmen des ersten Hochkonzentrationsschwellwertes auf der Basis von Hysteresestärke-Information mit Bestimmungsmitteln für den ersten Hochkonzentrationsschwellwert.

25. Verfahren zum Betreiben einer Gasdetektionsvorrichtung nach Anspruch 24, welches ferner den folgenden Schritt aufweist:
Berechnen eines Wertes einer gleitenden Differenz zur Bestimmung des ersten Hochkonzentrationsschwellwertes mit Berechnungsmitteln für den Wert einer gleitenden Differenz zur Schwellwertbestimmung aus einem gegenwärtigen Sensorausgangswert und einem m-ten vergangenen Sensorausgangswert, welcher ein Sensorausgangswert ist, der m Zyklen vor dem gegenwärtigen Sensorausgangswert erhalten wurde (m ist eine ganze Zahl, die nicht kleiner als 1 ist),
wobei die Hysteresestärke-Information den Wert der gleitenden Differenz zur Bestimmung des ersten Hochkonzentrationsschwellwertes beinhaltet, welcher zum Zeitpunkt der Umschaltung von dem Hochkonzentrationssignal zu dem Niedrigkonzentrationssignal, einem Zeitpunkt, der um eine vorbestimmte Zeit früher als der Zeitpunkt der Umschaltung ist, oder einem Zeitpunkt, der um eine vorbestimmte Zeit später als der Zeitpunkt der Umschaltung ist, erhalten wird, und
wobei der erste Hochkonzentrationsschwellwert mit dem Aktualisierungsmitteln für den ersten Hochkonzentrationsschwellwert entsprechend dem absoluten Betrag des Wertes der gleitenden Differenz zur Bestimmung des ersten Hochkonzentrationsschwellwertes, welcher als die Hysteresestärke-Information verwendet wird, in die Richtung zunehmender Empfindlichkeit verschoben wird.

26. Verfahren zum Betreiben einer Gasdetektionsvorrichtung nach Anspruch 22, welches ferner aufweist:
Bestimmen des Endes eines sensibilisierten Zeitabschnitts mit Endzeitpunkt-Bestimmungsmitteln.

27. Verfahren zum Betreiben einer Gasdetektionsvorrichtung nach Anspruch 22, wobei der sensibilisierte Zeitabschnitt bei der Umschaltung von dem Hochkonzentrationssignal zu dem Niedrigkonzentrationssignal beginnt und zu einem Zeitpunkt endet, nachdem sich die Änderungsrichtung des Sensorausgangswertes von der Richtung niedriger Konzentration zur Richtung hoher Konzentration ändert.

## Revendications

1. Appareil de détection de gaz utilisant un élément de capteur de gaz (11) dont la résistance de capteur change conformément à la concentration d'un gaz spécifique, comprenant :
des moyens d'acquisition (19) pour acquérir une valeur de sortie de capteur à des intervalles de cycle prédéterminés, la valeur de sortie de capteur correspondant à la résistance du capteur ; et
des moyens de détection de concentration pour détecter un changement de la concentration du gaz spécifique et pour générer un signal de faible concentration lorsque la concentration du gaz spécifique est faible et un signal de concentration élevée lorsque la concentration du gaz spécifique est élevée,
dans lequel les moyens de détection de concentration (S3B) comprennent
des moyens de détection d'augmentation de concentration pour générer le signal de concentration élevée au lieu du signal de faible concentration lorsque, pendant une période dans laquelle le signal de faible concentration est généré, la valeur de sortie de capteur se décale dans une direction de concentration élevée par rapport à une valeur de référence (B(n)), d'une quantité égale ou supérieure à un seuil de concentration élevée (Tu, Tu1, Tu2), et
des moyens de détection de diminution de concentration (S3C) pour générer le signal de faible concentration au lieu du signal de concentration élevée lorsque, pendant une période dans laquelle le signal de concentration élevée est généré, la valeur de sortie de capteur se décale dans une direction de faible concentration par rapport à la valeur de référence d'une quantité égale ou supérieure à un seuil de faible concentration (Td),
dans lequel les moyens de détection d'augmentation de concentration utilisent
un premier seuil de concentration élevée (Tu1) en tant que seuil de concentration élevée pendant une période sensibilisée de la période dans laquelle le signal de faible concentration est généré, la période sensibilisée suivant une commutation du signal de concentration élevée vers le signal de faible concentration, et
un deuxième seuil de concentration élevée (Tu2) en tant que seuil de concentration élevée pendant la partie restante de la période dans laquelle le signal de faible concentration est généré, et
dans lequel le premier seuil de concentration élevée est une valeur d'un côté de direction d'augmentation de sensibilité par rapport au deuxième seuil de concentration élevée.

2. Appareil de détection de gaz selon la revendication 1, comprenant des moyens de mise à jour de premier seuil de concentration élevée pour amener, dans la période sensibilisée, le premier seuil de concentration élevée (Tu1) à s'approcher graduellement du deuxième seuil de concentration élevée (Tu2) alors que le temps s'écoule.

3. Appareil de détection de gaz selon la revendication 1 ou 2, comprenant des moyens de détermination de premier seuil de concentration élevée pour déterminer le premier seuil de concentration élevée sur la base d'information d'intensité d'hystérésis.

4. Appareil de détection de gaz selon la revendication 3, comprenant :
des moyens de calcul de valeur de différence mobile de détermination de seuil pour calculer une valeur de différence mobile de détermination de premier seuil de concentration élevée à partir d'une valeur de sortie de capteur actuelle et d'une m^{ème} valeur de sortie de capteur passée qui est une valeur de sortie de capteur obtenue m cycles avant la valeur de sortie de capteur actuelle (m est un entier qui n'est pas inférieur à 1),
dans lequel les informations d'intensité d'hystérésis comprennent la valeur de différence mobile de détermination de premier seuil de concentration élevée qui est obtenue à l'instant de commutation du signal de concentration élevée vers le signal de faible concentration, à un instant qui est un temps prédéterminé plus tôt que l'instant de commutation, ou à un instant qui est un temps prédéterminé plus tard que le temps de commutation, et
dans lequel les moyens de mise à jour de premier seuil de concentration élevée décalent le premier seuil de concentration élevée (Tu1) dans la direction d'augmentation de sensibilité conformément à la valeur absolue de la valeur de différence mobile de détermination de premier seuil de concentration élevée, qui est utilisée en tant qu'informations d'intensité d'hystérésis.

5. Appareil de détection de gaz selon la revendication 3 ou 4, dans lequel les informations d'intensité d'hystérésis comprennent une différence maximum qui est une différence entre une valeur de sortie de capteur crête et une valeur de sortie de capteur de point de départ, la valeur de sortie de capteur crête étant une valeur qui se décale dans la direction de concentration élevée au degré le plus élevé parmi les valeurs de sortie de capteur obtenues dans une période de génération de signal de concentration élevée immédiatement avant l'instant de commutation du signal de concentration élevée vers le signal de faible concentration, et la valeur de sortie de capteur de point de départ étant une valeur de sortie de capteur obtenue au début de la période de génération de signal de concentration élevée, et
dans lequel les moyens de mise à jour de premier seuil de concentration élevée décalent le premier seuil de concentration élevée dans la direction d'augmentation de sensibilité alors que la valeur absolue de la différence maximum augmente.

6. Appareil de détection de gaz selon la revendication 3 ou 4, dans lequel les informations d'intensité d'hystérésis comprennent une valeur de différence maximum qui est une différence sélectionnée parmi les différences entre la valeur de référence et les valeurs de sortie de capteur respectives obtenues dans une période de génération de signal de concentration élevée immédiatement avant l'instant de commutation du signal de concentration élevée vers le signal de faible concentration, la différence sélectionnée ayant la valeur absolue la plus élevée parmi les différences, et
dans lequel les moyens de mise à jour de premier seuil de concentration élevée décalent le premier seuil de concentration élevée (Tu1) dans la direction d'augmentation de sensibilité alors que la valeur absolue de la différence maximum augmente.

7. Appareil de détection de gaz selon l'une quelconque des revendications 3 à 6, dans lequel les informations d'intensité d'hystérésis comprennent la durée d'une période de génération de signal de concentration élevée immédiatement avant l'instant de commutation du signal de concentration élevée vers le signal de faible concentration, et
dans lequel les moyens de mise à jour de premier seuil de concentration élevée décalent le premier seuil de concentration élevée dans la direction d'augmentation de sensibilité alors que la durée augmente.

8. Appareil de détection de gaz selon l'une quelconque des revendications 1 à 7, comprenant des moyens de fin pour mettre fin à la période sensibilisée lorsqu'une première période de temps prédéterminée s'est écoulée après l'instant de commutation du signal de concentration élevée vers le signal de faible concentration.

9. Appareil de détection de gaz selon l'une quelconque des revendications 1 à 7, comprenant des moyens de détermination d'avance de synchronisation de fin pour déterminer au préalable la fin de la période sensibilisée sur la base d'informations de période d'hystérésis.

10. Appareil de détection de gaz selon la revendication 9, comprenant :
des moyens de calcul de valeur de différence mobile de détermination de synchronisation de fin pour calculer une valeur de différence mobile de détermination de synchronisation de fin à partir d'une valeur de sortie de capteur actuelle et d'une j^{ème} valeur de sortie de capteur passée qui est une valeur de sortie de capteur obtenue j cycles avant la valeur de sortie de capteur actuelle (j est un entier qui n'est pas inférieur à 1),
dans lequel les informations de période d'hystérésis comprennent la valeur de différence mobile de détermination de synchronisation de fin qui est obtenue à l'instant de commutation du signal de concentration élevée vers le signal de faible concentration, à un instant qui est un temps prédéterminé plus tôt que l'instant de commutation, ou à un instant qui est un temps prédéterminé plus tard que l'instant de commutation, et
dans lequel les moyens de détermination d'avance de synchronisation de fin retardent la fin de la période sensibilisée à un degré plus élevé avec l'augmentation de la valeur absolue de la valeur de différence mobile de détermination de synchronisation de fin, qui est utilisée en tant qu'informations de période d'hystérésis.

11. Appareil de détection de gaz selon la revendication 9 ou 10, dans lequel les informations de période d'hystérésis comprennent une différence maximum qui est une différence entre une valeur de sortie de capteur crête et une valeur de sortie de capteur de point de départ, la valeur de sortie de capteur crête étant une valeur qui se décale dans la direction de concentration élevée au degré le plus élevé parmi les valeurs de sortie de capteur obtenues dans une période de génération de signal de concentration élevée immédiatement avant l'instant de commutation du signal de concentration élevée vers le signal de faible concentration, et la valeur de sortie de capteur de point de départ étant une valeur de sortie de capteur obtenue au début de la période de génération de signal de concentration élevée, et
dans lequel les moyens de détermination d'avance de synchronisation de fin retardent la fin de la période sensibilisée à un degré plus élevé avec l'augmentation de la valeur absolue de la différence maximum.

12. Appareil de détection de gaz selon la revendication 9 ou 10, dans lequel les informations de période d'hystérésis comprennent une valeur de différence maximum qui est une différence sélectionnée parmi les différences entre la valeur de référence et les valeurs de sortie de capteur respectives obtenues dans une période de génération de signal de concentration élevée immédiatement avant l'instant de commutation du signal de concentration élevée vers le signal de faible concentration, la différence sélectionnée ayant la valeur absolue la plus grande parmi les différences,
dans lequel les moyens de détermination d'avance de synchronisation de fin retardent la fin de la période sensibilisée à un degré plus élevé avec l'augmentation de la valeur absolue de la différence maximum.

13. Appareil de détection de gaz selon l'une quelconque des revendications 9 à 12, dans lequel les informations de période d'hystérésis comprennent la durée d'une période de génération de signal de concentration élevée immédiatement avant l'instant de commutation du signal de concentration élevée vers le signal de faible concentration, et
dans lequel les moyens de détermination d'avance de synchronisation de fin retardent la fin de la période sensibilisée à un degré plus élevé alors que la durée augmente.

14. Appareil de détection de gaz selon l'une quelconque des revendications 1 à 7, comprenant :
des moyens de calcul de valeur de différence mobile de détermination d'alternance pour calculer une valeur de différence mobile de détermination d'alternance à partir d'une valeur de sortie de capteur actuelle et d'une i^{ème} valeur de sortie de capteur passée qui est une valeur de sortie de capteur obtenue i cycles avant la valeur de sortie de capteur actuelle (i est un entier qui n'est pas inférieur à 1) ; et
des moyens de détermination de valeur de différence mobile pour déterminer si la valeur de différence mobile de détermination d'alternance est située du côté vers 0 par rapport à une valeur prédéterminée servant en tant que référence,
dans lequel les moyens de détection d'augmentation de concentration utilisent, au lieu du premier seuil de concentration élevée (Tu1), le deuxième seuil de concentration élevée (Tu2) en tant que seuil de concentration élevée après que la valeur de différence mobile de détermination d'alternance s'est décalée du côté vers 0.

15. Appareil de détection de gaz selon l'une quelconque des revendications 1 à 7, comprenant :
des moyens de calcul de valeur de différence mobile de détermination d'alternance pour calculer une valeur de différence mobile de détermination d'alternance à partir d'une valeur de sortie de capteur actuelle et d'une i^{ème} valeur de sortie de capteur passée qui est une valeur de sortie de capteur obtenue i cycles avant la valeur de sortie de capteur actuelle (i est un entier qui n'est pas inférieur à 1) ; et
des moyens de détermination de valeur de différence mobile pour déterminer si la valeur de différence mobile de détermination d'alternance est située du côté vers 0 par rapport à une valeur prédéterminée servant en tant que référence,
dans lequel les moyens de détection d'augmentation de concentration utilisent, au lieu du premier seuil de concentration élevée (Tu1), le deuxième seuil de concentration élevée (Tu2) en tant que seuil de concentration élevée après qu'une période de temps prédéterminée s'est écoulée après que la valeur de différence mobile de détermination d'alternance s'est décalée du côté vers 0.

16. Appareil de détection de gaz selon la revendication 14 ou 15, dans lequel, à partir de l'instant de commutation du signal de concentration élevée vers le signal de faible concentration, les moyens de calcul de valeur de différence mobile de détermination d'alternance débutent le calcul de la valeur de différence mobile de détermination d'alternance, ou reprennent le calcul de la valeur de différence mobile de détermination d'alternance sans utiliser les valeurs de sortie de capteur obtenues avant l'instant de commutation, et
dans lequel, pendant une période de l'instant de commutation jusqu'à un instant auquel l'obtention des valeurs de sortie de capteur correspondant au nombre prédéterminé (i) de cycles est achevée, la différence entre une valeur de sortie de capteur obtenue à l'instant de commutation et une valeur de sortie de capteur obtenue à chaque cycle suivant est utilisée en tant que valeur de différence mobile de détermination d'alternance dans les cycles respectifs.

17. Appareil de détection de gaz selon la revendication 14 ou 15, dans lequel, à partir de l'instant de commutation du signal de concentration élevée vers le signal de faible concentration, les moyens de calcul de valeur de différence mobile de détermination d'alternance débutent le calcul de la valeur de différence mobile de détermination d'alternance, ou reprennent le calcul de la valeur de différence mobile de détermination d'alternance sans utiliser les valeurs de sortie de capteur obtenues avant l'instant de commutation, et
dans lequel, pendant une période de l'instant de commutation jusqu'à un instant auquel l'obtention des valeurs de sortie de capteur correspondant au nombre prédéterminé (i) de cycles est achevée, une valeur obtenue par la multiplication, par i/b, de la différence entre une valeur de sortie de capteur obtenue à l'instant de commutation et une valeur de sortie de capteur obtenue b cycles après l'instant de commutation est utilisée en tant que valeur de différence mobile de détermination d'alternance dans les cycles respectifs.

18. Appareil de détection de gaz selon l'une quelconque des revendications 1 à 7, comprenant :
des moyens de détermination pour déterminer si la valeur de sortie de capteur actuelle s'est décalée du côté dans la direction de faible concentration par rapport à un seuil d'annulation qui est une valeur intermédiaire entre une valeur de sortie de capteur de point de départ et une valeur de sortie de capteur crête, la valeur de sortie de capteur de point de départ étant une valeur de sortie de capteur obtenue au début d'une période de génération de signal de concentration élevée immédiatement avant la commutation du signal de concentration élevée vers le signal de faible concentration, et la valeur de sortie de capteur crête étant une valeur qui se décale dans la direction de concentration élevée au degré le plus élevé parmi les valeurs de sortie de capteur obtenues dans la période de génération de signal de concentration élevée,
dans lequel les moyens de détection d'augmentation de concentration utilisent, au lieu du premier seuil de concentration élevée (Tu1), le deuxième seuil de concentration élevée (Tu2) en tant que seuil de concentration élevée lorsque la valeur de sortie de capteur actuelle s'est décalée du côté dans la direction de faible concentration par rapport au seuil d'annulation.

19. Appareil de détection de gaz selon la revendication 18, dans lequel le seuil d'annulation est une valeur qui est décalée vers la valeur de sortie de capteur crête à partir de la valeur de sortie de capteur de point de départ d'une quantité obtenue par la division par a de la différence entre la valeur de sortie de capteur crête et la valeur de sortie de capteur de point de départ, où a > 1.

20. Appareil de détection de gaz selon la revendication 1,
dans lequel les moyens de détection de concentration comprennent des moyens de détermination de synchronisation de fin pour déterminer la fin de ladite période sensibilisée.

21. Système de ventilation automatique pour un véhicule comprenant un appareil de détection de gaz (10) selon l'une quelconque des revendications 1 à 20.

22. Procédé de mise en oeuvre d'un appareil de détection de gaz avec un élément de capteur de gaz dont la résistance de capteur change conformément à la concentration d'un gaz spécifique, des moyens d'acquisition pour acquérir une valeur de sortie de capteur à des intervalles de cycle prédéterminés, la valeur de sortie de capteur correspondant à la résistance de capteur, et avec des moyens de détection de concentration pour détecter un changement de la concentration du gaz spécifique et pour générer un signal de faible concentration lorsque la concentration du gaz spécifique est faible et un signal de concentration élevée lorsque la concentration du gaz spécifique est élevée,
comprenant les étapes :
de génération du signal de concentration élevée au lieu du signal de faible concentration par des moyens de détection d'augmentation de concentration lorsque, pendant une période dans laquelle le signal de faible concentration est généré, la valeur de sortie de capteur se décale dans une direction de concentration élevée par rapport à une valeur de référence d'une quantité égale ou supérieure à un seuil de concentration élevée, et
de génération du signal de faible concentration au lieu du signal de concentration élevée par des moyens de détection de diminution de concentration lorsque, pendant une période dans laquelle le signal de concentration élevée est généré, la valeur de sortie de capteur se décale dans une direction de faible concentration par rapport à la valeur de référence d'une quantité égale ou supérieure à un seuil de faible concentration,
dans lequel les moyens de détection d'augmentation de concentration utilisent
un premier seuil de concentration élevée en tant que seuil de concentration élevée pendant une période sensibilisée de la période dans laquelle le signal de faible concentration est généré, la période sensibilisée suivant une commutation du signal de concentration élevée vers le signal de faible concentration, et
un deuxième seuil de concentration élevée en tant que seuil de concentration élevée pendant la partie restante de la période dans laquelle le signal de faible concentration est généré, et
dans lequel le premier seuil de concentration élevée est une valeur d'un côté de direction d'augmentation de sensibilité par rapport au deuxième seuil de concentration élevée.

23. Procédé de mise en oeuvre d'un appareil de détection de gaz selon la revendication 22, comprenant en outre l'étape consistant à : amener, dans la période sensibilisée, le premier seuil de concentration élevée à s'approcher graduellement du deuxième seuil de concentration élevée alors que le temps s'écoule par des moyens de mise à jour de premier seuil de concentration élevée.

24. Procédé de mise en oeuvre d'un appareil de détection de gaz selon la revendication 22 ou 23, comprenant en outre l'étape consistant à : déterminer le premier seuil de concentration élevée sur la base d'informations d'intensité d'hystérésis par des moyens de détermination de premier seuil de concentration élevée.

25. Procédé de mise en oeuvre d'un appareil de détection de gaz selon la revendication 24, comprenant en outre l'étape de :
calcul d'une valeur de différence mobile de détermination de premier seuil de concentration élevée par des moyens de calcul de valeur de différence mobile de détermination de seuil à partir d'une valeur de sortie de capteur actuelle et d'une m^{ème} valeur de sortie de capteur passée qui est une valeur de sortie de capteur obtenue m cycles avant la valeur de sortie de capteur actuelle (m est un entier qui n'est pas inférieur à 1),
dans lequel les informations d'intensité d'hystérésis comprennent la valeur de différence mobile de détermination de premier seuil de concentration élevée qui est obtenue à l'instant de commutation du signal de concentration élevée vers le signal de faible concentration, à un instant un temps prédéterminé plus tôt que l'instant de commutation, ou à un instant un temps prédéterminé plus tard que l'instant de commutation, et
dans lequel le premier seuil de concentration élevée est décalé par les moyens de mise à jour de premier seuil de concentration élevée dans la direction d'augmentation de sensibilité conformément à la valeur absolue de la valeur de différence mobile de détermination de premier seuil de concentration élevée, qui est utilisée en tant qu'informations d'intensité d'hystérésis.

26. Procédé de mise en oeuvre d'un appareil de détection de gaz selon la revendication 22, comprenant en outre
la détermination de la fin d'une période sensibilisée par des moyens de détermination de synchronisation de fin.

27. Procédé de mise en oeuvre d'un appareil de détection de gaz selon la revendication 22, dans lequel la période sensibilisée débute à l'instant de commutation du signal de concentration élevée vers le signal de faible concentration, et se termine à un instant après le changement de la direction de changement de la valeur de sortie de capteur de la direction de faible concentration vers la direction de concentration élevée.
